# EUROPÄISCHE PATENTSCHRIFT

(11) **EP 2 167 473 B1**
(45) Veröffentlichungstag und Bekanntmachung des Hinweises auf die Patenterteilung: **05.07.2017**
(21) Anmeldenummer: 08758845.5
(22) Anmeldetag: 29.05.2008
(51) Int. Cl.: C07D 231/20, A01N 43/56

(54) **3-CYCLOPROPYL-4-(3-THIOBENZOYL)PYRAZOLE UND IHRE VERWENDUNG ALS HERBIZIDE**
3-CYCLOPROPYL-4-(3-THIOBENZOYL)PYRAZOLES AND THEIR USE AS HERBICIDES
3-CYCLOPROPYL-4-(3-THIOBENZOYL)PYRAZOLES ET LEUR UTILISATION EN TANT QU'HERBICIDES

(30) Priorität: 11.06.2007 DE 102007026875
(43) Veröffentlichungstag der Anmeldung: 31.03.2010
(73) Patentinhaber: Bayer Intellectual Property GmbH, 40789 Monheim am Rhein (DE)
(72) Erfinder: AHRENS, Hartmut, 63329 Egelsbach (DE); VAN ALMSICK, Andreas, 61184 Karben (DE); LEHR, Stefan, 65835 Liederbach (DE); SCHMITT, Monika, 60318 Frankfurt a M. (DE); DITTGEN, Jan, 60316 Frankfurt a. M. (DE); FEUCHT, Dieter, 65760 Eschborn (DE); HILLS, Martin, Jeffrey, 65510 Idstein (DE); KEHNE, Heinz, 65719 Hofheim (DE); ROSINGER, Christopher, Hugh, 65719 Hofheim (DE)
(74) Vertreter: BIP Patents
(86) Internationale Anmeldenummer: PCT/EP2008/004262
(87) Internationale Veröffentlichungsnummer: WO 2008/151719

(56) Entgegenhaltungen:
- EP-A1- 0 352 543
- EP-A1- 0 580 439
- WO-A1-94/02465
- WO-A1-94/18179
- WO-A1-97/41106
- GB-A- 1 447 032
- DATABASE WPI Week 200014 Thomson Scientific, London, GB; AN 2000-161086 XP002500085 & WO 00/03993 A1 (ISHIHARA SANGYO KAISHA LTD) 27. Januar 2000 (2000-01-27) in der Anmeldung erwähnt
- DATABASE CA [Online] CHEMICAL ABSTRACTS SERVICE, COLUMBUS, OHIO, US; 1978, MASTER, H. E. ET AL: "Preparation of 4'-amino-3-carboxy-6-methoxydiphenyl sulfide and its derivatives" XP002500081 gefunden im STN Database accession no. 1978:546566 & JOURNAL OF THE INDIAN CHEMICAL SOCIETY , 55(3), 288-9 CODEN: JICSAH; ISSN: 0019-4522, 1978,
- DATABASE CA [Online] CHEMICAL ABSTRACTS SERVICE, COLUMBUS, OHIO, US; 1976, GOGHARI, M. H. ET AL: "Fries reaction. XV. Preparation of hydroxydiaryl sulfones" XP002500082 gefunden im STN Database accession no. 1976:73810 & CHEMICAL ERA , 11(6), 34-6 CODEN: CHERDB; ISSN: 0009-2533, 1975,
- PATWA B S ET AL: "FRIES REACTION. ÖPART XI, PREPARATION OF HYDROXYDIARYLSULPHONES" JOURNAL OF THE INSTITUTION OF CHEMISTS (INDIA), CALCUTTA, IN, Bd. 46, Nr. 4, 1. Juli 1974 (1974-07-01), Seiten 140-142, XP000600341 ISSN: 0020-3254
- DATABASE REGISTRY CHEMICAL ABSTRACTS SERVICES, COLUMBUS, OHIO, US; Chemical Library; Supplier: Ambinter 7. März 2001 (2001-03-07), XP002500083 Database accession no. RN: 326023-24-9
- DATABASE REGISTRY CHEMICAL ABSTRACTS SERVICES, COLUMBUS, OHIO, US; Chemical Library; Supplier: Oak Samples Ltd. 5. März 2001 (2001-03-05), XP002500084 Database accession no. RN: 325704-11-8
- DATABASE REGISTRY CHEMICAL ABSTRACTS SERVICES, COLUMBUS, OHIO, US; Chemical Library; Supplier: ChemBridge Corp. 12. Mai 2002 (2002-05-12), XP002524369 Database accession no. RN: 413594-90-8

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, daß bestimmte 4-Benzoylpyrazole herbizide Eigenschaften besitzen. So werden in EP 0 352 543 A1 4-Benzoylpyrazole genannt, die im Phenylring unter anderem durch einen Thiorest substituiert sein können. In WO 97/41106 und WO 00/03993 werden 3-Cyclopropyl-4-benzoylpyrazole genannt, die im Phenylring unter anderem durch einen Thiorest substituiert sein können. Die vorgenannten Schriften offenbaren keine Ausführungsbeispiele für eine Substitution des Phenylrings in meta-Position durch einen Thiorest.

Die aus diesen Schriften bekannten Verbindungen zeigen jedoch häufig eine nicht ausreichende herbizide Wirksamkeit. Aufgabe der vorliegenden Erfindung ist daher die Bereitstellung von herbizid wirksamen Verbindungen mit - gegenüber den aus dem Stand der Technik bekannten Verbindungen - verbesserten herbiziden Eigenschaften.

Es wurde nun gefunden, daß bestimmte 4-Benzoylpyrazole, die in 3-Position durch eine Cyclopropyl-Gruppe substituiert sind und deren Phenylring in 3-Position eine substituierte Sulfenyl-, Sulfinyl- oder Sulfonylgruppe trägt, als Herbizide besonders gut geeignet sind. Ein Gegenstand der vorliegenden Erfindung sind 3-Cyclopropyl-4-(3-thiobenzoyl)pyrazole der Formel (I) oder deren Salze 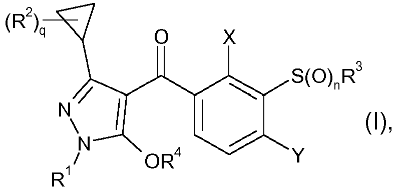 worin
R¹ bedeutet (C₁-C₄)-Alkyl,
R² bedeutet Halogen oder (C₁-C₄)-Alkyl,
R³ bedeutet (C₃-C₈)-Cycloalkyl-(C₁-C₉)-alkyl, (C₁-C₆)-Halogenalkyl, (C₃-C₈)-Halogencycloalkyl-(C₁-C₉)-alkyl, (C₂-C₆)-Nitroalkyl, (C₃-C₈)-Cycloalkoxy-(C₁-C₉)-alkyl, (C₃-C₈)-Cycloalkyl-(C₁-C₈)-alkoxy-(C₁-C₈)-alkyl, (C₁-C₆)-Alkoxy-(C₁-C₉)-alkyl, (C₂-C₆)-Alkenyloxy-(C₁-C₉)-alkyl, (C₂-C₆)-Alkinyloxy-(C₁-C₉)-alkyl, (C₁-C₆)-Halogenalkoxy-(C₁-C₉)-alkyl, (C₃-C₈)-Halogencycloalkyl(C₁-C₉)-alkoxy-(C₁-C₉)-alkyl, (C₂-C₆)-Halogenalkenyloxy-(C₁-C₉)-alkyl, (C₂-C₆)-Halogenalkinyloxy-(C₁-C₉)-alkyl, (C₂-C₆)-Nitroalkoxy-(C₁-C₉)-alkyl, Phenyloxy-(C₁-C₉)-alkyl, wobei die Phenyl-Gruppe jeweils durch m gleiche oder verschiedene Reste aus der Gruppe bestehend aus (C₁-C₃)-Alkyl, Halogen, Nitro, (C₁-C₃)-Alkoxy substituiert sein kann,
R⁴ bedeutet Wasserstoff, (C₁-C₆)-Alkylsulfonyl, (C₁-C₄)-Alkoxy-(C₁-C₆)-alkylsulfonyl, oder jeweils durch m gleiche oder verschiedene Reste aus der Gruppe bestehend aus Halogen, (C₁-C₄)-Alkyl und (C₁-C₄)-Alkoxy substituiertes Phenylsulfonyl, Thien-2-ylsulfonyl, Benzoyl, (Ethylthio)carbonyl, Benzoyl-(C₁-C₆)-alkyl oder Benzyl,
X und Y bedeuten unabhängig voneinander Wasserstoff, Mercapto, Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, OR⁵, Methylsulfonylethoxymethyl, Methylsulfonylethylsulfonylmethyl, Methoxyethylsulfonylmethyl, OCOR⁵, OSO₂R⁵, S(O)ₙR⁵, SO₂OR⁵, SO₂N(R⁵)₂, (C₁-C₃)-Alkoxy-(C₁-C₃)-alkoxy)-(C₁-C₃)-alkyl, NR⁵SO₂R⁵, NR⁵COR⁵, (C₁-C₆)-AlkylS(O)ₙR⁵, (C₁-C₆)-Alkyl-OR⁵, (C₁-C₆)-Alkyl-OCOR⁵, (C₁-C₆)-Alkyl-OSO2R⁵, (C₁-C₆)-Alkyl-SO₂OR⁵, (C₁-C₆)-Alkyl-SO₂N(R⁵)₂ oder (C₁-C₆)-Alkyl-NR⁵COR⁵;
R⁵ bedeutet Wasserstoff, (C₁-C₆)-Alkyl, (C₂-C₆)-Alkenyl, (C₂-C₆)-Alkinyl, (C₃-C₆)-Cycloalkyl, Phenyl oder Phenyl-(C₁-C₆)-alkyl, wobei die sechs letztgenannten Reste durch s Reste der Gruppe Hydroxy, Mercapto, Amino, Cyano, Nitro, Rhodano, OR⁶, SR⁶, N(R⁶)₂, NOR⁶, OCOR⁶, SCOR⁶, NR⁶COR⁶, CO₂R⁶, COSR⁶, CON(R⁶)₂, (C₁)-C₄-Alkyliminooxy, (C₁-C₄)-Alkoxyamino, (C₁-C₄)-Alkylcarbonyl, (C₁-C₄)-Alkoxy-(C₂-C₆)-alkoxycarbonyl und (C₁-C₄)-Alkylsulfonyl substituiert sind;
R⁶ bedeutet Wasserstoff, (C₁-C₆)-Alkyl, (C₂-C₆)-Alkenyl oder (C₂-C₆)-Alkinyl,
m bedeutet 0, 1, 2, 3, 4 oder 5,
n bedeutet 0, 1 oder 2,
q bedeutet 0, 1, 2, 3, 4 oder 5,
s bedeutet 0, 1, 2 oder 3,
mit der Maßgabe, dass R nicht (C₁-C₆)-Halogenalkyl bedeutet, wenn n für 0 steht.

Für den Fall, daß R⁴ Wasserstoff bedeutet, können die erfindungsgemäßen Verbindungen der Formel (I) in Abhängigkeit von äußeren Bedingungen, wie Lösungsmittel und pH-Wert, in unterschiedlichen tautomeren Strukturen auftreten:

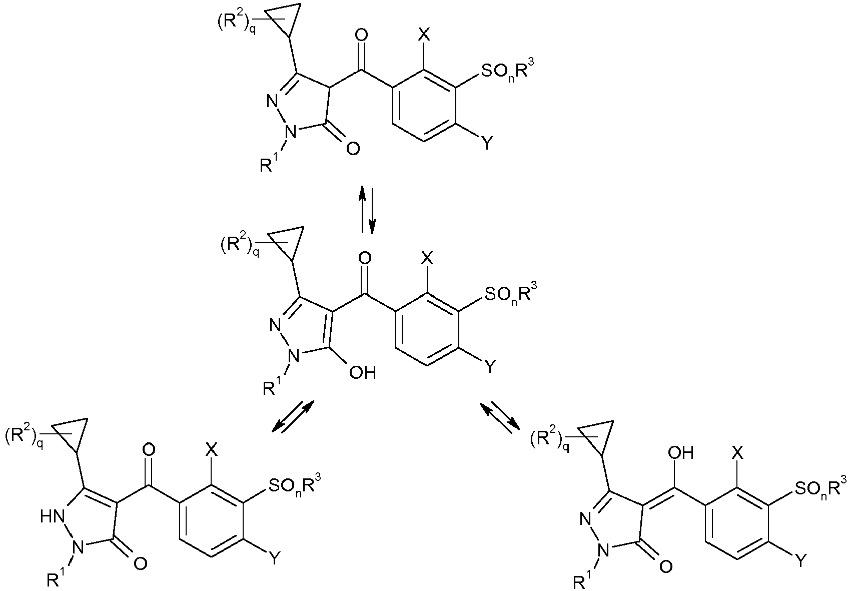

Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Lithium, Natrium, Kalium, Magnesium und Calcium sowie Ammoniak und organische Amine wie Triethylamin und Pyridin. Ebenso können Salze gebildet werden durch Anlagerung von organischen Säuren, wie Ameisen- oder Essigsäure, und anorganischen Säuren, wie Phosphor-, Salz- oder Schwefelsäure. Solche Salze sind ebenfalls Gegenstand der Erfindung.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder Iod. Tosyl bedeutet 4-Methylphenylsulfonyl.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Bevorzugt sind Verbindungen der allgemeinen Formel (I), worin
R¹ bedeutet (C₁-C₄)-Alkyl,
R² bedeutet Halogen, Methyl oder Ethyl,
R³ bedeutet cyclo-Propylmethyl, cyclo-Propylmethoxyethyl, Methoxyethyl, Methoxypropyl, Ethoxyethyl,
R⁴ bedeutet Wasserstoff, n-Propylsulfonyl, Phenylsulfonyl, Methoxyethylsulfonyl, Benzoylmethyl, Benzoyl, 4-Methylbenzoylmethyl, (Ethylthio)carbonyl, 4-Methylphenylsulfonyl, Thien-2-ylsulfonyl,
X bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
Y bedeutet Halogen, Trifluormethyl, (C₁-C₄)-Alkoxy, Methylsulfonyl oder Ethylsulfonyl,
n bedeutet 0, 1 oder 2,
q bedeutet 0, 1 oder 2.
Besonders bevorzugt sind Verbindungen der allgemeinen Formel (I), worin
R¹ bedeutet Methyl oder Ethyl,
R³ bedeutet cyclo-Propylmethyl, cyclo-Propylmethoxyethyl, Methoxyethyl, Methoxypropyl, Ethoxyethyl,
R⁴ bedeutet Wasserstoff, n-Propylsulfonyl, Phenylsulfonyl, Methoxyethylsulfonyl, Benzoylmethyl, Benzoyl, 4-Methylbenzoylmethyl, (Ethylthio)carbonyl 4-Methylphenylsulfonyl, Thien-2-ylsulfonyl,
X bedeutet Nitro, Brom, Chlor, Fluor, Methyl, Ethyl, Trifluormethyl, Methoxy, Ethoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
Y bedeutet Brom, Chlor, Fluor, Trifluormethyl, Methoxy, Methylsulfonyl oder Ethylsulfonyl,
n bedeutet 0, 1 oder 2,
q bedeutet 0.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R⁴ für Wasserstoff steht, können beispielsweise nach dem in Schema 1 angegebenen und von B. S. Jensen (Acta Chemica Scandinavica 13 (1959), 1668 - 1670) bekannten Verfahren durch basenkatalysierte Umsetzung eines Benzoesäurehalogenids (III) mit einem Pyrazolon (II) oder gemäß dem in Schema 2 angegebenen und beispielsweise aus EP-A 0 186 117 bekannten Verfahren durch basenkatalysierte Umsetzung eines Benzoesäurehalogenids (III) mit einem Pyrazolon (II) und anschließender Umlagerung hergestellt werden.

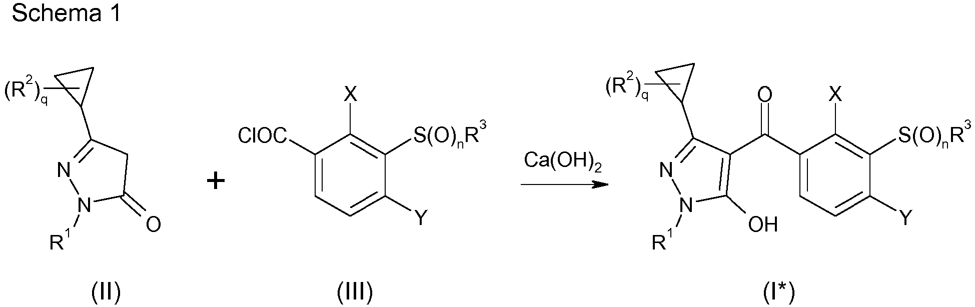

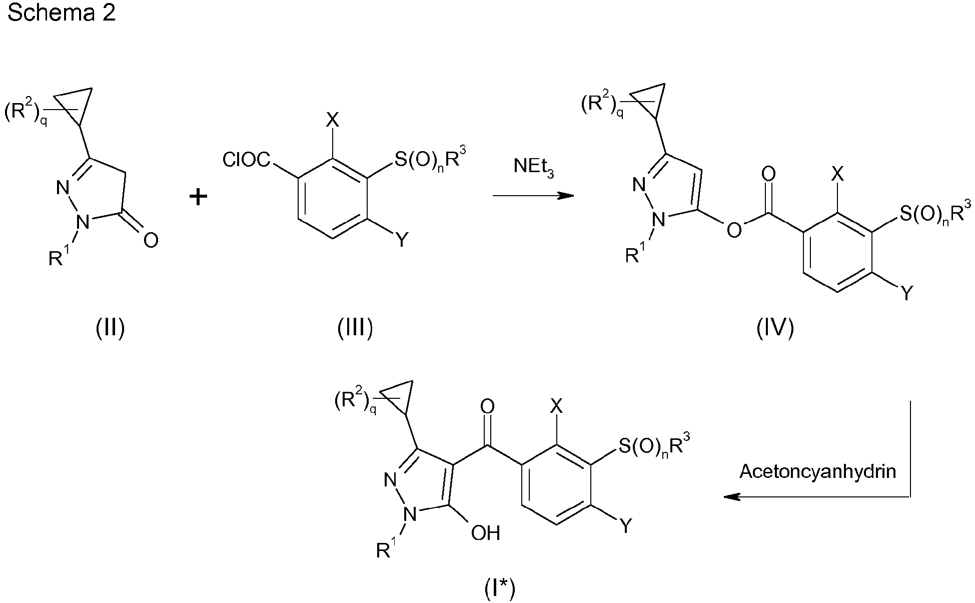

Erfindungsgemäße Verbindungen, in denen R⁴ eine andere Bedeutung als Wasserstoff hat, werden gemäß Schema 3 zweckmäßigerweise aus den nach Schema 1 oder 2 erhältlichen Verbindungen durch basenkatalysierte Reaktion mit einem geeigneten Acylierungsmittel R⁴-Z der Formel (V), worin Z für eine Abgangsgruppe wie Halogen steht, hergestellt. Solche Methoden sind dem Fachmann grundsätzlich bekannt und beispielsweise in DOS 25 13 750 beschrieben.

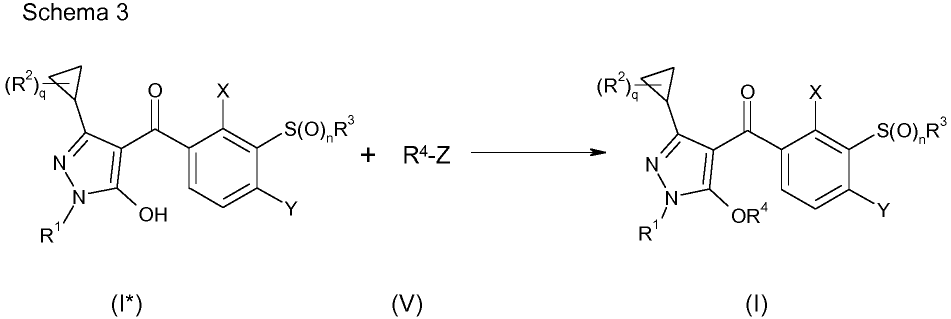

Erfindungsgemäße Verbindungen können auch gemäß dem in Schema 4 angegebenen und aus WO 98/42678 bekannten Verfahren durch Umsetzung eines Pyrazolons (II) und eines Halogen-Benzoesäurechlorids (IIIa), anschließender nucleophiler aromatischer Substitution durch eine Thioverbindung HS-R³ und gegebenenfalls Oxidation der Thiogruppe hergestellt werden. Darin bedeutet L beispielsweise Chlor, Brom, Iod oder Trifluormethylsulfonyl. Solche Substitutionsreaktionen sind dem Fachmann bekannt und beispielsweise in Houben-Weyl, Methoden der Organischen Chemie, Georg Thieme Verlag Stuttgart, Bd. E 11, Erweiterungs- und Folgebände zur vierten Auflage 1985, S. 174 ff. beschrieben.

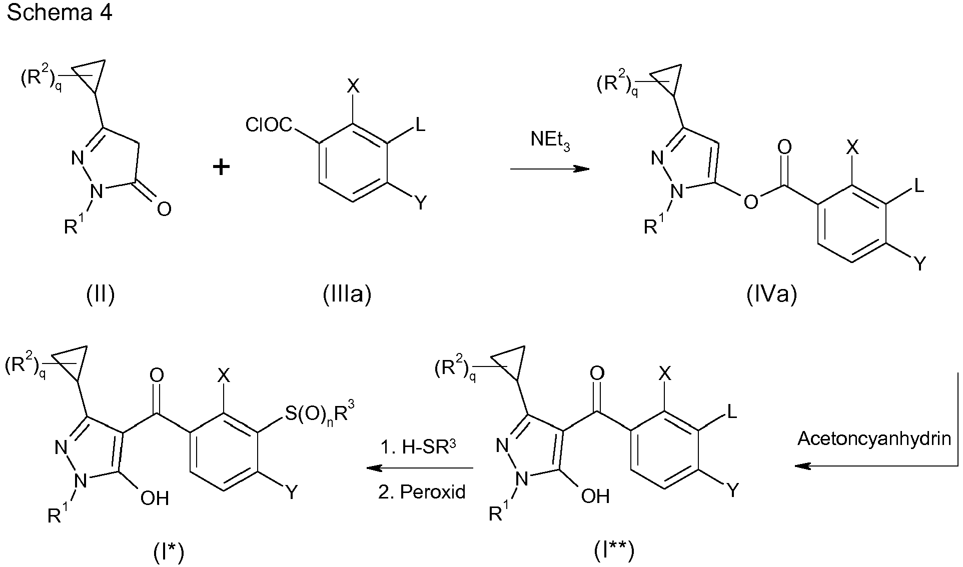

Die oben genannten Verbindungen der Formel (III) können beispielsweise durch Umsetzung mit Säurechloriden wie Thionylchlorid aus den entsprechenden Benzoesäuren der Formel (IIIb) gemäß dem Fachmann bekannten Methoden hergestellt werden.

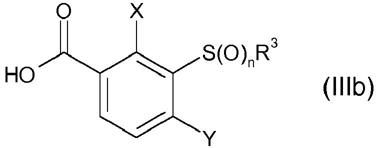

Verbindungen der Formel (IIIb) können beispielsweise gemäß Schema 6 hergestellt werden: Dazu wird in einem ersten Schritt ein 3-Aminoderivat der Formel (IIIc) diazotiert und anschließend mit Kaliumethylxanthogenat und nachfolgender Verseifung zu einem 3-Thioderivat der Formel (IIId) umgesetzt. Solche Reaktionen sind beispielsweise beschrieben in Houben-Weyl, Methoden der Organischen Chemie, Georg Thieme Verlag Stuttgart, Bd. E 9, vierte Auflage 1955, S. 12 ff.
In einem zweiten Schritt können die 3-Thioderivate der Formel (IIId) mit Alkylierungsreagenzien durch nucleophile Substitution an einem gesättigten Kohlenstoff-Atom oder über eine konjugierte Addition an ein acceptor-substituiertes Olefin zu einem Derivat der Formel (IIIe) alkyliert werden. Solche Reaktionen sind beispielsweise beschrieben in Houben-Weyl, Methoden der Organischen Chemie, Georg Thieme Verlag Stuttgart, Bd. E 11, Erweiterungs- und Folgebände zur vierten Auflage 1985, S. 165 ff. Eine nachfolgende Oxidation der Verbindungen der Formel (IIIe) führt zu solchen der Formel (IIIb), worin n für 1 oder 2 steht.

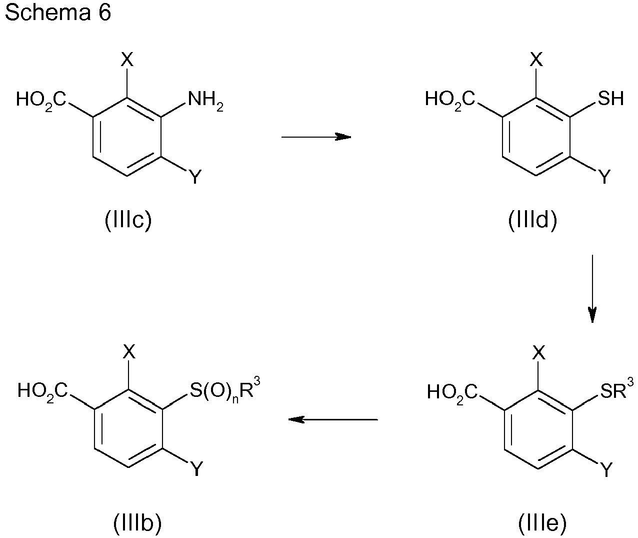

Die in obigen Schemata verwendeten Ausgangsverbindungen sind entweder käuflich oder nach an sich bekannten Methoden herstellbar. So können die Pyrazolone der Formel (II) beispielsweise nach den in EP-A 0 240 001 und J. Prakt. Chem. 315, 382, (1973) beschriebenen Methoden und die Benzoesäuren der Formel (III) sowie die Benzoylchloride der Formel (IIIa) nach den in EP-A 0 527 036 und WO 03/014071 beschriebenen Verfahren hergestellt werden.
Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Apera spica venti, Chenopodium album, Lamium purpureum, Polygonum convulvulus, Stellaria media, Veronica hederifolia, Veronica persica und Viola tricolor.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Getreide, wie Weizen, Gerste und Mais, insbesondere Weizen, auf.
Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzen-kulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z.B. WO 92/11376, WO 92/14827, WO 91/19806),
- transgene Kulturpflanzen, welche gegen bestimmte Herbizide vom Typ Glufosinate (vgl. z.B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 14th edition, The British Crop Protection Council and the Royal Soc. of Chemistry, 2006 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone; CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flucarbazone; flufenacet; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; foramsulfuron; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; iodosulfuron-methyl-natrium; isocarbamid; isopropalin; isoproturon; isouron; isoxaben; isoxapyrifop; karbutilate; lactofen; lenacil; linuron; MCPA; MCPB; mecoprop; mefenacet; mefluidid; mesosulfuron; mesotrione; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; pinoxaden; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propoxycarbazone; propyzamide; prosulfalin; prosulfocarb; prosulfuron (CGA-152005); prynachlor; pyrazolinate; pyrazon; pyrasulfotole; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulcotrione; sulfentrazon (FMC-97285, F-6285); sulfazuron; sulfometuron-methyl; sulfosate (ICI-A0224); TCA; tebutam (GCP-5544); tebuthiuron; tembotrione; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiencarbazone; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1 H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung von 3-Cyclopropyl-4-(3-cyclopropylmethylthio-2-methyl-4-methylsulfonylbenzoyl)-5-hydroxy-1-methylpyrazol (Beispiel-Nr. 1-38)

### Schritt 1: 3-Mercapto-2-methyl-4-methylsulfonylbenzoesäure

Raumtemperatur (RT) anstieg. Zur Aufarbeitung wurden vorsichtig 150 ml 1M HCl Eine Lösung von 2.03 g (50.9 mmol) NaOH in 60 ml Wasser wurde mit 11.0 g (48.0 mmol) 3-Amino-2-methyl-4-methylsulfonylbenzoesäure (Synthese beschrieben von T. L. Siddall et al in Pest Management Science (2002), 58 (12), 1175-1186) versetzt. Dann wurden 3.31 g (48.0 mmol) Natriumnitrit zugegeben. Die Lösung wurde bei 5 - 8 °C zu einer Mischung aus konzentrierter HCl und Eis tropfenweise gegeben. Das Gemisch wurde 15 Minuten bei dieser Temperatur nachgerührt und dann mit Natriumacetat neutralisiert. Der Inhalt wurde danach tropfenweise zu einer auf 70 - 80 °C befindlichen Lösung von 21.54 g (134.3 mmol) Kaliumethylxanthogenat in 80 ml Wasser gegeben. Das Gemisch wurde noch 15 Minuten bei 80 °C gerührt und dann bei RT zur Aufarbeitung mit 1 M HCl angesäuert. Nach fünf Minuten wurde abdekantiert, und zu dem Rückstand wurden 85 ml 10 %-ige Natronlauge gegeben. Das Gemisch wurde erhitzt, wobei mit Hilfe eines Destillationsaufsatzes entstehendes Destillat abgetrennt wurde, so dass eine Innentemperatur von 100 °C erreicht werden konnte. Nachdem das Gemisch 1.25 h lang bei dieser Temperatur erhitzt worden war zeigte eine HPLC-Analyse an, dass die Reaktion vollständig abgelaufen war. Anschließend wurden 21 ml einer gesättigten wässrigen Natriumhydrogensulfit-Lösung zugegeben und das Gemisch wurde für 10 Minuten auf 100 °C erhitzt. Zur Aufarbeitung wurde das abgekühlte Reaktionsgemisch mit 1 M HCl angesäuert, auf 0 - 5 °C abgekühlt und unter Stickstoff-Atmosphäre filtriert. Es wurden 10 g eines Feststoffs isoliert; ein 1 H-NMR-Spektrum zeigte die Identität des Produkts an.

### Schritt 2: 3-Cyclopropylmethylthio-2-methyl-4-methylsulfonylbenzoesäure

9.0 g (36.5 mmol) 3-Mercapto-2,4-dimethyl-sulfonylbenzoesäure wurden unter Stickstoff-Atmosphäre in 70 ml N,N-Dimethylformamid (DMF) gelöst und dann portionsweise mit 3.07 g (76.7 mmol, 60 Gew.-% Reinheit) NaH versetzt. Das Gemisch wurde 15 Minuten bei RT gerührt, danach wurden 5.43 g (40.2 mmol) Cyclopropylmethylbromid langsam zugetropft. Das Gemisch wurde 16 h bei RT gerührt. Zur Aufarbeitung wurde unter Vakuum das Lösungsmittel entfernt, und der Rückstand wurde in einem Gemisch von Wasser und Methanol aufgenommen. Es wurden 8 g (200 mmol) NaOH zugegeben, und das Reaktionsgemisch wurde so lange bei RT gerührt, bis eine HPLC-Analyse keinen Cyclopropylmethylester mehr anzeigte. Das Gemisch wurde von den Lösungsmitteln befreit, Wasser wurde zum Rückstand gegeben, die wässrige Phase wurde mit 1M HCl angesäuert und anschließend zweimal mit Essigsäureethylester (EE) extrahiert. Die vereinigten organischen Phasen wurden getrocknet, filtriert und vom Lösungsmittel befreit. Der Rückstand wurde mit n-Heptan gewaschen. Das Heptan wurde abdekantiert und der Rückstand unter Vakuum getrocknet. Es wurden 11.1 g des reinen Produkts isoliert.

### Schritt 3: 3-Cyclopropyl-4-(3-cyclopropylmethylthio-2-methyl-4-methylsulfonyl benzoyl)-5-hydroxy-1-methylpyrazol

190 mg (0.63 mmol) 3-Cyclopropylmethylthio-2-methyl-4-methylsulfonylbenzoesäure wurden in 15 ml trockenem Dichlormethan vorgelegt und mit 121 mg (0.95 mmol) Oxalsäuredichlorid sowie einigen Tropfen DMF versetzt. Das Gemisch wurde für 15 min. auf Rückfluss erhitzt, danach war keine Gasabspaltung mehr zu beobachten. Der Inhalt wurde auf RT abgekühlt und eingeengt. Das so erhaltene Säurechlorid wurde in 15 ml Acetonitril gelöst und mit 96 mg (0.70 mmol) 3-Cyclopropyl-5-hydroxy-1-methylpyrazol versetzt. Danach wurden 128 mg (1.27 mmol) Triethylamin langsam zugetropft, und das Reaktionsgemisch wurde 16 h bei RT gerührt. Zu dem so erhaltenen Enolester wurden 10 Tropfen Acetoncyanhydrin sowie eine Spatelspitze KCN gegeben. Das Gemisch wurde 16 h bei RT gerührt und wurde dann eingeengt. Der Rückstand wurde mit 15 ml Dichlormethan und anschließend mit 2 ml 1M HCl versetzt. Nach der Phasentrennung wurde die organische Phase vom Lösungsmittel befreit. Der Rückstand wurde chromatographisch gereinigt, wobei 65.7 mg sauberes Produkt isoliert wurden.

### Herstellung von 3-Cyclopropyl-4-(3-cyclopropylmethylsulfonyl-2-methyl-4-methylsulfonylbenzoyl)-5-hydroxy-1-methylpyrazol (Beispiel-Nr. 1-50)

### Schritt 1: 3-Cyclopropylmethylsulfonyl-2-methyl-4-methylsulfonylbenzoesäure

952 mg (3.17 mmol) 3-Cyclopropylmethylthio-2-methyl-4-methylsulfonylbenzoesäure wurden in 15 ml Eisessig gelöst. 31 mg (0.095 mmol) Natriumwolframat(VI)dihydrat wurden zugegeben, danach wurde das Gemisch auf 60 °C erhitzt. Bei dieser Temperatur wurden vorsichtig 1.44 g (30 %-ig, 12.7 mmol) einer wäßrigen Wasserstoffperoxid-Lösung tropfenweise zugegeben. Das Gemisch wurde zwei Tage bei dieser Temperatur gerührt. Dann wurde abgekühlt und zur Aufarbeitung auf Wasser gegossen. Das Gemisch wurde zweimal mit EE extrahiert, die vereinigten organischen Phasen wurden mit einer wässrigen gesättigten Natriumhydrogensulfit-Lösung gewaschen, und nach dem analytischen Nachweis der Abwesenheit von Peroxiden wurde das Gemisch getrocknet, filtriert und unter Vakuum von den Lösungsmitteln befreit. 744 mg des Produkts wurden isoliert.

### Schritt 2: Synthese von 3-Cyclopropyl-4-(3-cyclopropylmethylsulfonyl-2-methyl-4-methylsulfonylbenzoyl)-5-hydroxy-1-methylpyrazol

149 mg (0.45 mmol) 3-Cyclopropylmethylsulfonyl-2-methyl-4-methylsulfonylbenzoesäure wurden in 15 ml trockenem CH₂Cl₂ vorgelegt und mit 114 mg (0.90 mmol) Oxalsäuredichlorid sowie einigen Tropfen DMF versetzt. Das Gemisch wurde für 15 min. auf Rückfluss erhitzt, danach war keine Gasabspaltung mehr zu beobachten. Der Inhalt wurde auf RT abgekühlt und eingeengt. Das so erhaltene Säurechlorid wurde in 15 ml trockenem Dichlormethan gelöst und mit 68 mg (0.49 mmol) 3-Cyclopropyl-5-hydroxy-1-methylpyrazol versetzt. Danach wurden 91 mg (0.90 mmol) Triethylamin langsam zugetropft, und das Reaktionsgemisch wurde 16 h bei RT gerührt. Zur Aufarbeitung wurden 2 ml 1M HCl zugegeben, und nach der Phasentrennung wurde die organische Phase vom Lösungsmittel befreit. Der so erhaltene Enolester wurde in 15 ml Acetonitril aufgenommen und mit 10 Tropfen Acetoncyanhydrin sowie einer Spatelspitze KCN versetzt. Das Gemisch wurde 16 h bei RT gerührt und wurde dann eingeengt. Der Rückstand wurde mit 15 ml CH₂Cl₂ und anschließend mit 2 ml 1M HCl versetzt. Nach der Phasentrennung wurde die organische Phase vom Lösungsmittel befreit. Der Rückstand wurde chromatographisch gereinigt, wobei 103 mg sauberes Produkt isoliert wurden.

### Herstellung von 4-(2-Chlor-3-(2-methoxyethyl)thio-4-methylsulfonylbenzoyl)-3-cyclopropyl-5-hydroxy-1-methylpyrazol (Beispiel-Nr. 1-3)

### Schritt 1: Synthese von 2-Chlor-3-(2'-methoxyethyl)thio-4-methylsulfonylbenzoesäure

5.0 g (19.8 mmol) 2-Chlor-3-fluor-4-methylsulfonylbenzoesäure (Synthese beschrieben in WO 98/42648) wurden in 40 ml DMF aufgenommen. Es wurden 871 mg (21.8 mmol, 60 Gew.-% Reinheit) NaH zugegeben. Das Gemisch wurde 30 Minuten bei RT gerührt. Danach wurde ein Reaktionsgemisch, das das Natriumsalz von 2-Methoxyethanthiol enthält (hergestellt aus einer Lösung von 2.19 g (23.7 mmol) 2-Methoxyethanthiol in 10 ml DMF, welche tropfenweise zu einer Suspension von 950 mg (23.7 mmol, 60 Gew.-% Reinheit) NaH in 30 ml DMF gegeben wurde, danach 30 Minuten bei RT gerührt), portionsweise zugegeben. Dabei wurde die Temperatur auf unter 30 °C gehalten. Das Reaktionsgemisch wurde 16 h bei RT gerührt, zur Aufarbeitung mit Wasser verdünnt und mit Diethylether gewaschen. Die wässrige Phase wurde mit 1 M HCl angesäuert und mit tert-Butylmethylether extrahiert. Die organische Phase wurde getrocknet und filtriert. Zusätzlich wurde die wässrige Phase mit EE extrahiert, und die organische Phase wurde ebenfalls getrocknet und filtriert. Die Filtrate der organischen Phasen wurden vereinigt und vom Lösungsmittel befreit. Der Rückstand wurde unter Vakuum getrocknet und chromatographisch gereinigt. Es wurden 4.8 g des sauberen Produkts erhalten.

### Schritt 2: Synthese von 4-(2-Chlor-3-(2-methoxyethyl)thio-4-methylsulfonylbenzoyl)-3-cyclopropyl-5-hydroxy-1-methylpyrazol

550 mg (1.69 mmol) 2-Chlor-3-(2-methoxyethyl)thio-4-methylsulfonylbenzoesäure wurden in 20 ml trockenem CH₂Cl₂ vorgelegt und mit 430 mg (3.39 mmol) Oxalsäuredichlorid sowie zwei Tropfen DMF versetzt. Das Gemisch wurde für 15 min. auf Rückfluss erhitzt. Der Inhalt wurde auf RT abgekühlt und eingeengt. Das so erhaltene Säurechlorid wurde in 20 ml trockenem CH₂Cl₂ gelöst und mit 257 mg (1.86 mmol) 3-Cyclopropyl-5-hydroxy-1-methylpyrazol versetzt. Danach wurden 343 mg (3.39 mmol) Triethylamin langsam zugetropft, und das Reaktionsgemisch wurde 16 h bei RT gerührt. Zur Aufarbeitung wurden 5 ml 1M HCl zugegeben, und nach der Phasentrennung wurde die organische Phase vom Lösungsmittel befreit. Der Rückstand wurde chromatographisch gereinigt, und der so erhaltene Enolester wurde in 20 ml Acetonitril aufgenommen und mit 343 mg (3.39 mmol) Triethylamin, acht Tropfen Acetoncyanhydrin sowie einer Spatelspitze KCN versetzt. Das Gemisch wurde 16 h bei RT gerührt und wurde dann eingeengt. Der Rückstand wurde mit 20 ml CH₂Cl₂ und dann mit 5 ml 1M HCl versetzt. Nach der Phasentrennung wurde die organische Phase vom Lösungsmittel befreit. Der Rückstand wurde chromatographisch gereinigt, wobei 579 mg sauberes Produkt isoliert wurden.

### Herstellung von 4-(2-Chlor-3-(2-methoxyethyl)sulfinyl-4-methylsulfonylbenzoyl)-3-cyclopropyl-5-hydroxy-1-methylpyrazol (Beispiel-Nr. 1-9) und 4-(2-Chlor-3-(2-methoxyethyl)sulfonyl-4-methylsulfonylbenzoyl)-3-cyclopropyl-5-hydroxy-1-methyl pyrazol (Beispiel-Nr. 1-15)

193 mg (0.43 mmol) 4-(2-Chlor-3-(2-methoxyethyl)thio-4-methylsulfonylbenzoyl)-3-cyclopropyl-5-hydroxy-1-methylpyrazol wurden in 20 ml CH₂Cl₂ gelöst und anschließend mit 321 mg (70 Gew.-% Reinheit, 1.30 mmol) Metachlorperbenzoesäure versetzt. Danach wurde das Gemisch 16 h bei RT gerührt. Zur Aufarbeitung wurde mit CH₂Cl₂ verdünnt und mit 10 %-iger wässriger Natriumhydrogensulfit-Lösung gewaschen. Der pH-Wert der wässrigen Phase wurde dabei mit 1 M HCl im sauren Bereich gehalten. Anschließend wurde nach der Phasentrennung und nach dem analytischen Nachweis der Abwesenheit von Peroxiden die organische Phase getrocknet, filtriert und vom Lösungsmittel befreit. Der Rückstand wurde chromatographisch aufgetrennt, wobei 9.6 mg sauberes 4-(2-Chlor-3-(2-methoxyethyl)sulfinyl-4-methylsulfonylbenzoyl)-3-cyclopropyl-5-hydroxy-1-methylpyrazol sowie 71.6 mg sauberes 4-(2-Chlor-3-(2-methoxyethyl)sulfonyl-4-methylsulfonylbenzoyl)-3-cyclopropyl-5-hydroxy-1-methylpyrazol erhalten wurden.

### Herstellung von 3-Cyclopropyl-5-hydroxy-4-(3-(2-methoxyethyl)thio-2-methyl-4-trifluormethylbenzoyl)-1-methylpyrazol (Beispiel-Nr. 1-2091)

### Schritt 1: Synthese von 3-Fluor-2-methyl-4-trifluormethylbenzoesäure

25.0 g (120.1 mmol) 3-Fluor-4-trifluormethylbenzoesäure wurden in 250 ml trockenem THF gelöst, und 100.9 ml (2.5M in Hexan, 252.3 mmol) n-Butyllithium wurden bei einer Temperatur von -40 °C tropfenweise zugegeben. Das Gemisch wurde 3.5 h gerührt, anschließend wurde eine Lösung von 51.2 g (360.4 mmol) Iodmethan in 50 ml trockenem THF zugegeben. Das Gemisch wurde 16 h gerührt, wobei nach einer halben Stunde die Temperatur langsam auf RT anstieg. Zur Aufarbeitung wurden vorsichtig 150 ml 1M HCl zugegeben. Das Gemisch wurde mit Diethylether extrahiert, anschließend wurde die organische Phase mit 1 M NaOH extrahiert. Die wässrige Phase wurde angesäuert und danach mit Diethylether extrahiert. Die organische Phase wurde mit Wasser gewaschen, getrocknet und eingeengt. Der Rückstand wurde mit n-Heptan verrührt, und der Feststoff wurde über eine Filtration abgetrennt. 13.5 g des sauberen Produkts wurden isoliert.

### Schritt 2: Synthese von 3-(2-Methoxyethyl)thio-2-methyl-4-trifluormethyl benzoesäure

1.45 g (6.53 mmol) 3-Fluor-2-methyl-4-trifluormethylbenzoesäure wurden in 40 ml DMF vorgelegt. 809 mg (20.2 mmol) NaH wurden portionsweise zugegeben. Gegen Beendigung der Gasabspaltung wurden 1.20 g (13.1 mmol) 2-Methoxyethanthiol tropfenweise zugegeben. Das Gemisch wurde 10 Minuten lang bei RT gerührt und danach 15 h lang auf 80 °C erhitzt. Das Reaktionsgemisch wurde abgekühlt, unter Vakuum eingeengt, zur Aufarbeitung wurde Wasser zugegeben und mit 1 M HCl angesäuert. Das Produkt fiel aus und wurde über eine Filtration abgetrennt. Das Produkt wurde anschließend mit Wasser und n-Heptan gewaschen. 1.7 g des sauberen Produkts wurden isoliert.

### Schritt 3: Synthese von 3-Cyclopropyl-5-hydroxy-4-(3-(2-methoxyethyl)thio-2-methyl-4-trifluormethylbenzoyl)-1-methylpyrazol

520 mg (1.77 mmol) 3-(2-Methoxyethyl)thio-2-methyl-4-trifluormethylbenzoesäure wurden in 20 ml trockenem CH₂Cl₂ vorgelegt und mit 449 mg (3.53 mmol) Oxalsäuredichlorid sowie zwei Tropfen DMF versetzt. Das Gemisch wurde für 15 min. auf Rückfluss erhitzt, danach war keine Gasabspaltung mehr zu beobachten. Der Inhalt wurde auf RT abgekühlt und eingeengt. Das so erhaltene Säurechlorid wurde in 20 ml trockenem CH₂Cl₂ gelöst und mit 269 mg (1.94 mmol) 3-Cyclopropyl-5-hydroxy-1-methylpyrazol versetzt. Danach wurden 358 mg (3.53 mmol) Triethylamin langsam zugetropft, und das Reaktionsgemisch wurde 16 h bei RT gerührt. Zur Aufarbeitung wurden 5 ml 1 M HCl zugegeben und nach der Phasentrennung wurde vom Lösungsmittel befreit. Der so erhaltene Enolester wurde nach einer chromatographischen Reinigung in 20 ml Acetonitril aufgenommen, und es wurden 358 mg (3.53 mmol) Triethylamin zugegeben. Anschließend wurden acht Tropfen Acetoncyanhydrin sowie eine Spatelspitze KCN zugegeben. Das Gemisch wurde 16 h bei RT gerührt und wurde dann eingeengt. Der Rückstand wurde mit 20 ml CH₂Cl₂ und anschließend mit 5 ml 1M HCl versetzt. Nach der Phasentrennung wurde vom Lösungsmittel befreit. Der Rückstand wurde chromatographisch gereinigt, wobei 354 mg sauberes Produkt isoliert wurden.

### Herstellung von 4-(4-Chlor-3'-cyclopropylmethylthio-2-methylbenzoyl)-3-cyclopropyl-5-hydroxy-1-methylpyrazol (Beispiel-Nr. 1-1406)

### Schritt 1: Synthese von 4-Chlor-3-(dimethylaminothiocarbonyloxy)-2-methylbenzoesäuremethylester

11.0 g (54.8 mmol) 4-Chlor-3-hydroxy-2-methylbenzoesäuremethylester (Synthese beschrieben in DE 10039723) wurden unter Stickstoff-Atmosphäre in 200 ml DMF mit 12.3 g (109.7 mmol) 1,4-Diazabicyclo[2.2.2]octan und anschließend mit 13.6 g (109.7 mmol) Dimethylaminothiocarbonylchlorid versetzt. Das Gemisch wurde 16 h bei RT gerührt, zur Aufarbeitung wurde es auf Eiswasser gegossen. Das Produkt fiel aus und wurde über eine Filtration abgetrennt. Der Rückstand wurde mit 1 M HCl gewaschen. Es wurden 14.7 g sauberes Produkt erhalten.

### Schritt 2: Synthese von 4-Chlor-3-(dimethylaminocarbonylthio)-2-methylbenzoesäuremethylester

12.1 g (42.0 mmol) 4-Chlor-3-(dimethylaminothiocarbonyloxy)-2-methylbenzoesäure-methylester wurden unter Stickstoff-Atmosphäre in 30 ml 1,3-Dimethoxybenzol 6 h lang auf 220°C erhitzt. Zur Aufarbeitung wurde das Reaktionsgemisch abgekühlt und unter Vakuum eingeengt. Nach chromatographischer Reinigung des Rückstands wurden 5.2 g sauberes Produkt isoliert.

### Schritt 3: Synthese von 4-Chlor-3-mercapto-2-methylbenzoesäure

4.80 g (16.7 mmol) 4-Chlor-3-(dimethylaminocarbonylthio)-2-methylbenzoesäuremethylester wurden in 150 ml Methanol mit 6.61 g (85 Gew.-% Reinheit, 100.1 mmol) KOH versetzt und unter Rückfluss zwei Tage gerührt. Das Reaktionsgemisch wurde vom Lösungsmittel befreit, und der Rückstand wurde mit Wasser versetzt, danach mit 1 M HCl angesäuert, und der Feststoff wurde über eine Filtration abgetrennt. Es wurden 3.2 g sauberes Produkt erhalten.

### Schritt 4: Synthese von 4-Chlor-3-mercapto-2-methylbenzoesäuremethylester

3.60 g (17.8 mmol) 4-Chlor-3-mercapto-2-methylbenzoesäure wurden unter Stickstoff-Atmosphäre in 50 ml absolutem Methanol mit 1 ml konzentrierter Schwefelsäure 17 h lang unter Rückfluss erhitzt. Das Gemisch wurde vom Lösungsmittel befreit, und der Rückstand wurde in Wasser aufgenommen. Nach zweimaliger Extraktion mit Essigsäureethylester wurden die vereinigten organischen Phasen getrocknet, unter Stickstoff-Atmosphäre filtriert und vom Lösungsmittel befreit. Es wurden 3.2 g sauberes Produkt isoliert.

### Schritt 5: Synthese von 4-Chlor-3-cyclopropylmethylthio-2-methylbenzoesäuremethylester

1.05 g (4.85 mmol) 4-Chlor-3-mercapto-2-methylbenzoesäuremethylester wurden mit 1.66 g (5.09 mmol) Caesiumcarbonat in 20 ml Acetonitril versetzt. 687 mg (5.09 mmol) Cyclopropylmethylbromid wurden langsam zugetropft, und das Reaktionsgemisch wurde 16 h bei RT gerührt. Zur Aufarbeitung wurde das Lösungsmittel abgetrennt, und der Rückstand wurde mit Wasser versetzt. Das Gemisch wurde dreimal mit Essigsäureethylester extrahiert, die vereinigten organischen Phasen wurden getrocknet, filtriert, und das Lösungsmittel wurde entfernt. Es wurden 1.2 g sauberes Produkt isoliert.

### Schritt 6: Synthese von 4-Chlor-3-cyclopropylmethylthio-2-methylbenzoesäure

1.20 g (4.43 mmol) 4-Chlor-3-cyclopropylmethylthio-2-methylbenzoesäuremethylester wurden in 30 ml Methanol mit 3 ml 20 proz. Natronlauge versetzt und 16 h bei RT gerührt. Zur Aufarbeitung wurde das Gemisch am Rotationsverdampfer eingeengt, und der Rückstand wurde in Wasser aufgenommen. Es wurde mit 1 M HCl angesäuert, und das Produkt wurde anschließend als Fesstoff abfiltriert. Es wurden 1.1 g sauberes Produkt erhalten.

### Schritt 7: Synthese von 4-(4'-Chlor-3'-cyclopropylmethylthio-2'-methylbenzoyl)-3-cyclopropyl-5-hydroxy-1-methylpyrazol

272 mg (1.06 mmol) 4-Chlor-3-cyclopropylmethylthio-2-methylbenzoesäure wurden in 20 ml Dichlormethan mit 161 mg (1.17 mmol) 3-Cyclopropyl-5-hydroxy-1-methylpyrazol sowie wenigen Tropfen N,N-4-Dimethylaminopyridin versetzt. 244 mg (1.27 mmol) 1-(3-Dimethylaminopropyl)-3-ethylcarbodiimidhydrochlorid wurden zugegeben, und das Gemisch wurde 16 h bei RT gerührt. Zur Aufarbeitung wurden 3ml 1M HCl zugegeben, und die organische Phase wurde vom Lösungsmittel befreit. Der Rückstand wurde in 15 ml Acetonitril mit 214 mg (2.12 mmol) Triethylamin, 10 Tropfen Acetoncyanhydrin sowie einer Spatelspitze KCN versetzt. Das Reaktionsgemisch wurde 16 h bei RT und anschließend vom Lösungsmittel befreit. Der Rückstand wurde in 15 ml CH₂Cl₂ mit 2 ml 1 M HCl versetzt. Die organische Phase wurde vom Lösungsmittel befreit, und der Rückstand wurde danach chromatographisch gereinigt. Es wurden 154 mg sauberes Produkt erhalten.

### Herstellung von 4-(4-Chlor-3-cyclopropylmethylsulfonyl-2-methylbenzoyl)-3-cyclopropyl-5-hydroxy-1-methylpyrazol (Beispiel-Nr. 1-1418)

78 mg (0.21 mmol) 4-(4-Chlor-3-cyclopropylmethylthio-2-methylbenzoyl)-3-cyclopropyl-5-hydroxy-1-methylpyrazol wurden in 20 ml CH₂Cl₂ mit 128 mg (70 Gew.-% Reinheit, 0.52 mmol) Metachlorperbenzoesäure versetzt. Danach wurde das Gemisch 5 h bei RT gerührt. Zur Aufarbeitung wurde das Gemisch mit 10 %-iger wässriger Natriumhydrogensulfit-Lösung gewaschen. Der pH-Wert der wässrigen Phase wurde dabei im sauren Bereich gehalten, andernfalls wurde mit 1 M HCl angesäuert. Anschließend wurde nach der Phasentrennung und nach dem analytischen Nachweis der Abwesenheit von Peroxiden die organische Phase getrocknet, filtriert und vom Lösungsmittel befreit. Der Rückstand wurde chromatographisch gereinigt, wobei 29.1 mg sauberes Produkt erhalten wurden.

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:

| | | | | | | | |
|---|---|---|---|---|---|---|---|
| Bu | = Butyl | Et | = Ethyl | Me | = Methyl | Pr | = Propyl |
| c | = cyclo | i | = iso | | | Ph | = Phenyl |

**Tabelle A: Erfindungsgemäße Verbindungen der allgemeinen Formel (I), worin R¹ für Methyl, R⁴ für Wasserstoff und q für null stehen**

| | | | | | |
|---|---|---|---|---|---|
| 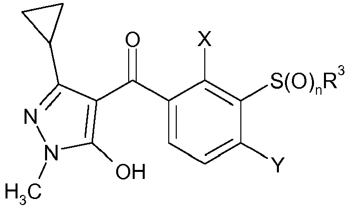 | | | | | |

| **Nr.** | **X** | **R³** | **n** | **Y** | **Physikalische Daten:** ¹H-NMR: δ [CDCl₃] |
|---|---|---|---|---|---|
| 1-1 | | | | | |
| 1-2 | Cl | CH₂-c-Pr | 0 | SO₂Me | |
| 1-3 | Cl | (CH₂)₂OMe | 0 | SO₂Me | 8.23 (d, 1H), 7.51 (d, 1H), 3.64 (t, 2H), 3.62 (s, 3H), 3.49 (s, 3H), 3.29 (s, 3H), 3.24 (t, 2H), 0.91 (m, 1H), 0.76 (m, 2H), 0.44 (m, 2H) |
| 1-4 | Cl | (CH₂)₃OMe | 0 | SO₂Me | |
| 1-5 | Cl | (CH₂)₂OEt | 0 | SO₂Me | |
| 1-6 | Cl | (CH₂)₂OCH₂-c-Pr | 0 | SO₂Me | |
| 1-7 | | | | | |
| 1-8 | Cl | CH₂-c-Pr | 1 | SO₂Me | |
| 1-9 | Cl | (CH₂)₂OMe | 1 | SO₂Me | 8.22 (d, 1H), 7.62 (d, 1H), 4.09-4.01 (m, 2H), 3. 91-3.85 (m, 1H), 3.62 (s, 3H), 3.50 - 3.42 (m, 7H), 0.92 (m, 1H), 0.78 (m, 2H), 0.47 (m, 2H) |
| 1-10 | Cl | (CH₂)₃OMe | 1 | SO₂Me | |
| 1-11 | Cl | (CH₂)₂OEt | 1 | SO₂Me | |
| 1-12 | Cl | (CH₂)₂OCH₂-c-Pr | 1 | SO₂Me | |
| 1-13 | | | | | |
| 1-14 | Cl | CH₂-c-Pr | 2 | SO₂Me | |
| 1-15 | Cl | (CH₂)₂OMe | 2 | SO₂Me | 8.43 (d, 1H), 7.72 (d, 1H), 4.02 (t, |
| | | | | | 2H), 3.92 (t, 2H), 3.62 (ss, 6H), 3.29 (s, 3H), 0.92 (m, 1H), 0.78 (m, 2H), 0.65 - 0.42 (m, 2H) |
| 1-16 | Cl | (CH₂)₃OMe | 2 | SO₂Me | |
| 1-17 | Cl | (CH₂)₂OEt | 2 | SO₂Me | |
| 1-18 | Cl | (CH₂)₂OCH₂-c-Pr | 2 | SO₂Me | |
| 1-19 | | | | | |
| 1-20 | Br | CH₂-c-Pr | 0 | SO₂Me | |
| 1-21 | Br | (CH₂)₂OMe | 0 | SO₂Me | |
| 1-22 | Br | (CH₂)₃OMe | 0 | SO₂Me | |
| 1-23 | Br | (CH₂)₂OEt | 0 | SO₂Me | |
| 1-24 | Br | (CH₂)₂OCH₂-c-Pr | 0 | SO₂Me | |
| 1-25 | | | | | |
| 1-26 | Br | CH₂-c-Pr | 1 | SO₂Me | |
| 1-27 | Br | (CH₂)₂OMe | 1 | SO₂Me | |
| 1-28 | Br | (CH₂)₃OMe | 1 | SO₂Me | |
| 1-29 | Br | (CH₂)₂OEt | 1 | SO₂Me | |
| 1-30 | Br | (CH₂)₂OCH₂-c-Pr | 1 | SO₂Me | |
| 1-31 | | | | | |
| 1-32 | Br | CH₂-c-Pr | 2 | SO₂Me | |
| 1-33 | Br | (CH₂)₂OMe | 2 | SO₂Me | |
| 1-34 | Br | (CH₂)₃OMe | 2 | SO₂Me | |
| 1-35 | Br | (CH₂)₂OEt | 2 | SO₂Me | |
| 1-36 | Br | (CH₂)₂OCH₂-c-Pr | 2 | SO₂Me | |
| 1-37 | | | | | |
| 1-38 | Me | CH₂-c-Pr | 0 | SO₂Me | 8.17 (d, 1H), 7.47 (d, 1H), 3.62 (s, 3H), 3.48 (s, 3H), 2.83 (d, 2H), 2.67 (s, 3H), 1.02 (m, 1H), 0.90 - 0.82 (m, 1H), 0.77 (m, 2H), 0.57 (m, 2H), 0.48 (m, 2H), 0.23 (m, 2H) |
| 1-39 | Me | (CH₂)₂OMe | 0 | SO₂Me | 8.19 (d, 1H), 7.48 (d, 1H), 3.63-3.60 (s + t, 5H), 3.47 (s, 3H), 3.34 (s, 3H), 3.12 (t, 2H), 2.63 (s, 3H), 0.91 - 0.84 (m, 1H), 0.76 (m, 2H), 0.48 (m, 2H) |
| 1-40 | Me | (CH₂)₃OMe | 0 | SO₂Me | 8.17 (d, 1H), 7.47 (d, 1H), 3.61 (s, 3H), 3.47 (t, 2H), 3.46 (s, 3H), 3.32 (s, 3H), 2.96 (t, 2H), 2.64 (s, 3H), 1.92 (quint., 2H), 0.89-0.82 (m, 1H), 0.77 (m, 2H), 0.47 (m, 2H) |
| 1-41 | Me | (CH₂)₂OEt | 0 | SO₂Me | 8.18 (d, 1H), 7.48 (d, 1H), 3.62 (t, 2H), 3.61 (s, 3H), 3.48 (s, 3H), 3.47 (q, 2H), 3.10 (t, 2H), 2.65 (s, 3H), 1.18 (t, 3H), 0.86 (m, 1H), 0.77 (m, 2H), 0.48 (m, 2H) |
| 1-42 | Me | (CH₂)₂OCH₂-c-Pr | 0 | SO₂Me | |
| 1-43 | | | | | |
| 1-44 | Me | CH₂-c-Pr | 1 | SO₂Me | |
| 1-45 | Me | (CH₂)₂OMe | 1 | SO₂Me | 8.10 (d, 1H), 7.59 (d, 1H), 4.04-4.00 (m, 1H), 3.91 - 3.86 (m, 1H), 3.75 - 3.45 (m, 2H), 3.61 (s, 3H), 3.43 (s, 3H), 3.38 (s, 3H), 2.78 (s, 3H), 0.90 - 0.71 (m, 3H), 0.56 (m, 1H), 0.43 (m, 1H) |
| 1-46 | Me | (CH₂)₃OMe | 1 | SO₂Me | 8.08 (d, 1H), 7.57 (d, 1H), 3.62-3.56 (m, 6H), 3.38 - 3.25 (m, 1H), 3.37 (s, 3H), 3.35 (s, 3H), 2.81 (s, 3H), 2.28 - 2.16 (m, 2H), 0.90 - 0.72 (m, 3H), 0.55 (m, 1H), 0.45 (m, 1H) |
| 1-47 | Me | (CH₂)₂OEt | 1 | SO₂Me | 8.11 (d, 1H), 7.57 (d, 1H), 4.02 (m, 1H), 3.92 (m, 1H), 3.66 - 3.53 (m, 4H), 3.60 (s, 3H), 3.38 (s, 3H), 2.78 (s, 3H), 1.22 (t, 3H), 0.90 - 0.72 (m, 3H), 0.55 (m, 1H), 0.45 (m, 1H) |
| 1-48 | Me | (CH₂)₂OCH₂-c-Pr | 1 | SO₂Me | |
| 1-49 | | | | | |
| 1-50 | Me | CH₂-c-Pr | 2 | SO₂Me | 8.37 (d, 1H), 7.67 (d, 1H), 3.65-3.61 (s + d, 5H), 3.57 (s, 3H), 2.80 (s, 3H), 1.30 (m, 1H), 0.88 - 0.70 (m, 5H), 0.52 (m, 2H), 0.42 (m, 2H) |
| 1-51 | Me | (CH₂)₂OMe | 2 | SO₂Me | 8.37 (d, 1H), 7.66 (d, 1H), 4.06 - 3.90 (m, 4H), 3.62 (s, 3H), 3.56 (s, 3H), 3.33 (s, 3H), 2.74 (s, 3H), 0.89 - 0.80 (m, 1H), 0.76 (m, 2H), 0.52 (m, 2H) |
| 1-52 | Me | (CH₂)₃OMe | 2 | SO₂Me | 8.37 (d, 1H), 7.67 (d, 1H), 3.71 (t, 2H), 3.61 (s, 3H), 3.57 (s + t, 5H), 3.33 (s, 3H), 2.73 (s, 3H), 2.27 (quint., 2H), 0.86-0.76 (m, 3H), 0.55 - 0.50 (m, 2H) |
| 1-53 | Me | (CH₂)₂OEt | 2 | SO₂Me | 8.36 (d, 1H), 7.67 (d, 1H), 4.02 (t, 2H), 3.92 (t, 2H), 3.61 (s, 3H), 3.56 (s, 3H), 3.50 (q, 2H), 2.76 (s, 3H), 1.10 (t, 3H), 0.85 - 0.75 (m, 3H), 0.52 (m, 2H) |
| 1-54 | Me | (CH₂)₂OCH₂-c-Pr | 2 | SO₂Me | |
| 1-55 | | | | | |
| 1-56 | Et | CH₂-c-Pr | 0 | SO₂Me | |
| 1-57 | Et | (CH₂)₂OMe | 0 | SO₂Me | |
| 1-58 | Et | (CH₂)₃OMe | 0 | SO₂Me | |
| 1-59 | Et | (CH₂)₂OEt | 0 | SO₂Me | |
| 1-60 | Et | (CH₂)₂OCH₂-c-Pr | 0 | SO₂Me | |
| 1-61 | | | | | |
| 1-62 | Et | CH₂-c-Pr | 1 | SO₂Me | |
| 1-63 | Et | (CH₂)₂OMe | 1 | SO₂Me | |
| 1-64 | Et | (CH₂)₃OMe | 1 | SO₂Me | |
| 1-65 | Et | (CH₂)₂OEt | 1 | SO₂Me | |
| 1-66 | Et | (CH₂)₂OCH₂-c-Pr | 1 | SO₂Me | |
| 1-67 | | | | | |
| 1-68 | Et | CH₂-c-Pr | 2 | SO₂Me | |
| 1-69 | Et | (CH₂)₂OMe | 2 | SO₂Me | |
| 1-70 | Et | (CH₂)₃OMe | 2 | SO₂Me | |
| 1-71 | Et | (CH₂)₂OEt | 2 | SO₂Me | |
| 1-72 | Et | (CH₂)₂OCH₂-c-Pr | 2 | SO₂Me | |
| 1-73 | | | | | |
| 1-74 | CF₃ | CH₂-c-Pr | 0 | SO₂Me | |
| 1-75 | CF₃ | (CH₂)₂OMe | 0 | SO₂Me | |
| 1-76 | CF₃ | (CH₂)₃OMe | 0 | SO₂Me | |
| 1-77 | CF₃ | (CH₂)₂OEt | 0 | SO₂Me | |
| 1-78 | CF₃ | (CH₂)₂OCH₂-c-Pr | 0 | SO₂Me | |
| 1-79 | | | | | |
| 1-80 | CF₃ | CH₂-c-Pr | 1 | SO₂Me | |
| 1-81 | CF₃ | (CH₂)₂OMe | 1 | SO₂Me | |
| 1-82 | CF₃ | (CH₂)₃OMe | 1 | SO₂Me | |
| 1-83 | CF₃ | (CH₂)₂OEt | 1 | SO₂Me | |
| 1-84 | CF₃ | (CH₂)₂OCH₂-c-Pr | 1 | SO₂Me | |
| 1-85 | | | | | |
| 1-86 | CF₃ | CH₂-c-Pr | 2 | SO₂Me | |
| 1-87 | CF₃ | (CH₂)₂0Me | 2 | SO₂Me | |
| 1-88 | CF₃ | (CH₂)₃OMe | 2 | SO₂Me | |
| 1-89 | CF₃ | (CH₂)₂OEt | 2 | SO₂Me | |
| 1-90 | CF₃ | (CH₂)₂OCH₂-c-Pr | 2 | SO₂Me | |
| 1-91 | | | | | |
| 1-92 | OMe | CH₂-c-Pr | 0 | SO₂Me | |
| 1-93 | OMe | (CH₂)₂OMe | 0 | SO₂Me | |
| 1-94 | OMe | (CH₂)₃OMe | 0 | SO₂Me | |
| 1-95 | OMe | (CH₂)₂OEt | 0 | SO₂Me | |
| 1-96 | OMe | (CH₂)₂OCH₂-c-Pr | 0 | SO₂Me | |
| 1-97 | | | | | |
| 1-98 | OMe | CH₂-c-Pr | 1 | SO₂Me | |
| 1-99 | OMe | (CH₂)₂OMe | 1 | SO₂Me | |
| 1-100 | OMe | (CH₂)₃OMe | 1 | SO₂Me | |
| 1-101 | OMe | (CH₂)₂OEt | 1 | SO₂Me | |
| 1-102 | OMe | (CH₂)₂OCH₂-c-Pr | 1 | SO₂Me | |
| 1-103 | | | | | |
| 1-104 | OMe | CH₂-c-Pr | 2 | SO₂Me | |
| 1-105 | OMe | (CH₂)₂OMe | 2 | SO₂Me | |
| 1-106 | OMe | (CH₂)₃OMe | 2 | SO₂Me | |
| 1-107 | OMe | (CH₂)₂OEt | 2 | SO₂Me | |
| 1-108 | OMe | (CH₂)₂OCH₂-c-Pr | 2 | SO₂Me | |
| 1-109 | | | | | |
| 1-110 | OEt | CH₂-c-Pr | 0 | SO₂Me | |
| 1-111 | OEt | (CH₂)₂OMe | 0 | SO₂Me | |
| 1-112 | OEt | (CH₂)₃OMe | 0 | SO₂Me | |
| 1-113 | OEt | (CH₂)₂OEt | 0 | SO₂Me | |
| 1-114 | OEt | (CH₂)₂OCH₂-c-Pr | 0 | SO₂Me | |
| 1-115 | | | | | |
| 1-116 | OEt | CH₂-c-Pr | 1 | SO₂Me | |
| 1-117 | OEt | (CH₂)₂OMe | 1 | SO₂Me | |
| 1-118 | OEt | (CH₂)₃OMe | 1 | SO₂Me | |
| 1-119 | OEt | (CH₂)₂OEt | 1 | SO₂Me | |
| 1-120 | OEt | (CH₂)₂OCH₂-c-Pr | 1 | SO₂Me | |
| 1-121 | | | | | |
| 1-122 | OEt | CH₂-c-Pr | 2 | SO₂Me | |
| 1-123 | OEt | (CH₂)₂OMe | 2 | SO₂Me | |
| 1-124 | OEt | (CH₂)₃OMe | 2 | SO₂Me | |
| 1-125 | OEt | (CH₂)₂OEt | 2 | SO₂Me | |
| 1-126 | OEt | (CH₂)₂OCH₂-c-Pr | 2 | SO₂Me | |
| 1-127 | | | | | |
| 1-128 | NO₂ | CH₂-c-Pr | 0 | SO₂Me | |
| 1-129 | NO₂ | (CH₂)₂OMe | 0 | SO₂Me | |
| 1-130 | NO₂ | (CH₂)₃OMe | 0 | SO₂Me | |
| 1-131 | NO₂ | (CH₂)₂OEt | 0 | SO₂Me | |
| 1-132 | NO₂ | (CH₂)₂OCH₂-c-Pr | 0 | SO₂Me | |
| 1-133 | | | | | |
| 1-134 | NO₂ | CH₂-c-Pr | 1 | SO₂Me | |
| 1-135 | NO₂ | (CH₂)₂OMe | 1 | SO₂Me | |
| 1-136 | NO₂ | (CH₂)₃OMe | 1 | SO₂Me | |
| 1-137 | NO₂ | (CH₂)₂OEt | 1 | SO₂Me | |
| 1-138 | NO₂ | (CH₂)₂OCH₂-c-Pr | 1 | SO₂Me | |
| 1-139 | | | | | |
| 1-140 | NO₂ | CH₂-c-Pr | 2 | SO₂Me | |
| 1-141 | NO₂ | (CH₂)₂OMe | 2 | SO₂Me | |
| 1-142 | NO₂ | (CH₂)₃OMe | 2 | SO₂Me | |
| 1-143 | NO₂ | (CH₂)₂OEt | 2 | SO₂Me | |
| 1-144 | NO₂ | (CH₂)₂OCH₂-c-Pr | 2 | SO₂Me | |
| 1-145 | | | | | |
| 1-146 | SO₂Me | CH₂-c-Pr | 0 | SO₂Me | |
| 1-147 | SO₂Me | (CH₂)₂OMe | 0 | SO₂Me | |
| 1-148 | SO₂Me | (CH₂)₃OMe | 0 | SO₂Me | |
| 1-149 | SO₂Me | (CH₂)₂OEt | 0 | SO₂Me | |
| 1-150 | SO₂Me | (CH₂)₂OCH₂-c-Pr | 0 | SO₂Me | |
| 1-151 | | | | | |
| 1-152 | SO₂Me | CH₂-c-Pr | 1 | SO₂Me | |
| 1-153 | SO₂Me | (CH₂)₂OMe | 1 | SO₂Me | |
| 1-154 | SO₂Me | (CH₂)₃OMe | 1 | SO₂Me | |
| 1-155 | SO₂Me | (CH₂)₂OEt | 1 | SO₂Me | |
| 1-156 | SO₂Me | (CH₂)₂OCH₂-c-Pr | 1 | SO₂Me | |
| 1-157 | | | | | |
| 1-158 | SO₂Me | CH₂-c-Pr | 2 | SO₂Me | |
| 1-159 | SO₂Me | (CH₂)₂OMe | 2 | SO₂Me | |
| 1-160 | SO₂Me | (CH₂)₃OMe | 2 | SO₂Me | |
| 1-161 | SO₂Me | (CH₂)₂OEt | 2 | SO₂Me | |
| 1-162 | SO₂Me | (CH₂)₂OCH₂-c-Pr | 2 | SO₂Me | |
| 1-163 | | | | | |
| 1-164 | CH₂OMe | CH₂-c-Pr | 0 | SO₂Me | |
| 1-165 | CH₂OMe | (CH₂)₂OMe | 0 | SO₂Me | |
| 1-166 | CH₂OMe | (CH₂)₃OMe | 0 | SO₂Me | |
| 1-167 | CH₂OMe | (CH₂)₂OEt | 0 | SO₂Me | |
| 1-168 | CH₂OMe | (CH₂)₂OCH₂-c-Pr | 0 | SO₂Me | |
| 1-169 | | | | | |
| 1-170 | CH₂OMe | CH₂-c-Pr | 1 | SO₂Me | |
| 1-171 | CH₂OMe | (CH₂)₂OMe | 1 | SO₂Me | |
| 1-172 | CH₂OMe | (CH₂)₃OMe | 1 | SO₂Me | |
| 1-173 | CH₂OMe | (CH₂)₂OEt | 1 | SO₂Me | |
| 1-174 | CH₂OMe | (CH₂)₂OCH₂-c-Pr | 1 | SO₂Me | |
| 1-175 | | | | | |
| 1-176 | CH₂OMe | CH₂-c-Pr | 2 | SO₂Me | |
| 1-177 | CH₂OMe | (CH₂)₂OMe | 2 | SO₂Me | |
| 1-178 | CH₂OMe | (CH₂)₃OMe | 2 | SO₂Me | |
| 1-179 | CH₂OMe | (CH₂)₂OEt | 2 | SO₂Me | |
| 1-180 | CH₂OMe | (CH₂)₂OCH₂-c-Pr | 2 | SO₂Me | |
| 1-181 | | | | | |
| 1-182 | CH₂SO₂Me | CH₂-c-Pr | 0 | SO₂Me | |
| 1-183 | CH₂SO₂Me | (CH₂)₂OMe | 0 | SO₂Me | |
| 1-184 | CH₂SO₂Me | (CH₂)₃OMe | 0 | SO₂Me | |
| 1-185 | CH₂SO₂Me | (CH₂)₂OEt | 0 | SO₂Me | |
| 1-186 | CH₂SO₂Me | (CH₂)₂OCH₂-c-Pr | 0 | SO₂Me | |
| 1-187 | | | | | |
| 1-188 | CH₂SO₂Me | CH₂-c-Pr | 1 | SO₂Me | |
| 1-189 | CH₂SO₂Me | (CH₂)₂OMe | 1 | SO₂Me | |
| 1-190 | CH₂SO₂Me | (CH₂)₃OMe | 1 | SO₂Me | |
| 1-191 | CH₂SO₂Me | (CH₂)₂OEt | 1 | SO₂Me | |
| 1-192 | CH₂SO₂Me | (CH₂)₂OCH₂-c-Pr | 1 | SO₂Me | |
| 1-193 | | | | | |
| 1-194 | CH₂SO₂Me | CH₂-c-Pr | 2 | SO₂Me | |
| 1-195 | CH₂SO₂Me | (CH₂)₂OMe | 2 | SO₂Me | |
| 1-196 | CH₂SO₂Me | (CH₂)₃OMe | 2 | SO₂Me | |
| 1-197 | CH₂SO₂Me | (CH₂)₂OEt | 2 | SO₂Me | |
| 1-198 | CH₂SO₂Me | (CH₂)₂OCH₂-c-Pr | 2 | SO₂Me | |
| 1-199 | | | | | |
| 1-200 | CH₂O(CH₂)₂OMe | CH₂-c-Pr | 0 | SO₂Me | |
| 1-201 | CH₂O(CH₂)₂OMe | (CH₂)₂OMe | 0 | SO₂Me | |
| 1-202 | CH₂O(CH₂)₂OMe | (CH₂)₃OMe | 0 | SO₂Me | |
| 1-203 | CH₂O(CH₂)₂OMe | (CH₂)₂OEt | 0 | SO₂Me | |
| 1-204 | CH₂O(CH₂)₂OMe | (CH₂)₂OCH₂-c-Pr | 0 | SO₂Me | |
| 1-205 | | | | | |
| 1-206 | CH₂O(CH₂)₂OMe | CH₂-c-Pr | 1 | SO₂Me | |
| 1-207 | CH₂O(CH₂)₂OMe | (CH₂)₂OMe | 1 | SO₂Me | |
| 1-208 | CH₂O(CH₂)₂OMe | (CH₂)₃OMe | 1 | SO₂Me | |
| 1-209 | CH₂O(CH₂)₂OMe | (CH₂)₂OEt | 1 | SO₂Me | |
| 1-210 | CH₂O(CH₂)₂OMe | (CH₂)₂OCH₂-c-Pr | 1 | SO₂Me | |
| 1-211 | | | | | |
| 1-212 | CH₂O(CH₂)₂OMe | CH₂-c-Pr | 2 | SO₂Me | |
| 1-213 | CH₂O(CH₂)₂OMe | (CH₂)₂OMe | 2 | SO₂Me | |
| 1-214 | CH₂O(CH₂)₂OMe | (CH₂)₃OMe | 2 | SO₂Me | |
| 1-215 | CH₂O(CH₂)₂OMe | (CH₂)₂OEt | 2 | SO₂Me | |
| 1-216 | CH₂O(CH₂)₂OMe | (CH₂)₂OCH₂-c-Pr | 2 | SO₂Me | |
| 1-217 | | | | | |
| 1-218 | CH₂O(CH₂)₂OEt | CH₂-c-Pr | 0 | SO₂Me | |
| 1-219 | CH₂O(CH₂)₂OEt | (CH₂)₂OMe | 0 | SO₂Me | |
| 1-220 | CH₂O(CH₂)₂OEt | (CH₂)₃OMe | 0 | SO₂Me | |
| 1-221 | CH₂O(CH₂)₂OEt | (CH₂)₂OEt | 0 | SO₂Me | |
| 1-222 | CH₂O(CH₂)₂OEt | (CH₂)₂OCH₂-c-Pr | 0 | SO₂Me | |
| 1-223 | | | | | |
| 1-224 | CH₂O(CH₂)₂OEt | CH₂-c-Pr | 1 | SO₂Me | |
| 1-225 | CH₂O(CH₂)₂OEt | (CH₂)₂OMe | 1 | SO₂Me | |
| 1-226 | CH₂O(CH₂)₂OEt | (CH₂)₃OMe | 1 | SO₂Me | |
| 1-227 | CH₂O(CH₂)₂OEt | (CH₂)₂OEt | 1 | SO₂Me | |
| 1-228 | CH₂O(CH₂)₂OEt | (CH₂)₂OCH₂-c-Pr | 1 | SO₂Me | |
| 1-229 | | | | | |
| 1-230 | CH₂O(CH₂)₂OEt | CH₂-c-Pr | 2 | SO₂Me | |
| 1-231 | CH₂O(CH₂)₂OEt | (CH₂)₂OMe | 2 | SO₂Me | |
| 1-232 | CH₂O(CH₂)₂OEt | (CH₂)₃OMe | 2 | SO₂Me | |
| 1-233 | CH₂O(CH₂)₂OEt | (CH₂)₂OEt | 2 | SO₂Me | |
| 1-234 | CH₂O(CH₂)₂OEt | (CH₂)₂OCH₂-c-Pr | 2 | SO₂Me | |
| 1-235 | | | | | |
| 1-236 | CH₂O(CH₂)₃OMe | CH₂-c-Pr | 0 | SO₂Me | |
| 1-237 | CH₂O(CH₂)₃OMe | (CH₂)₂OMe | 0 | SO₂Me | |
| 1-238 | CH₂O(CH₂)₃OMe | (CH₂)₃OMe | 0 | SO₂Me | |
| 1-239 | CH₂O(CH₂)₃OMe | (CH₂)₂OEt | 0 | SO₂Me | |
| 1-240 | CH₂O(CH₂)₃OMe | (CH₂)₂OCH₂-c-Pr | 0 | SO₂Me | |
| 1-241 | | | | | |
| 1-242 | CH₂O(CH₂)₃OMe | CH₂-c-Pr | 1 | SO₂Me | |
| 1-243 | CH₂O(CH₂)₃OMe | (CH₂)₂OMe | 1 | SO₂Me | |
| 1-244 | CH₂O(CH₂)₃OMe | (CH₂)₃OMe | 1 | SO₂Me | |
| 1-245 | CH₂O(CH₂)₃OMe | (CH₂)₂OEt | 1 | SO₂Me | |
| 1-246 | CH₂O(CH₂)₃OMe | (CH₂)₂OCH₂-c-Pr | 1 | SO₂Me | |
| 1-247 | | | | | |
| 1-248 | CH₂O(CH₂)₃OMe | CH₂-c-Pr | 2 | SO₂Me | |
| 1-249 | CH₂O(CH₂)₃OMe | (CH₂)₂OMe | 2 | SO₂Me | |
| 1-250 | CH₂O(CH₂)₃OMe | (CH₂)₃OMe | 2 | SO₂Me | |
| 1-251 | CH₂O(CH₂)₃OMe | (CH₂)₂OEt | 2 | SO₂Me | |
| 1-252 | CH₂O(CH₂)₃OMe | (CH₂)₂OCH₂-c-Pr | 2 | SO₂Me | |
| 1-253 | | | | | |
| 1-254 | CH₂OCH₂OMe | CH₂-c-Pr | 0 | SO₂Me | |
| 1-255 | CH₂OCH₂OMe | (CH₂)₂OMe | 0 | SO₂Me | |
| 1-256 | CH₂OCH₂OMe | (CH₂)₃OMe | 0 | SO₂Me | |
| 1-257 | CH₂OCH₂OMe | (CH₂)₂OEt | 0 | SO₂Me | |
| 1-258 | CH₂OCH₂OMe | (CH₂)₂OCH₂-c-Pr | 0 | SO₂Me | |
| 1-259 | | | | | |
| 1-260 | CH₂OCH₂OMe | CH₂-c-Pr | 1 | SO₂Me | |
| 1-261 | CH₂OCH₂OMe | (CH₂)₂OMe | 1 | SO₂Me | |
| 1-262 | CH₂OCH₂OMe | (CH₂)₃OMe | 1 | SO₂Me | |
| 1-263 | CH₂OCH₂OMe | (CH₂)₂OEt | 1 | SO₂Me | |
| 1-264 | CH₂OCH₂OMe | (CH₂)₂OCH₂-c-Pr | 1 | SO₂Me | |
| 1-265 | | | | | |
| 1-266 | CH₂OCH₂OMe | CH₂-c-Pr | 2 | SO₂Me | |
| 1-267 | CH₂OCH₂OMe | (CH₂)₂OMe | 2 | SO₂Me | |
| 1-268 | CH₂OCH₂OMe | (CH₂)₃OMe | 2 | SO₂Me | |
| 1-269 | CH₂OCH₂OMe | (CH₂)₂OEt | 2 | SO₂Me | |
| 1-270 | CH₂OCH₂OMe | (CH₂)₂OCH₂-c-Pr | 2 | SO₂Me | |
| 1-271 | | | | | |
| 1-272 | CH₂OCH₂OEt | CH₂-c-Pr | 0 | SO₂Me | |
| 1-273 | CH₂OCH₂OEt | (CH₂)₂OMe | 0 | SO₂Me | |
| 1-274 | CH₂OCH₂OEt | (CH₂)₃OMe | 0 | SO₂Me | |
| 1-275 | CH₂OCH₂OEt | (CH₂)₂OEt | 0 | SO₂Me | |
| 1-276 | CH₂OCH₂OEt | (CH₂)₂OCH₂-c-Pr | 0 | SO₂Me | |
| 1-277 | | | | | |
| 1-278 | CH₂OCH₂OEt | CH₂-c-Pr | 1 | SO₂Me | |
| 1-279 | CH₂OCH₂OEt | (CH₂)₂OMe | 1 | SO₂Me | |
| 1-280 | CH₂OCH₂OEt | (CH₂)₃OMe | 1 | SO₂Me | |
| 1-281 | CH₂OCH₂OEt | (CH₂)₂OEt | 1 | SO₂Me | |
| 1-282 | CH₂OCH₂OEt | (CH₂)₂OCH₂-c-Pr | 1 | SO₂Me | |
| 1-283 | | | | | |
| 1-284 | CH₂OCH₂OEt | CH₂-c-Pr | 2 | SO₂Me | |
| 1-285 | CH₂OCH₂OEt | (CH₂)₂OMe | 2 | SO₂Me | |
| 1-286 | CH₂OCH₂OEt | (CH₂)₃OMe | 2 | SO₂Me | |
| 1-287 | CH₂OCH₂OEt | (CH₂)₂OEt | 2 | SO₂Me | |
| 1-288 | CH₂OCH₂OEt | (CH₂)₂OCH₂-c-Pr | 2 | SO₂Me | |
| 1-289 | | | | | |
| 1-290 | CH₂O(CH₂)₂SO₂Me | CH₂-c-Pr | 0 | SO₂Me | |
| 1-291 | CH₂O(CH₂)₂SO₂Me | (CH₂)₂OMe | 0 | SO₂Me | |
| 1-292 | CH₂O(CH₂)₂SO₂Me | (CH₂)₃OMe | 0 | SO₂Me | |
| 1-293 | CH₂O(CH₂)₂SO₂Me | (CH₂)₂OEt | 0 | SO₂Me | |
| 1-294 | CH₂O(CH₂)₂SO₂Me | (CH₂)₂OCH₂-c-Pr | 0 | SO₂Me | |
| 1-295 | | | | | |
| 1-296 | CH₂O(CH₂)₂SO₂Me | CH₂-c-Pr | 1 | SO₂Me | |
| 1-297 | CH₂O(CH₂)₂SO₂Me | (CH₂)₂OMe | 1 | SO₂Me | |
| 1-298 | CH₂O(CH₂)₂SO₂Me | (CH₂)₃OMe | 1 | SO₂Me | |
| 1-299 | CH₂O(CH₂)₂SO₂Me | (CH₂)₂OEt | 1 | SO₂Me | |
| 1-300 | CH₂O(CH₂)₂SO₂Me | (CH₂)₂OCH₂-c-Pr | 1 | SO₂Me | |
| 1-301 | | | | | |
| 1-302 | CH₂O(CH₂)₂SO₂Me | CH₂-c-Pr | 2 | SO₂Me | |
| 1-303 | CH₂O(CH₂)₂SO₂Me | (CH₂)₂OMe | 2 | SO₂Me | |
| 1-304 | CH₂O(CH₂)₂SO₂Me | (CH₂)₃OMe | 2 | SO₂Me | |
| 1-305 | CH₂(CH₂)₂SO₂Me | (CH₂)₂OEt | 2 | SO₂Me | |
| 1-306 | CH₂O(CH₂)₂SO₂Me | (CH₂)₂OCH₂-c-Pr | 2 | SO₂Me | |
| 1-307 | | | | | |
| 1-308 | CH₂SO₂(CH₂)₂OMe | CH₂-c-Pr | 0 | SO₂Me | |
| 1-309 | CH₂SO₂(CH₂)₂OMe | (CH₂)₂OMe | 0 | SO₂Me | |
| 1-310 | CH₂SO₂(CH₂)₂OMe | (CH₂)₃OMe | 0 | SO₂Me | |
| 1-311 | CH₂SO₂(CH₂)₂OMe | (CH₂)₂OEt | 0 | SO₂Me | |
| 1-312 | CH₂SO₂(CH₂)₂OMe | (CH₂)₂OCH₂-c-Pr | 0 | SO₂Me | |
| 1-313 | | | | | |
| 1-314 | CH₂SO₂(CH₂)₂OMe | CH₂-c-Pr | 1 | SO₂Me | |
| 1-315 | CH₂SO₂(CH₂)₂OMe | (CH₂)₂OMe | 1 | SO₂Me | |
| 1-316 | CH₂SO₂(CH₂)₂OMe | (CH₂)₃OMe | 1 | SO₂Me | |
| 1-317 | CH₂SO₂(CH₂)₂OMe | (CH₂)₂OEt | 1 | SO₂Me | |
| 1-318 | CH₂SO₂(CH₂)₂OMe | (CH₂)₂OCH₂-c-Pr | 1 | SO₂Me | |
| 1-319 | | | | | |
| 1-320 | CH₂SO₂(CH₂)₂OMe | CH₂-c-Pr | 2 | SO₂Me | |
| 1-321 | CH₂SO₂(CH₂)₂OMe | (CH₂)₂OMe | 2 | SO₂Me | |
| 1-322 | CH₂SO₂(CH₂)₂OMe | (CH₂)₃OMe | 2 | SO₂Me | |
| 1-323 | CH₂SO₂(CH₂)₂OMe | (CH₂)₂OEt | 2 | SO₂Me | |
| 1-324 | CH₂SO₂(CH₂)₂OMe | (CH₂)₂OCH₂-c-Pr | 2 | SO₂Me | |
| 1-325 | | | | | |
| 1-326 | CH₂SO₂(CH₂)₂SO₂Me | CH₂-c-Pr | 0 | SO₂Me | |
| 1-327 | CH₂SO₂(CH₂)₂SO₂Me | (CH₂)₂OMe | 0 | SO₂Me | |
| 1-328 | CH₂SO₂(CH₂)₂SO₂Me | (CH₂)₃OMe | 0 | SO₂Me | |
| 1-329 | CH₂SO₂(CH₂)₂SO₂Me | (CH₂)₂OEt | 0 | SO₂Me | |
| 1-330 | CH₂SO₂(CH₂)₂SO₂Me | (CH₂)₂OCH₂-c-Pr | 0 | SO₂Me | |
| 1-331 | | | | | |
| 1-332 | CH₂SO₂(CH₂)₂SO₂Me | CH₂-c-Pr | 1 | SO₂Me | |
| 1-333 | CH₂SO₂(CH₂)₂SO₂Me | (CH₂)₂OMe | 1 | SO₂Me | |
| 1-334 | CH₂SO₂(CH₂)₂SO₂Me | (CH₂)₃OMe | 1 | SO₂Me | |
| 1-335 | CH₂SO₂(CH₂)₂SO₂Me | (CH₂)₂OEt | 1 | SO₂Me | |
| 1-336 | CH₂SO₂(CH₂)₂SO₂Me | (CH₂)₂OCH₂-c-Pr | 1 | SO₂Me | |
| 1-337 | | | | | |
| 1-338 | CH₂SO₂(CH₂)₂SO₂Me | CH₂-c-Pr | 2 | SO₂Me | |
| 1-339 | CH₂SO₂(CH₂)₂SO₂Me | (CH₂)₂OMe | 2 | SO₂Me | |
| 1-340 | CH₂SO₂(CH₂)₂SO₂Me | (CH₂)₃OMe | 2 | SO₂Me | |
| 1-341 | CH₂SO₂(CH₂)₂SO₂Me | (CH₂)₂OEt | 2 | SO₂Me | |
| 1-342 | CH₂SO₂(CH₂)₂SO₂Me | (CH₂)₂OCH₂-c-Pr | 2 | SO₂Me | |
| 1-343 | | | | | |
| 1-344 | Cl | CH₂-c-Pr | 0 | SO₂Et | |
| 1-345 | Cl | (CH₂)₂OMe | 0 | SO₂Et | |
| 1-346 | Cl | (CH₂)₃OMe | 0 | SO₂Et | |
| 1-347 | Cl | (CH₂)₂OEt | 0 | SO₂Et | |
| 1-348 | Cl | (CH₂)₂OCH₂-c-Pr | 0 | SO₂Et | |
| 1-349 | | | | | |
| 1-350 | Cl | CH₂-c-Pr | 1 | SO₂Et | |
| 1-351 | Cl | (CH₂)₂OMe | 1 | SO₂Et | |
| 1-352 | Cl | (CH₂)₃OMe | 1 | SO₂Et | |
| 1-353 | Cl | (CH₂)₂OEt | 1 | SO₂Et | |
| 1-354 | Cl | (CH₂)₂OCH₂-c-Pr | 1 | SO₂Et | |
| 1-355 | | | | | |
| 1-356 | Cl | CH₂-c-Pr | 2 | SO₂Et | |
| 1-357 | Cl | (CH₂)₂OMe | 2 | SO₂Et | |
| 1-358 | Cl | (CH₂)₃OMe | 2 | SO₂Et | |
| 1-359 | Cl | (CH₂)₂OEt | 2 | SO₂Et | |
| 1-360 | Cl | (CH₂)₂OCH₂-c-Pr | 2 | SO₂Et | |
| 1-361 | | | | | |
| 1-362 | Br | CH₂-c-Pr | 0 | SO₂Et | |
| 1-363 | Br | (CH₂)₂OMe | 0 | SO₂Et | |
| 1-364 | Br | (CH₂)₃OMe | 0 | SO₂Et | |
| 1-365 | Br | (CH₂)₂OEt | 0 | SO₂Et | |
| 1-366 | Br | (CH₂)₂OCH₂-c-Pr | 0 | SO₂Et | |
| 1-367 | | | | | |
| 1-368 | Br | CH₂-c-Pr | 1 | SO₂Et | |
| 1-369 | Br | (CH₂)₂OMe | 1 | SO₂Et | |
| 1-370 | Br | (CH₂)₃OMe | 1 | SO₂Et | |
| 1-371 | Br | (CH₂)₂OEt | 1 | SO₂Et | |
| 1-372 | Br | (CH₂)₂OCH₂-c-Pr | 1 | SO₂Et | |
| 1-373 | | | | | |
| 1-374 | Br | CH₂-c-Pr | 2 | SO₂Et | |
| 1-375 | Br | (CH₂)₂OMe | 2 | SO₂Et | |
| 1-376 | Br | (CH₂)₃OMe | 2 | SO₂Et | |
| 1-377 | Br | (CH₂)₂OEt | 2 | SO₂Et | |
| 1-378 | Br | (CH₂)₂OCH₂-c-Pr | 2 | SO₂Et | |
| 1-379 | | | | | |
| 1-380 | Me | CH₂-c-Pr | 0 | SO₂Et | |
| 1-381 | Me | (CH₂)₂OMe | 0 | SO₂Et | |
| 1-382 | Me | (CH₂)₃OMe | 0 | SO₂Et | |
| 1-383 | Me | (CH₂)₂OEt | 0 | SO₂Et | |
| 1-384 | Me | (CH₂)₂OCH₂-c-Pr | 0 | SO₂Et | |
| 1-385 | | | | | |
| 1-386 | Me | CH₂-c-Pr | 1 | SO₂Et | |
| 1-387 | Me | (CH₂)₂OMe | 1 | SO₂Et | |
| 1-388 | Me | (CH₂)₃OMe | 1 | SO₂Et | |
| 1-389 | Me | (CH₂)₂OEt | 1 | SO₂Et | |
| 1-390 | Me | (CH₂)₂OCH₂-c-Pr | 1 | SO₂Et | |
| 1-391 | | | | | |
| 1-392 | Me | CH₂-c-Pr | 2 | SO₂Et | |
| 1-393 | Me | (CH₂)₂OMe | 2 | SO₂Et | 8.30 (d, 1H), 7.64 (d, 1H), 4.02-3.87 (m, 4H), 3.81 (q, 2H), 3.62 (s, 3H), 3.33 (s, 3H), 2.77 (s, 3H), 1.37 (t, 3H), 0.86 - 0.70 (m, 3H), 0.55 - 0.44 (m, 2H) |
| 1-394 | Me | (CH₂)₃OMe | 2 | SO₂Et | |
| 1-395 | Me | (CH₂)₂OEt | 2 | SO₂Et | |
| 1-396 | Me | (CH₂)₂OCH₂-c-Pr | 2 | SO₂Et | |
| 1-397 | | | | | |
| 1-398 | Et | CH₂-c-Pr | 0 | SO₂Et | |
| 1-399 | Et | (CH₂)₂OMe | 0 | SO₂Et | |
| 1-400 | Et | (CH₂)₃OMe | 0 | SO₂Et | |
| 1-401 | Et | (CH₂)₂OEt | 0 | SO₂Et | |
| 1-402 | Et | (CH₂)₂OCH₂-c-Pr | 0 | SO₂Et | |
| 1-403 | | | | | |
| 1-404 | Et | CH₂-c-Pr | 1 | SO₂Et | |
| 1-405 | Et | (CH₂)₂OMe | 1 | SO₂Et | |
| 1-406 | Et | (CH₂)₃OMe | 1 | SO₂Et | |
| 1-407 | Et | (CH₂)₂OEt | 1 | SO₂Et | |
| 1-408 | Et | (CH₂)₂OCH₂-c-Pr | 1 | SO₂Et | |
| 1-409 | | | | | |
| 1-410 | Et | CH₂-c-Pr | 2 | SO₂Et | |
| 1-411 | Et | (CH₂)₂OMe | 2 | SO₂Et | |
| 1-412 | Et | (CH₂)₃OMe | 2 | SO₂Et | |
| 1-413 | Et | (CH₂)₂OEt | 2 | SO₂Et | |
| 1-414 | Et | (CH₂)₂OCH₂-c-Pr | 2 | SO₂Et | |
| 1-415 | | | | | |
| 1-416 | CF₃ | CH₂-c-Pr | 0 | SO₂Et | |
| 1-417 | CF₃ | (CH₂)₂OMe | 0 | SO₂Et | |
| 1-418 | CF₃ | (CH₂)₃OMe | 0 | SO₂Et | |
| 1-419 | CF₃ | (CH₂)₂OEt | 0 | SO₂Et | |
| 1-420 | CF₃ | (CH₂)₂OCH₂-c-Pr | 0 | SO₂Et | |
| 1-421 | | | | | |
| 1-422 | CF₃ | CH₂-c-Pr | 1 | SO₂Et | |
| 1-423 | CF₃ | (CH₂)₂OMe | 1 | SO₂Et | |
| 1-424 | CF₃ | (CH₂)₃OMe | 1 | SO₂Et | |
| 1-425 | CF₃ | (CH₂)₂OEt | 1 | SO₂Et | |
| 1-426 | CF₃ | (CH₂)₂OCH₂-c-Pr | 1 | SO₂Et | |
| 1-427 | | | | | |
| 1-428 | CF₃ | CH₂-c-Pr | 2 | SO₂Et | |
| 1-429 | CF₃ | (CH₂)₂OMe | 2 | SO₂Et | |
| 1-430 | CF₃ | (CH₂)₃OMe | 2 | SO₂Et | |
| 1-431 | CF₃ | (CH₂)₂OEt | 2 | SO₂Et | |
| 1-432 | CF₃ | (CH₂)₂OCH₂-c-Pr | 2 | SO₂Et | |
| 1-433 | | | | | |
| 1-434 | OMe | CH₂-c-Pr | 0 | SO₂Et | |
| 1-435 | OMe | (CH₂)₂OMe | 0 | SO₂Et | |
| 1-436 | OMe | (CH₂)₃OMe | 0 | SO₂Et | |
| 1-437 | OMe | (CH₂)₂OEt | 0 | SO₂Et | |
| 1-438 | OMe | (CH₂)₂OCH₂-c-Pr | 0 | SO₂Et | |
| 1-439 | OMe | c-Pr | 1 | SO₂Et | |
| 1-440 | OMe | CH₂-c-Pr | 1 | SO₂Et | |
| 1-441 | OMe | (CH₂)₂OMe | 1 | SO₂Et | |
| 1-442 | OMe | (CH₂)₃OMe | 1 | SO₂Et | |
| 1-443 | OMe | (CH₂)₂OEt | 1 | SO₂Et | |
| 1-444 | OMe | (CH₂)₂OCH₂-c-Pr | 1 | SO₂Et | |
| 1-445 | | | | | |
| 1-446 | OMe | CH₂-c-Pr | 2 | SO₂Et | |
| 1-447 | OMe | (CH₂)₂OMe | 2 | SO₂Et | |
| 1-448 | OMe | (CH₂)₃OMe | 2 | SO₂Et | |
| 1-449 | OMe | (CH₂)₂OEt | 2 | SO₂Et | |
| 1-450 | OMe | (CH₂)₂OCH₂-c-Pr | 2 | SO₂Et | |
| 1-451 | | | | | |
| 1-452 | OEt | CH₂-c-Pr | 0 | SO₂Et | |
| 1-453 | OEt | (CH₂)₂OMe | 0 | SO₂Et | |
| 1-454 | OEt | (CH₂)₃OMe | 0 | SO₂Et | |
| 1-455 | OEt | (CH₂)₂OEt | 0 | SO₂Et | |
| 1-456 | OEt | (CH₂)₂OCH₂-c-Pr | 0 | SO₂Et | |
| 1-457 | | | | | |
| 1-458 | OEt | CH₂-c-Pr | 1 | SO₂Et | |
| 1-459 | OEt | (CH₂)₂OMe | 1 | SO₂Et | |
| 1-460 | OEt | (CH₂)₃OMe | 1 | SO₂Et | |
| 1-461 | OEt | (CH₂)₂OEt | 1 | SO₂Et | |
| 1-462 | OEt | (CH₂)₂OCH₂-c-Pr | 1 | SO₂Et | |
| 1-463 | | | | | |
| 1-464 | OEt | CH₂-c-Pr | 2 | SO₂Et | |
| 1-465 | OEt | (CH₂)₂OMe | 2 | SO₂Et | |
| 1-466 | OEt | (CH₂)₃OMe | 2 | SO₂Et | |
| 1-467 | OEt | (CH₂)₂OEt | 2 | SO₂Et | |
| 1-468 | OEt | (CH₂)₂OCH₂-c-Pr | 2 | SO₂Et | |
| 1-469 | | | | | |
| 1-470 | NO₂ | CH₂-c-Pr | 0 | SO₂Et | |
| 1-471 | NO₂ | (CH₂)₂OMe | 0 | SO₂Et | |
| 1-472 | NO₂ | (CH₂)₃OMe | 0 | SO₂Et | |
| 1-473 | NO₂ | (CH₂)₂OEt | 0 | SO₂Et | |
| 1-474 | NO₂ | (CH₂)₂OCH₂-c-Pr | 0 | SO₂Et | |
| 1-475 | | | | | |
| 1-476 | NO₂ | CH₂-c-Pr | 1 | SO₂Et | |
| 1-477 | NO₂ | (CH₂)₂OMe | 1 | SO₂Et | |
| 1-478 | NO₂ | (CH₂)₃OMe | 1 | SO₂Et | |
| 1-479 | NO₂ | (CH₂)₂OEt | 1 | SO₂Et | |
| 1-480 | NO₂ | (CH₂)₂OCH₂-c-Pr | 1 | SO₂Et | |
| 1-481 | | | | | |
| 1-482 | NO₂ | CH₂-c-Pr | 2 | SO₂Et | |
| 1-483 | NO₂ | (CH₂)₂OMe | 2 | SO₂Et | |
| 1-484 | NO₂ | (CH₂)₃OMe | 2 | SO₂Et | |
| 1-485 | NO₂ | (CH₂)₂OEt | 2 | SO₂Et | |
| 1-486 | NO₂ | (CH₂)₂OCH₂-c-Pr | 2 | SO₂Et | |
| 1-487 | | | | | |
| 1-488 | SO₂Me | CH₂-c-Pr | 0 | SO₂Et | |
| 1-489 | SO₂Me | (CH₂)₂OMe | 0 | SO₂Et | |
| 1-490 | SO₂Me | (CH₂)₃OMe | 0 | SO₂Et | |
| 1-491 | SO₂Me | (CH₂)₂OEt | 0 | SO₂Et | |
| 1-492 | SO₂Me | (CH₂)₂OCH₂-c-Pr | 0 | SO₂Et | |
| 1-493 | | | | | |
| 1-494 | SO₂Me | CH₂-c-Pr | 1 | SO₂Et | |
| 1-495 | SO₂Me | (CH₂)₂OMe | 1 | SO₂Et | |
| 1-496 | SO₂Me | (CH₂)₃OMe | 1 | SO₂Et | |
| 1-497 | SO₂Me | (CH₂)₂OEt | 1 | SO₂Et | |
| 1-498 | SO₂Me | (CH₂)₂OCH₂-c-Pr | 1 | SO₂Et | |
| 1-499 | | | | | |
| 1-500 | SO₂Me | CH₂-c-Pr | 2 | SO₂Et | |
| 1-501 | SO₂Me | (CH₂)₂OMe | 2 | SO₂Et | |
| 1-502 | SO₂Me | (CH₂)₃OMe | 2 | SO₂Et | |
| 1-503 | SO₂Me | (CH₂)₂OEt | 2 | SO₂Et | |
| 1-504 | SO₂Me | (CH₂)₂OCH₂-c-Pr | 2 | SO₂Et | |
| 1-505 | | | | | |
| 1-506 | CH₂OMe | CH₂-c-Pr | 0 | SO₂Et | |
| 1-507 | CH₂OMe | (CH₂)₂OMe | 0 | SO₂Et | |
| 1-508 | CH₂OMe | (CH₂)₃OMe | 0 | SO₂Et | |
| 1-509 | CH₂OMe | (CH₂)₂OEt | 0 | SO₂Et | |
| 1-510 | CH₂OMe | (CH₂)₂OCH₂-c-Pr | 0 | SO₂Et | |
| 1-511 | | | | | |
| 1-512 | CH₂OMe | CH₂-c-Pr | 1 | SO₂Et | |
| 1-513 | CH₂OMe | (CH₂)₂OMe | 1 | SO₂Et | |
| 1-514 | CH₂OMe | (CH₂)₃OMe | 1 | SO₂Et | |
| 1-515 | CH₂OMe | (CH₂)₂OEt | 1 | SO₂Et | |
| 1-516 | CH₂OMe | (CH₂)₂OCH₂-c-Pr | 1 | SO₂Et | |
| 1-517 | | | | | |
| 1-518 | CH₂OMe | CH₂-c-Pr | 2 | SO₂Et | |
| 1-519 | CH₂OMe | (CH₂)₂OMe | 2 | SO₂Et | |
| 1-520 | CH₂OMe | (CH₂)₃OMe | 2 | SO₂Et | |
| 1-521 | CH₂OMe | (CH₂)₂OEt | 2 | SO₂Et | |
| 1-522 | CH₂OMe | (CH₂)₂OCH₂-c-Pr | 2 | SO₂Et | |
| 1-523 | | | | | |
| 1-524 | CH₂SO₂Me | CH₂-c-Pr | 0 | SO₂Et | |
| 1-525 | CH₂SO₂Me | (CH₂)₂OMe | 0 | SO₂Et | |
| 1-526 | CH₂SO₂Me | (CH₂)₃OMe | 0 | SO₂Et | |
| 1-527 | CH₂SO₂Me | (CH₂)₂OEt | 0 | SO₂Et | |
| 1-528 | CH₂SO₂Me | (CH₂)₂OCH₂-c-Pr | 0 | SO₂Et | |
| 1-529 | | | | | |
| 1-530 | CH₂SO₂Me | CH₂-c-Pr | 1 | SO₂Et | |
| 1-531 | CH₂SO₂Me | (CH₂)₂OMe | 1 | SO₂Et | |
| 1-532 | CH₂SO₂Me | (CH₂)₃OMe | 1 | SO₂Et | |
| 1-533 | CH₂SO₂Me | (CH₂)₂OEt | 1 | SO₂Et | |
| 1-534 | CH₂SO₂Me | (CH₂)₂OCH₂-c-Pr | 1 | SO₂Et | |
| 1-535 | | | | | |
| 1-536 | CH₂SO₂Me | CH₂-c-Pr | 2 | SO₂Et | |
| 1-537 | CH₂SO₂Me | (CH₂)₂OMe | 2 | SO₂Et | |
| 1-538 | CH₂SO₂Me | (CH₂)₃OMe | 2 | SO₂Et | |
| 1-539 | CH₂SO₂Me | (CH₂)₂OEt | 2 | SO₂Et | |
| 1-540 | CH₂SO₂Me | (CH₂)₂OCH₂-c-Pr | 2 | SO₂Et | |
| 1-541 | | | | | |
| 1-542 | CH₂O(CH₂)₂OMe | CH₂-c-Pr | 0 | SO₂Et | |
| 1-543 | CH₂O(CH₂)₂OMe | (CH₂)₂OMe | 0 | SO₂Et | |
| 1-544 | CH₂O(CH₂)₂OMe | (CH₂)₃OMe | 0 | SO₂Et | |
| 1-545 | CH₂O(CH₂)₂OMe | (CH₂)₂OEt | 0 | SO₂Et | |
| 1-546 | CH₂O(CH₂)₂OMe | (CH₂)₂OCH₂-c-Pr | 0 | SO₂Et | |
| 1-547 | | | | | |
| 1-548 | CH₂O(CH₂)₂OMe | CH₂-c-Pr | 1 | SO₂Et | |
| 1-549 | CH₂O(CH₂)₂OMe | (CH₂)₂OMe | 1 | SO₂Et | |
| 1-550 | CH₂O(CH₂)₂OMe | (CH₂)₃OMe | 1 | SO₂Et | |
| 1-551 | CH₂O(CH₂)₂OMe | (CH₂)₂OEt | 1 | SO₂Et | |
| 1-552 | CH₂O(CH₂)₂OMe | (CH₂)₂OCH₂-c-Pr | 1 | SO₂Et | |
| 1-553 | | | | | |
| 1-554 | CH₂O(CH₂)₂OMe | CH₂-c-Pr | 2 | SO₂Et | |
| 1-555 | CH₂O(CH₂)₂OMe | (CH₂)₂OMe | 2 | SO₂Et | |
| 1-556 | CH₂O(CH₂)₂OMe | (CH₂)₃OMe | 2 | SO₂Et | |
| 1-557 | CH₂O(CH₂)₂OMe | (CH₂)₂OEt | 2 | SO₂Et | |
| 1-558 | CH₂O(CH₂)₂OMe | (CH₂)₂OCH₂-c-Pr | 2 | SO₂Et | |
| 1-559 | | | | | |
| 1-560 | CH₂O(CH₂)₂OEt | CH₂-c-Pr | 0 | SO₂Et | |
| 1-561 | CH₂O(CH₂)₂OEt | (CH₂)₂OMe | 0 | SO₂Et | |
| 1-562 | CH₂O(CH₂)₂OEt | (CH₂)₃OMe | 0 | SO₂Et | |
| 1-563 | CH₂O(CH₂)₂OEt | (CH₂)₂OEt | 0 | SO₂Et | |
| 1-564 | CH₂O(CH₂)₂OEt | (CH₂)₂OCH₂-c-Pr | 0 | SO₂Et | |
| 1-565 | | | | | |
| 1-566 | CH₂O(CH₂)₂OEt | CH₂-c-Pr | 1 | SO₂Et | |
| 1-567 | CH₂O(CH₂)₂OEt | (CH₂)₂OMe | 1 | SO₂Et | |
| 1-568 | CH₂O(CH₂)₂OEt | (CH₂)₃OMe | 1 | SO₂Et | |
| 1-569 | CH₂O(CH₂)₂OEt | (CH₂)₂OEt | 1 | SO₂Et | |
| 1-570 | CH₂O(CH₂)₂OEt | (CH₂)₂OCH₂-c-Pr | 1 | SO₂Et | |
| 1-571 | | | | | |
| 1-572 | CH₂O(CH₂)₂OEt | CH₂-c-Pr | 2 | SO₂Et | |
| 1-573 | CH₂O(CH₂)₂OEt | (CH₂)₂OMe | 2 | SO₂Et | |
| 1-574 | CH₂O(CH₂)₂OEt | (CH₂)₃OMe | 2 | SO₂Et | |
| 1-575 | CH₂O(CH₂)₂OEt | (CH₂)₂OEt | 2 | SO₂Et | |
| 1-576 | CH₂O(CH₂)₂OEt | (CH₂)₂OCH₂-c-Pr | 2 | SO₂Et | |
| 1-577 | | | | | |
| 1-578 | CH₂O(CH₂)₃OMe | CH₂-c-Pr | 0 | SO₂Et | |
| 1-579 | CH₂O(CH₂)₃OMe | (CH₂)₂OMe | 0 | SO₂Et | |
| 1-580 | CH₂O(CH₂)₃OMe | (CH₂)₃OMe | 0 | SO₂Et | |
| 1-581 | CH₂O(CH₂)₃OMe | (CH₂)₂OEt | 0 | SO₂Et | |
| 1-582 | CH₂O(CH₂)₃OMe | (CH₂)₂OCH₂-c-Pr | 0 | SO₂Et | |
| 1-583 | | | | | |
| 1-584 | CH₂O(CH₂)₃OMe | CH₂-c-Pr | 1 | SO₂Et | |
| 1-585 | CH₂O(CH₂)₃OMe | (CH₂)₂OMe | 1 | SO₂Et | |
| 1-586 | CH₂O(CH₂)₃OMe | (CH₂)₃OMe | 1 | SO₂Et | |
| 1-587 | CH₂O(CH₂)₃OMe | (CH₂)₂OEt | 1 | SO₂Et | |
| 1-588 | CH₂O(CH₂)₃OMe | (CH₂)₂OCH₂-c-Pr | 1 | SO₂Et | |
| 1-589 | | | | | |
| 1-590 | CH₂O(CH₂)₃OMe | CH₂-c-Pr | 2 | SO₂Et | |
| 1-591 | CH₂O(CH₂)₃OMe | (CH₂)₂OMe | 2 | SO₂Et | |
| 1-592 | CH₂O(CH₂)₃OMe | (CH₂)₃OMe | 2 | SO₂Et | |
| 1-593 | CH₂O(CH₂)₃OMe | (CH₂)₂OEt | 2 | SO₂Et | |
| 1-594 | CH₂O(CH₂)₃OMe | (CH₂)₂OCH₂-c-Pr | 2 | SO₂Et | |
| 1-595 | | | | | |
| 1-596 | CH₂OCH₂OMe | CH₂-c-Pr | 0 | SO₂Et | |
| 1-597 | CH₂OCH₂OMe | (CH₂)₂OMe | 0 | SO₂Et | |
| 1-598 | CH₂OCH₂OMe | (CH₂)₃OMe | 0 | SO₂Et | |
| 1-599 | CH₂OCH₂OMe | (CH₂)₂OEt | 0 | SO₂Et | |
| 1-600 | CH₂OCH₂OMe | (CH₂)₂OCH₂-c-Pr | 0 | SO₂Et | |
| 1-601 | | | | | |
| 1-602 | CH₂OCH₂OMe | CH₂-c-Pr | 1 | SO₂Et | |
| 1-603 | CH₂OCH₂OMe | (CH₂)₂OMe | 1 | SO₂Et | |
| 1-604 | CH₂OCH₂OMe | (CH₂)₃OMe | 1 | SO₂Et | |
| 1-605 | CH₂OCH₂OMe | (CH₂)₂OEt | 1 | SO₂Et | |
| 1-606 | CH₂OCH₂OMe | (CH₂)₂OCH₂-c-Pr | 1 | SO₂Et | |
| 1-607 | | | | | |
| 1-608 | CH₂OCH₂OMe | CH₂-c-Pr | 2 | SO₂Et | |
| 1-609 | CH₂OCH₂OMe | (CH₂)₂OMe | 2 | SO₂Et | |
| 1-610 | CH₂OCH₂OMe | (CH₂)₃OMe | 2 | SO₂Et | |
| 1-611 | CH₂OCH₂OMe | (CH₂)₂OEt | 2 | SO₂Et | |
| 1-612 | CH₂OCH₂OMe | (CH₂)₂OCH₂-c-Pr | 2 | SO₂Et | |
| 1-613 | | | | | |
| 1-614 | CH₂OCH₂OEt | CH₂-c-Pr | 0 | SO₂Et | |
| 1-615 | CH₂OCH₂OEt | (CH₂)₂OMe | 0 | SO₂Et | |
| 1-616 | CH₂OCH₂OEt | (CH₂)₃OMe | 0 | SO₂Et | |
| 1-617 | CH₂OCH₂OEt | (CH₂)₂OEt | 0 | SO₂Et | |
| 1-618 | CH₂OCH₂OEt | (CH₂)₂OCH₂-c-Pr | 0 | SO₂Et | |
| 1-619 | | | | | |
| 1-620 | CH₂OCH₂OEt | CH₂-c-Pr | 1 | SO₂Et | |
| 1-621 | CH₂OCH₂OEt | (CH₂)₂OMe | 1 | SO₂Et | |
| 1-622 | CH₂OCH₂OEt | (CH₂)₃OMe | 1 | SO₂Et | |
| 1-623 | CH₂OCH₂OEt | (CH₂)₂OEt | 1 | SO₂Et | |
| 1-624 | CH₂OCH₂OEt | (CH₂)₂OCH₂-c-Pr | 1 | SO₂Et | |
| 1-625 | | | | | |
| 1-626 | CH₂OCH₂OEt | CH₂-c-Pr | 2 | SO₂Et | |
| 1-627 | CH₂OCH₂OEt | (CH₂)₂OMe | 2 | SO₂Et | |
| 1-628 | CH₂OCH₂OEt | (CH₂)₃OMe | 2 | SO₂Et | |
| 1-629 | CH₂OCH₂OEt | (CH₂)₂OEt | 2 | SO₂Et | |
| 1-630 | CH₂OCH₂OEt | (CH₂)₂OCH₂-c-Pr | 2 | SO₂Et | |
| 1-631 | | | | | |
| 1-632 | CH₂O(CH₂)₂SO₂Me | CH₂-c-Pr | 0 | SO₂Et | |
| 1-633 | CH₂O(CH₂)₂SO₂Me | (CH₂)₂OMe | 0 | SO₂Et | |
| 1-634 | CH₂O(CH₂)₂SO₂Me | (CH₂)₃OMe | 0 | SO₂Et | |
| 1-635 | CH₂O(CH₂)₂SO₂Me | (CH₂)₂OEt | 0 | SO₂Et | |
| 1-636 | CH₂O(CH₂)₂SO₂Me | (CH₂)₂OCH₂-c-Pr | 0 | SO₂Et | |
| 1-637 | | | | | |
| 1-638 | CH₂O(CH₂)₂SO₂Me | CH₂-c-Pr | 1 | SO₂Et | |
| 1-639 | CH₂O(CH₂)₂SO₂Me | (CH₂)₂OMe | 1 | SO₂Et | |
| 1-640 | CH₂O(CH₂)₂SO₂Me | (CH₂)₃OMe | 1 | SO₂Et | |
| 1-641 | CH₂O(CH₂)₂SO₂Me | (CH₂)₂OEt | 1 | SO₂Et | |
| 1-642 | CH₂O(CH₂)₂SO₂Me | (CH₂)₂OCH₂-c-Pr | 1 | SO₂Et | |
| 1-643 | | | | | |
| 1-644 | CH₂O(CH₂)₂SO₂Me | CH₂-c-Pr | 2 | SO₂Et | |
| 1-645 | CH₂O(CH₂)₂SO₂Me | (CH₂)₂OMe | 2 | SO₂Et | |
| 1-646 | CH₂O(CH₂)₂SO₂Me | (CH₂)₃OMe | 2 | SO₂Et | |
| 1-647 | CH₂O(CH₂)₂SO₂Me | (CH₂)₂OEt | 2 | SO₂Et | |
| 1-648 | CH₂O(CH₂)₂SO₂Me | (CH₂)₂OCH₂-c-Pr | 2 | SO₂Et | |
| 1-649 | | | | | |
| 1-650 | CH₂SO₂(CH₂)₂OMe | CH₂-c-Pr | 0 | SO₂Et | |
| 1-651 | CH₂SO₂(CH₂)₂OMe | (CH₂)₂OMe | 0 | SO₂Et | |
| 1-652 | CH₂SO₂(CH₂)₂OMe | (CH₂)₃OMe | 0 | SO₂Et | |
| 1-653 | CH₂SO₂(CH₂)₂OMe | (CH₂)₂OEt | 0 | SO₂Et | |
| 1-654 | CH₂SO₂(CH₂)₂OMe | (CH₂)₂OCH₂-c-Pr | 0 | SO₂Et | |
| 1-655 | | | | | |
| 1-656 | CH₂SO₂(CH₂)₂OMe | CH₂-c-Pr | 1 | SO₂Et | |
| 1-657 | CH₂SO₂(CH₂)₂OMe | (CH₂)₂OMe | 1 | SO₂Et | |
| 1-658 | CH₂SO₂(CH₂)₂OMe | (CH₂)₃OMe | 1 | SO₂Et | |
| 1-659 | CH₂SO₂(CH₂)₂OMe | (CH₂)₂OEt | 1 | SO₂Et | |
| 1-660 | CH₂SO₂(CH₂)₂OMe | (CH₂)₂OCH₂-c-Pr | 1 | SO₂Et | |
| 1-661 | | | | | |
| 1-662 | CH₂SO₂(CH₂)₂OMe | CH₂-c-Pr | 2 | SO₂Et | |
| 1-663 | CH₂SO₂(CH₂)₂OMe | (CH₂)₂OMe | 2 | SO₂Et | |
| 1-664 | CH₂SO₂(CH₂)₂OMe | (CH₂)₃OMe | 2 | SO₂Et | |
| 1-665 | CH₂SO₂(CH₂)₂OMe | (CH₂)₂OEt | 2 | SO₂Et | |
| 1-666 | CH₂SO₂(CH₂)₂OMe | (CH₂)₂OCH₂-c-Pr | 2 | SO₂Et | |
| 1-667 | | | | | |
| 1-668 | CH₂SO₂(CH₂)₂SO₂Me | CH₂-c-Pr | 0 | SO₂Et | |
| 1-669 | CH₂SO₂(CH₂)₂SO₂Me | (CH₂)₂OMe | 0 | SO₂Et | |
| 1-670 | CH₂SO₂(CH₂)₂SO₂Me | (CH₂)₃OMe | 0 | SO₂Et | |
| 1-671 | CH₂SO₂(CH₂)₂SO₂Me | (CH₂)₂OEt | 0 | SO₂Et | |
| 1-672 | CH₂SO₂(CH₂)₂SO₂Me | (CH₂)₂OCH₂-c-Pr | 0 | SO₂Et | |
| 1-673 | | | | | |
| 1-674 | CH₂SO₂(CH₂)₂SO₂Me | CH₂-c-Pr | 1 | SO₂Et | |
| 1-675 | CH₂SO₂(CH₂)₂SO₂Me | (CH₂)₂OMe | 1 | SO₂Et | |
| 1-676 | CH₂SO₂(CH₂)₂SO₂Me | (CH₂)₃OMe | 1 | SO₂Et | |
| 1-677 | CH₂SO₂(CH₂)₂SO₂Me | (CH₂)₂OEt | 1 | SO₂Et | |
| 1-678 | CH₂SO**₂**(CH₂)₂SO ₂Me | (CH₂)₂OCH₂-c-Pr | 1 | SO₂Et | |
| 1-679 | | | | | |
| 1-680 | CH₂SO₂(CH₂)₂SO₂Me | CH₂-c-Pr | 2 | SO₂Et | |
| 1-681 | CH₂SO₂(CH₂)₂SO₂Me | (CH₂)₂OMe | 2 | SO₂Et | |
| 1-682 | CH₂SO₂(CH₂)₂SO₂Me | (CH₂)₃OMe | 2 | SO₂Et | |
| 1-683 | CH₂SO₂(CH₂)₂SO₂Me | (CH₂)₂OEt | 2 | SO₂Et | |
| 1-684 | CH₂SO₂(CH₂)₂SO₂Me | (CH₂)₂OCH₂-c-Pr | 2 | SO₂Et | |
| 1-685 | | | | | |
| 1-686 | Cl | CH₂-c-Pr | 0 | OMe | |
| 1-687 | Cl | (CH₂)₂OMe | 0 | OMe | |
| 1-688 | Cl | (CH₂)₃OMe | 0 | OMe | |
| 1-689 | Cl | (CH₂)₂OEt | 0 | OMe | |
| 1-690 | Cl | (CH₂)₂OCH₂-c-Pr | 0 | OMe | |
| 1-691 | | | | | |
| 1-692 | Cl | CH₂-c-Pr | 1 | OMe | |
| 1-693 | Cl | (CH₂)₂OMe | 1 | OMe | |
| 1-694 | Cl | (CH₂)₃OMe | 1 | OMe | |
| 1-695 | Cl | (CH₂)₂OEt | 1 | OMe | |
| 1-696 | Cl | (CH₂)₂OCH₂-c-Pr | 1 | OMe | |
| 1-697 | | | | | |
| 1-698 | Cl | CH₂-c-Pr | 2 | OMe | |
| 1-699 | Cl | (CH₂)₂OMe | 2 | OMe | |
| 1-700 | Cl | (CH₂)₃OMe | 2 | OMe | |
| 1-701 | Cl | (CH₂)₂OEt | 2 | OMe | |
| 1-702 | Cl | (CH₂)₂OCH₂-c-Pr | 2 | OMe | |
| 1-703 | | | | | |
| 1-704 | Br | CH₂-c-Pr | 0 | OMe | |
| 1-705 | Br | (CH₂)₂OMe | 0 | OMe | |
| 1-706 | Br | (CH₂)₃OMe | 0 | OMe | |
| 1-707 | Br | (CH₂)₂OEt | 0 | OMe | |
| 1-708 | Br | (CH₂)₂OCH₂-c-Pr | 0 | OMe | |
| 1-709 | | | | | |
| 1-710 | Br | CH₂-c-Pr | 1 | OMe | |
| 1-711 | Br | (CH₂)₂OMe | 1 | OMe | |
| 1-712 | Br | (CH₂)₃OMe | 1 | OMe | |
| 1-713 | Br | (CH₂)₂OEt | 1 | OMe | |
| 1-714 | Br | (CH₂)₂OCH₂-c-Pr | 1 | OMe | |
| 1-715 | | | | | |
| 1-716 | Br | CH₂-c-Pr | 2 | OMe | |
| 1-717 | Br | (CH₂)₂OMe | 2 | OMe | |
| 1-718 | Br | (CH₂)₃OMe | 2 | OMe | |
| 1-719 | Br | (CH₂)₂OEt | 2 | OMe | |
| 1-720 | Br | (CH₂)₂OCH₂-c-Pr | 2 | OMe | |
| 1-721 | | | | | |
| 1-722 | Me | CH₂-c-Pr | 0 | OMe | |
| 1-723 | Me | (CH₂)₂OMe | 0 | OMe | |
| 1-724 | Me | (CH₂)₃OMe | 0 | OMe | |
| 1-725 | Me | (CH₂)₂OEt | 0 | OMe | |
| 1-726 | Me | (CH₂)₂OCH₂-c-Pr | 0 | OMe | |
| 1-727 | | | | | |
| 1-728 | Me | CH₂-c-Pr | 1 | OMe | |
| 1-729 | Me | (CH₂)₂OMe | 1 | OMe | |
| 1-730 | Me | (CH₂)₃OMe | 1 | OMe | |
| 1-731 | Me | (CH₂)₂OEt | 1 | OMe | |
| 1-732 | Me | (CH₂)₂OCH₂-c-Pr | 1 | OMe | |
| 1-733 | | | | | |
| 1-734 | Me | CH₂-c-Pr | 2 | OMe | |
| 1-735 | Me | (CH₂)₂OMe | 2 | OMe | |
| 1-736 | Me | (CH₂)₃OMe | 2 | OMe | |
| 1-737 | Me | (CH₂)₂OEt | 2 | OMe | |
| 1-738 | Me | (CH₂)₂OCH₂-c-Pr | 2 | OMe | |
| 1-739 | | | | | |
| 1-740 | Et | CH₂-c-Pr | 0 | OMe | |
| 1-741 | Et | (CH₂)₂OMe | 0 | OMe | |
| 1-742 | Et | (CH₂)₃OMe | 0 | OMe | |
| 1-743 | Et | (CH₂)₂OEt | 0 | OMe | |
| 1-744 | Et | (CH₂)₂OCH₂-c-Pr | 0 | OMe | |
| 1-745 | | | | | |
| 1-746 | Et | CH₂-c-Pr | 1 | OMe | |
| 1-747 | Et | (CH₂)₂OMe | 1 | OMe | |
| 1-748 | Et | (CH₂)₃OMe | 1 | OMe | |
| 1-749 | Et | (CH₂)₂OEt | 1 | OMe | |
| 1-750 | Et | (CH₂)₂OCH₂-c-Pr | 1 | OMe | |
| 1-751 | | | | | |
| 1-752 | Et | CH₂-c-Pr | 2 | OMe | |
| 1-753 | Et | (CH₂)₂OMe | 2 | OMe | |
| 1-754 | Et | (CH₂)₃OMe | 2 | OMe | |
| 1-755 | Et | (CH₂)₂OEt | 2 | OMe | |
| 1-756 | Et | (CH₂)₂OCH₂-c-Pr | 2 | OMe | |
| 1-757 | | | | | |
| 1-758 | CF₃ | CH₂-c-Pr | 0 | OMe | |
| 1-759 | CF₃ | (CH₂)₂OMe | 0 | OMe | |
| 1-760 | CF₃ | (CH₂)₃OMe | 0 | OMe | |
| 1-761 | CF₃ | (CH₂)₂OEt | 0 | OMe | |
| 1-762 | CF₃ | (CH₂)₂OCH₂-c-Pr | 0 | OMe | |
| 1-763 | | | | | |
| 1-764 | CF₃ | CH₂-c-Pr | 1 | OMe | |
| 1-765 | CF₃ | (CH₂)₂OMe | 1 | OMe | |
| 1-766 | CF₃ | (CH₂)₃OMe | 1 | OMe | |
| 1-767 | CF₃ | (CH₂)₂OEt | 1 | OMe | |
| 1-768 | CF₃ | (CH₂)₂OCH₂-c-Pr | 1 | OMe | |
| 1-769 | | | | | |
| 1-770 | CF₃ | CH₂-c-Pr | 2 | OMe | |
| 1-771 | CF₃ | (CH₂)₂OMe | 2 | OMe | |
| 1-772 | CF₃ | (CH₂)₃OMe | 2 | OMe | |
| 1-773 | CF₃ | (CH₂)₂OEt | 2 | OMe | |
| 1-774 | CF₃ | (CH₂)₂OCH₂-c-Pr | 2 | OMe | |
| 1-775 | | | | | |
| 1-776 | OMe | CH₂-c-Pr | 0 | OMe | |
| 1-777 | OMe | (CH₂)₂OMe | 0 | OMe | |
| 1-778 | OMe | (CH₂)₃OMe | 0 | OMe | |
| 1-779 | OMe | (CH₂)₂OEt | 0 | OMe | |
| 1-780 | OMe | (CH₂)₂OCH₂-c-Pr | 0 | OMe | |
| 1-781 | | | | | |
| 1-782 | OMe | CH₂-c-Pr | 1 | OMe | |
| 1-783 | OMe | (CH₂)₂OMe | 1 | OMe | |
| 1-784 | OMe | (CH₂)₃OMe | 1 | OMe | |
| 1-785 | OMe | (CH₂)₂OEt | 1 | OMe | |
| 1-786 | OMe | (CH₂)₂OCH₂-c-Pr | 1 | OMe | |
| 1-787 | | | | | |
| 1-788 | OMe | CH₂-c-Pr | 2 | OMe | |
| 1-789 | OMe | (CH₂)₂OMe | 2 | OMe | |
| 1-790 | OMe | (CH₂)₃OMe | 2 | OMe | |
| 1-791 | OMe | (CH₂)₂OEt | 2 | OMe | |
| 1-792 | OMe | (CH₂)₂OCH₂-c-Pr | 2 | OMe | |
| 1-793 | | | | | |
| 1-794 | OEt | CH₂-c-Pr | 0 | OMe | |
| 1-795 | OEt | (CH₂)₂OMe | 0 | OMe | |
| 1-796 | OEt | (CH₂)₃OMe | 0 | OMe | |
| 1-797 | OEt | (CH₂)₂OEt | 0 | OMe | |
| 1-798 | OEt | (CH₂)₂OCH₂-c-Pr | 0 | OMe | |
| 1-799 | | | | | |
| 1-800 | OEt | CH₂-c-Pr | 1 | OMe | |
| 1-801 | OEt | (CH₂)₂OMe | 1 | OMe | |
| 1-802 | OEt | (CH₂)₃OMe | 1 | OMe | |
| 1-803 | OEt | (CH₂)₂OEt | 1 | OMe | |
| 1-804 | OEt | (CH₂)₂OCH₂-c-Pr | 1 | OMe | |
| 1-805 | | | | | |
| 1-806 | OEt | CH₂-c-Pr | 2 | OMe | |
| 1-807 | OEt | (CH₂)₂OMe | 2 | OMe | |
| 1-808 | OEt | (CH₂)₃OMe | 2 | OMe | |
| 1-809 | OEt | (CH₂)₂OEt | 2 | OMe | |
| 1-810 | OEt | (CH₂)₂OCH₂-c-Pr | 2 | OMe | |
| 1-811 | | | | | |
| 1-812 | NO₂ | CH₂-c-Pr | 0 | OMe | |
| 1-813 | NO₂ | (CH₂)₂OMe | 0 | OMe | |
| 1-814 | NO₂ | (CH₂)₃OMe | 0 | OMe | |
| 1-815 | NO₂ | (CH₂)₂OEt | 0 | OMe | |
| 1-816 | NO₂ | (CH₂)₂OCH₂-c-Pr | 0 | OMe | |
| 1-817 | | | | | |
| 1-818 | NO₂ | CH₂-c-Pr | 1 | OMe | |
| 1-819 | N0₂ | (CH₂)₂OMe | 1 | OMe | |
| 1-820 | NO₂ | (CH₂)₃OMe | 1 | OMe | |
| 1-821 | NO₂ | (CH₂)₂OEt | 1 | OMe | |
| 1-822 | NO₂ | (CH₂)₂OCH₂-c-Pr | 1 | OMe | |
| 1-823 | | | | | |
| 1-824 | NO₂ | CH₂-c-Pr | 2 | OMe | |
| 1-825 | NO₂ | (CH₂)₂OMe | 2 | OMe | |
| 1-826 | NO₂ | (CH₂)₃OMe | 2 | OMe | |
| 1-827 | NO₂ | (CH₂)₂OEt | 2 | OMe | |
| 1-828 | NO₂ | (CH₂)₂OCH₂-c-Pr | 2 | OMe | |
| 1-829 | | | | | |
| 1-830 | SO₂Me | CH₂-c-Pr | 0 | OMe | |
| 1-831 | SO₂Me | (CH₂)₂OMe | 0 | OMe | |
| 1-832 | SO₂Me | (CH₂)₃OMe | 0 | OMe | |
| 1-833 | SO₂Me | (CH₂)₂OEt | 0 | OMe | |
| 1-834 | SO₂Me | (CH₂)₂OCH₂-c-Pr | 0 | OMe | |
| 1-835 | | | | | |
| 1-836 | SO₂Me | CH₂-c-Pr | 1 | OMe | |
| 1-837 | SO₂Me | (CH₂)₂OMe | 1 | OMe | |
| 1-838 | SO₂Me | (CH₂)₃OMe | 1 | OMe | |
| 1-839 | SO₂Me | (CH₂)₂OEt | 1 | OMe | |
| 1-840 | SO₂Me | (CH₂)₂OCH₂-c-Pr | 1 | OMe | |
| 1-841 | | | | | |
| 1-842 | SO₂Me | CH₂-c-Pr | 2 | OMe | |
| 1-843 | SO₂Me | (CH₂)₂OMe | 2 | OMe | |
| 1-844 | SO₂Me | (CH₂)₃OMe | 2 | OMe | |
| 1-845 | SO₂Me | (CH₂)₂OEt | 2 | OMe | |
| 1-846 | SO₂Me | (CH₂)₂OCH₂-c-Pr | 2 | OMe | |
| 1-847 | | | | | |
| 1-848 | CH₂OMe | CH₂-c-Pr | 0 | OMe | |
| 1-849 | CH₂OMe | (CH₂)₂OMe | 0 | OMe | |
| 1-850 | CH₂OMe | (CH₂)₃OMe | 0 | OMe | |
| 1-851 | CH₂OMe | (CH₂)₂OEt | 0 | OMe | |
| 1-852 | CH₂OMe | (CH₂)₂OCH₂-c-Pr | 0 | OMe | |
| 1-853 | | | | | |
| 1-854 | CH₂OMe | CH₂-c-Pr | 1 | OMe | |
| 1-855 | CH₂OMe | (CH₂)₂OMe | 1 | OMe | |
| 1-856 | CH₂OMe | (CH₂)₃OMe | 1 | OMe | |
| 1-857 | CH₂OMe | (CH₂)₂OEt | 1 | OMe | |
| 1-858 | CH₂OMe | (CH₂)₂OCH₂-c-Pr | 1 | OMe | |
| 1-859 | | | | | |
| 1-860 | CH₂OMe | CH₂-c-Pr | 2 | OMe | |
| 1-861 | CH₂OMe | (CH₂)₂OMe | 2 | OMe | |
| 1-862 | CH₂OMe | (CH₂)₃OMe | 2 | OMe | |
| 1-863 | CH₂OMe | (CH₂)₂OEt | 2 | OMe | |
| 1-864 | CH₂OMe | (CH₂)₂OCH₂-c-Pr | 2 | OMe | |
| 1-865 | | | | | |
| 1-866 | CH₂SO₂Me | CH₂-c-Pr | 0 | OMe | |
| 1-867 | CH₂SO₂Me | (CH₂)₂OMe | 0 | OMe | |
| 1-868 | CH₂SO₂Me | (CH₂)₃OMe | 0 | OMe | |
| 1-869 | CH₂SO₂Me | (CH₂)₂OEt | 0 | OMe | |
| 1-870 | CH₂SO₂Me | (CH₂)₂OCH₂-c-Pr | 0 | OMe | |
| 1-871 | | | | | |
| 1-872 | CH₂SO₂Me | CH₂-c-Pr | 1 | OMe | |
| 1-873 | CH₂SO₂Me | (CH₂)₂OMe | 1 | OMe | |
| 1-874 | CH₂SO₂Me | (CH₂)₃OMe | 1 | OMe | |
| 1-875 | CH₂SO₂Me | (CH₂)₂OEt | 1 | OMe | |
| 1-876 | CH₂SO₂Me | (CH₂)₂OCH₂-c-Pr | 1 | OMe | |
| 1-877 | | | | | |
| 1-878 | CH₂SO₂Me | CH₂-c-Pr | 2 | OMe | |
| 1-879 | CH₂SO₂Me | (CH₂)₂OMe | 2 | OMe | |
| 1-880 | CH₂SO₂Me | (CH₂)₃OMe | 2 | OMe | |
| 1-881 | CH₂SO₂Me | (CH₂)₂ OEt | 2 | OMe | |
| 1-882 | CH₂SO₂Me | (CH₂)₂OCH₂-c-Pr | 2 | OMe | |
| 1-883 | | | | | |
| 1-884 | CH₂O(CH₂)₂OMe | CH₂-c-Pr | 0 | OMe | |
| 1-885 | CH₂O(CH₂)₂OMe | (CH₂)₂OMe | 0 | OMe | |
| 1-886 | CH₂O(CH₂)₂OMe | (CH₂)₃OMe | 0 | OMe | |
| 1-887 | CH₂O(CH₂)₂OMe | (CH₂)₂OEt | 0 | OMe | |
| 1-888 | CH₂O(CH₂)₂OMe | (CH₂)₂OCH₂-c-Pr | 0 | OMe | |
| 1-889 | | | | | |
| 1-890 | CH₂O(CH₂)₂OMe | CH₂-c-Pr | 1 | OMe | |
| 1-891 | CH₂O(CH₂)₂OMe | (CH₂)₂OMe | 1 | OMe | |
| 1-892 | CH₂O(CH₂)₂OMe | (CH₂)₃OMe | 1 | OMe | |
| 1-893 | CH₂O(CH₂)₂OMe | (CH₂)₂OEt | 1 | OMe | |
| 1-894 | CH₂O(CH₂)₂OMe | (CH₂)₂OCH₂-c-Pr | 1 | OMe | |
| 1-895 | | | | | |
| 1-896 | CH₂O(CH₂)₂OMe | CH₂-c-Pr | 2 | OMe | |
| 1-897 | CH₂O(CH₂)₂OMe | (CH₂)₂OMe | 2 | OMe | |
| 1-898 | CH₂O(CH₂)₂OMe | (CH₂)₃OMe | 2 | OMe | |
| 1-899 | CH₂O(CH₂)₂OMe | (CH₂)₂OEt | 2 | OMe | |
| 1-900 | CH₂O(CH₂)₂OMe | (CH₂)₂OCH₂-c-Pr | 2 | OMe | |
| 1-901 | | | | | |
| 1-902 | CH₂O(CH₂)₂OEt | CH₂-c-Pr | 0 | OMe | |
| 1-903 | CH₂O(CH₂)₂OEt | (CH₂)₂OMe | 0 | OMe | |
| 1-904 | CH₂O (CH₂)₂OEt | (CH₂)₃OMe | 0 | OMe | |
| 1-905 | CH₂O(CH₂)₂OEt | (CH₂)₂OEt | 0 | OMe | |
| 1-906 | CH₂O(CH₂)₂OEt | (CH₂)₂OCH₂-c-Pr | 0 | OMe | |
| 1-907 | | | | | |
| 1-908 | CH₂O(CH₂)₂OEt | CH₂-c-Pr | 1 | OMe | |
| 1-909 | CH₂O(CH₂)₂OEt | (CH₂)₂OMe | 1 | OMe | |
| 1-910 | CH₂O(CH₂)₂OEt | (CH₂)₃OMe | 1 | OMe | |
| 1-911 | CH₂O(CH₂)₂OEt | (CH₂)₂OEt | 1 | OMe | |
| 1-912 | CH₂O(CH₂)₂OEt | (CH₂)₂OCH₂-c-Pr | 1 | OMe | |
| 1-913 | | | | | |
| 1-914 | CH₂O(CH₂)₂OEt | CH₂-c-Pr | 2 | OMe | |
| 1-915 | CH₂O(CH₂)₂OEt | (CH₂)₂OMe | 2 | OMe | |
| 1-916 | CH₂O(CH₂)₂OEt | (CH₂)₃OMe | 2 | OMe | |
| 1-917 | CH₂O(CH₂)₂OEt | (CH₂)₂OEt | 2 | OMe | |
| 1-918 | CH₂O(CH₂)₂OEt | (CH₂)₂OCH₂-c-Pr | 2 | OMe | |
| 1-919 | | | | | |
| 1-920 | CH₂O(CH₂)₃OMe | CH₂-c-Pr | 0 | OMe | |
| 1-921 | CH₂O(CH₂)₃OMe | (CH₂)₂OMe | 0 | OMe | |
| 1-922 | CH₂O(CH₂)₃OMe | (CH₂)₃OMe | 0 | OMe | |
| 1-923 | CH₂O(CH₂)₃OMe | (CH₂)₂OEt | 0 | OMe | |
| 1-924 | CH₂O(CH₂)₃OMe | (CH₂)₂OCH₂-c-Pr | 0 | OMe | |
| 1-925 | | | | | |
| 1-926 | CH₂O(CH₂)₃OMe | CH₂-c-Pr | 1 | OMe | |
| 1-927 | CH₂O(CH₂)₃OMe | (CH₂)₂OMe | 1 | OMe | |
| 1-928 | CH₂O(CH₂)₃OMe | (CH₂)₃OMe | 1 | OMe | |
| 1-929 | CH₂O(CH₂)₃OMe | (CH₂)₂OEt | 1 | OMe | |
| 1-930 | CH₂O(CH₂)₃OMe | (CH₂)₂OCH₂-c-Pr | 1 | OMe | |
| 1-931 | | | | | |
| 1-932 | CH₂O(CH₂)₃OMe | CH₂-c-Pr | 2 | OMe | |
| 1-933 | CH₂O(CH₂)₃OMe | (CH₂)₂OMe | 2 | OMe | |
| 1-934 | CH₂O(CH₂)₃OMe | (CH₂)₃OMe | 2 | OMe | |
| 1-935 | CH₂O(CH₂)₃OMe | (CH₂)₂OEt | 2 | OMe | |
| 1-936 | CH₂O(CH₂)₃OMe | (CH₂)₂OCH₂-c-Pr | 2 | OMe | |
| 1-937 | | | | | |
| 1-938 | CH₂OCH₂OMe | CH₂-c-Pr | 0 | OMe | |
| 1-939 | CH₂OCH₂OMe | (CH₂)₂OMe | 0 | OMe | |
| 1-940 | CH₂OCH₂OMe | (CH₂)₃OMe | 0 | OMe | |
| 1-941 | CH₂OCH₂OMe | (CH₂)₂OEt | 0 | OMe | |
| 1-942 | CH₂OCH₂OMe | (CH₂)₂OCH₂-c-Pr | 0 | OMe | |
| 1-943 | | | | | |
| 1-944 | CH₂OCH₂OMe | CH₂-c-Pr | 1 | OMe | |
| 1-945 | CH₂OCH₂OMe | (CH₂)₂OMe | 1 | OMe | |
| 1-946 | CH₂OCH₂OMe | (CH₂)₃OMe | 1 | OMe | |
| 1-947 | CH₂OCH₂OMe | (CH₂)₂OEt | 1 | OMe | |
| 1-948 | CH₂OCH₂OMe | (CH₂)₂OCH₂-c-Pr | 1 | OMe | |
| 1-949 | | | | | |
| 1-950 | CH₂OCH₂OMe | CH₂-c-Pr | 2 | OMe | |
| 1-951 | CH₂OCH₂OMe | (CH₂)₂OMe | 2 | OMe | |
| 1-952 | CH₂OCH₂OMe | (CH₂)₃OMe | 2 | OMe | |
| 1-953 | CH₂OCH₂OMe | (CH₂)₂OEt | 2 | OMe | |
| 1-954 | CH₂OCH₂OMe | (CH₂)₂OCH₂-c-Pr | 2 | OMe | |
| 1-955 | | | | | |
| 1-956 | CH₂OCH₂OEt | CH₂-c-Pr | 0 | OMe | |
| 1-957 | CH₂OCH₂OEt | (CH₂)₂OMe | 0 | OMe | |
| 1-958 | CH₂OCH₂OEt | (CH₂)₃OMe | 0 | OMe | |
| 1-959 | CH₂OCH₂O | (CH₂)₂OEt | 0 | OMe | |
| 1-960 | CH₂OCH₂OEt | (CH₂)₂OCH₂-c-Pr | 0 | OMe | |
| 1-961 | | | | | |
| 1-962 | CH₂OCH₂OEt | CH₂-c-Pr | 1 | OMe | |
| 1-963 | CH₂OCH₂OEt | (CH₂)₂OMe | 1 | OMe | |
| 1-964 | CH₂OCH₂OEt | (CH₂)₃OMe | 1 | OMe | |
| 1-965 | CH₂OCH₂OEt | (CH₂)₂OEt | 1 | OMe | |
| 1-966 | CH₂OCH₂OEt | (CH₂)₂OCH₂-c-Pr | 1 | OMe | |
| 1-967 | | | | | |
| 1-968 | CH₂OCH₂OEt | CH₂-c-Pr | 2 | OMe | |
| 1-969 | CH₂OCH₂OEt | (CH₂)₂OMe | 2 | OMe | |
| 1-970 | CH₂OCH₂OEt | (CH₂)₃OMe | 2 | OMe | |
| 1-971 | CH₂OCH₂OEt | (CH₂)₂OEt | 2 | OMe | |
| 1-972 | CH₂OCH₂OEt | (CH₂)₂OCH₂-c-Pr | 2 | OMe | |
| 1-973 | | | | | |
| 1-974 | CH₂O(CH₂)₂SO₂Me | CH₂-c-Pr | 0 | OMe | |
| 1-975 | CH₂O(CH₂)₂SO₂Me | (CH₂)₂OMe | 0 | OMe | |
| 1-976 | CH₂O(CH₂)₂SO₂Me | (CH₂)₃OMe | 0 | OMe | |
| 1-977 | CH₂O(CH₂)₂SO₂Me | (CH₂)₂OEt | 0 | OMe | |
| 1-978 | CH₂O(CH₂)₂SO₂Me | (CH₂)₂OCH₂-c-Pr | 0 | OMe | |
| 1-979 | | | | | |
| 1-980 | CH₂O(CH₂)₂SO₂Me | CH₂-c-Pr | 1 | OMe | |
| 1-981 | CH₂O(CH₂)₂SO₂Me | (CH₂)₂OMe | 1 | OMe | |
| 1-982 | CH₂O(CH₂)₂SO₂Me | (CH₂)₃OMe | 1 | OMe | |
| 1-983 | CH₂O(CH₂)₂SO₂Me | (CH₂)₂OEt | 1 | OMe | |
| 1-984 | CH₂O(CH₂)₂SO₂Me | (CH₂)₂OCH₂-c-Pr | 1 | OMe | |
| 1-985 | | | | | |
| 1-986 | CH₂O(CH₂)₂SO₂Me | CH₂-c-Pr | 2 | OMe | |
| 1-987 | CH₂O(CH₂)₂SO₂Me | (CH₂)₂OMe | 2 | OMe | |
| 1-988 | CH₂O(CH₂)₂SO₂Me | (CH₂)₃OMe | 2 | OMe | |
| 1-989 | CH₂O(CH₂)₂SO₂Me | (CH₂)₂OEt | 2 | OMe | |
| 1-990 | CH₂O(CH₂)₂SO₂Me | (CH₂)₂OCH₂-c-Pr | 2 | OMe | |
| 1-991 | | | | | |
| 1-992 | CH₂SO₂(CH₂)₂OMe | CH₂-c-Pr | 0 | OMe | |
| 1-993 | CH₂SO₂(CH₂)₂OMe | (CH₂)₂OMe | 0 | OMe | |
| 1-994 | CH₂SO₂(CH₂)₂OMe | (CH₂)₃OMe | 0 | OMe | |
| 1-995 | CH₂SO₂(CH₂)₂OMe | (CH₂)₂OEt | 0 | OMe | |
| 1-996 | CH₂SO₂(CH₂)₂OMe | (CH₂)₂OCH₂-c-Pr | 0 | OMe | |
| 1-997 | | | | | |
| 1-998 | CH₂SO₂(CH₂)₂OMe | CH₂-c-Pr | 1 | OMe | |
| 1-999 | CH₂SO₂(CH₂)₂OMe | (CH₂)₂OMe | 1 | OMe | |
| 1-1000 | CH₂SO₂(CH₂)₂OMe | (CH₂)₃OMe | 1 | OMe | |
| 1-1001 | CH₂SO₂(CH₂)₂OMe | (CH₂)₂OEt | 1 | OMe | |
| 1-1002 | CH₂SO₂(CH₂)₂OMe | (CH₂)₂OCH₂-c-Pr | 1 | OMe | |
| 1-1003 | | | | | |
| 1-1004 | CH₂SO₂(CH₂)₂OMe | CH₂-c-Pr | 2 | OMe | |
| 1-1005 | CH₂SO₂(CH₂)₂OMe | (CH₂)₂OMe | 2 | OMe | |
| 1-1006 | CH₂SO₂(CH₂)₂OMe | (CH₂)₃OMe | 2 | OMe | |
| 1-1007 | CH₂SO₂(CH₂)₂OMe | (CH₂)₂OEt | 2 | OMe | |
| 1-1008 | CH₂SO₂(CH₂)₂OMe | (CH₂)₂OCH₂-c-Pr | 2 | OMe | |
| 1-1009 | | | | | |
| 1-1010 | CH₂SO₂(CH₂)₂SO₂Me | CH₂-c-Pr | 0 | OMe | |
| 1-1011 | CH₂SO₂(CH₂)₂SO₂Me | (CH₂)₂OMe | 0 | OMe | |
| 1-1012 | CH₂SO₂(CH₂)₂SO₂Me | (CH₂)₃OMe | 0 | OMe | |
| 1-1013 | CH₂SO₂(CH₂)₂SO₂Me | (CH₂)₂OEt | 0 | OMe | |
| 1-1014 | CH₂SO₂(CH₂)₂SO₂Me | (CH₂)₂OCH₂-c-Pr | 0 | OMe | |
| 1-1015 | | | | | |
| 1-1016 | CH₂SO₂(CH₂)₂SO₂Me | CH₂-c-Pr | 1 | OMe | |
| 1-1017 | CH₂SO₂(CH₂)₂SO₂Me | (CH₂)₂OMe | 1 | OMe | |
| 1-1018 | CH₂SO₂(CH₂)₂SO₂Me | (CH₂)₃OMe | 1 | OMe | |
| 1-1019 | CH₂SO₂(CH₂)₂SO₂Me | (CH₂)₂OEt | 1 | OMe | |
| 1-1020 | CH₂SO₂(CH₂)₂SO₂Me | (CH₂)₂OCH₂-c-Pr | 1 | OMe | |
| 1-1021 | | | | | |
| 1-1022 | CH₂SO₂(CH₂)₂SO₂Me | CH₂-c-Pr | 2 | OMe | |
| 1-1023 | CH₂SO₂(CH₂)₂SO₂Me | (CH₂)₂OMe | 2 | OMe | |
| 1-1024 | CH₂SO₂(CH₂)₂SO₂Me | (CH₂)₃OMe | 2 | OMe | |
| 1-1025 | CH₂SO₂(CH₂)₂SO₂Me | (CH₂)₂OEt | 2 | OMe | |
| 1-1026 | CH₂SO₂(CH₂)₂SO₂Me | (CH₂)₂OCH₂-c-Pr | 2 | OMe | |
| 1-1027 | | | | | |
| 1-1028 | Cl | CH₂-c-Pr | 0 | F | |
| 1-1029 | Cl | (CH₂)₂OMe | 0 | F | |
| 1-1030 | Cl | (CH₂)₃OMe | 0 | F | |
| 1-1031 | Cl | (CH₂)₂OEt | 0 | F | |
| 1-1032 | Cl | (CH₂)₂OCH₂-c-Pr | 0 | F | |
| 1-1033 | | | | | |
| 1-1034 | Cl | CH₂-c-Pr | 1 | F | |
| 1-1035 | Cl | (CH₂)₂OMe | 1 | F | |
| 1-1036 | Cl | (CH₂)₃OMe | 1 | F | |
| 1-1037 | Cl | (CH₂)₂OEt | 1 | F | |
| 1-1038 | Cl | (CH₂)₂OCH₂-c-Pr | 1 | F | |
| 1-1039 | | | | | |
| 1-1040 | Cl | CH₂-c-Pr | 2 | F | |
| 1-1041 | Cl | (CH₂)₂OMe | 2 | F | |
| 1-1042 | Cl | (CH₂)₃OMe | 2 | F | |
| 1-1043 | Cl | (CH₂)₂OEt | 2 | F | |
| 1-1044 | Cl | (CH₂)₂OCH₂-c-Pr | 2 | F | |
| 1-1045 | | | | | |
| 1-1046 | Br | CH₂-c-Pr | 0 | F | |
| 1-1047 | Br | (CH₂)₂OMe | 0 | F | |
| 1-1048 | Br | (CH₂)₃OMe | 0 | F | |
| 1-1049 | Br | (CH₂)₂OEt | 0 | F | |
| 1-1050 | Br | (CH₂)₂OCH₂-c-Pr | 0 | F | |
| 1-1051 | | | | | |
| 1-1052 | Br | CH₂-c-Pr | 1 | F | |
| 1-1053 | Br | (CH₂)₂OMe | 1 | F | |
| 1-1054 | Br | (CH₂)₃OMe | 1 | F | |
| 1-1055 | Br | (CH₂)₂OEt | 1 | F | |
| 1-1056 | Br | (CH₂)₂OCH₂-c-Pr | 1 | F | |
| 1-1057 | | | | | |
| 1-1058 | Br | CH₂-c-Pr | 2 | F | |
| 1-1059 | Br | (CH₂)₂OMe | 2 | F | |
| 1-1060 | Br | (CH₂)₃OMe | 2 | F | |
| 1-1061 | Br | (CH₂)₂OEt | 2 | F | |
| 1-1062 | Br | (CH₂)₂OCH₂-c-Pr | 2 | F | |
| 1-1063 | | | | | |
| 1-1064 | Me | CH₂-c-Pr | 0 | F | |
| 1-1065 | Me | (CH₂)₂OMe | 0 | F | |
| 1-1066 | Me | (CH₂)₃OMe | 0 | F | |
| 1-1067 | Me | (CH₂)₂OEt | 0 | F | |
| 1-1068 | Me | (CH₂)₂OCH₂-c-Pr | 0 | F | |
| 1-1069 | | | | | |
| 1-1070 | Me | CH₂-c-Pr | 1 | F | |
| 1-1071 | Me | (CH₂)₂OMe | 1 | F | |
| 1-1072 | Me | (CH₂)₃OMe | 1 | F | |
| 1-1073 | Me | (CH₂)₂OEt | 1 | F | |
| 1-1074 | Me | (CH₂)₂OCH₂-c-Pr | 1 | F | |
| 1-1075 | | | | | |
| 1-1076 | Me | CH₂-c-Pr | 2 | F | |
| 1-1077 | Me | (CH₂)₂OMe | 2 | F | |
| 1-1078 | Me | (CH₂)₃OMe | 2 | F | |
| 1-1079 | Me | (CH₂)₂OEt | 2 | F | |
| 1-1080 | Me | (CH₂)₂OCH₂-c-Pr | 2 | F | |
| 1-1081 | | | | | |
| 1-1082 | Et | CH₂-c-Pr | 0 | F | |
| 1-1083 | Et | (CH₂)₂OMe | 0 | F | |
| 1-1084 | Et | (CH₂)₃OMe | 0 | F | |
| 1-1085 | Et | (CH₂)₂OEt | 0 | F | |
| 1-1086 | Et | (CH₂)₂OCH₂-c-Pr | 0 | F | |
| 1-1087 | | | | | |
| 1-1088 | Et | CH₂-c-Pr | 1 | F | |
| 1-1089 | Et | (CH₂)₂OMe | 1 | F | |
| 1-1090 | Et | (CH₂)₃OMe | 1 | F | |
| 1-1091 | Et | (CH₂)₂OEt | 1 | F | |
| 1-1092 | Et | (CH₂)₂OCH₂-c-Pr | 1 | F | |
| 1-1093 | | | | | |
| 1-1094 | Et | CH₂-c-Pr | 2 | F | |
| 1-1095 | Et | (CH₂)₂OMe | 2 | F | |
| 1-1096 | Et | (CH₂)₃OMe | 2 | F | |
| 1-1097 | Et | (CH₂)₂OEt | 2 | F | |
| 1-1098 | Et | (CH₂)₂OCH₂-c-Pr | 2 | F | |
| 1-1099 | | | | | |
| 1-1100 | CF₃ | CH₂-c-Pr | 0 | F | |
| 1-1101 | CF₃ | (CH₂)₂OMe | 0 | F | |
| 1-1102 | CF₃ | (CH₂)₃OMe | 0 | F | |
| 1-1103 | CF₃ | (CH₂)₂OEt | 0 | F | |
| 1-1104 | CF₃ | (CH₂)₂OCH₂-c-Pr | 0 | F | |
| 1-1105 | | | | | |
| 1-1106 | CF₃ | CH₂-c-Pr | 1 | F | |
| 1-1107 | CF₃ | (CH₂)₂OMe | 1 | F | |
| 1-1108 | CF₃ | (CH₂)₃OMe | 1 | F | |
| 1-1109 | CF₃ | (CH₂)₂OEt | 1 | F | |
| 1-1110 | CF₃ | (CH₂)₂OCH₂-c-Pr | 1 | F | |
| 1-1111 | | | | | |
| 1-1112 | CF₃ | CH₂-c-Pr | 2 | F | |
| 1-1113 | CF₃ | (CH₂)₂OMe | 2 | F | |
| 1-1114 | CF₃ | (CH₂)₃OMe | 2 | F | |
| 1-1115 | CF₃ | (CH₂)₂OEt | 2 | F | |
| 1-1116 | CF₃ | (CH₂)₂OCH₂-c-Pr | 2 | F | |
| 1-1117 | | | | | |
| 1-1118 | OMe | CH₂-c-Pr | 0 | F | |
| 1-1119 | OMe | (CH₂)₂OMe | 0 | F | |
| 1-1120 | OMe | (CH₂)₃OMe | 0 | F | |
| 1-1121 | OMe | (CH₂)₂OEt | 0 | F | |
| 1-1122 | OMe | (CH₂)₂OCH₂-c-Pr | 0 | F | |
| 1-1123 | | | | | |
| 1-1124 | OMe | CH₂-c-Pr | 1 | F | |
| 1-1125 | OMe | (CH₂)₂OMe | 1 | F | |
| 1-1126 | OMe | (CH₂)₃OMe | 1 | F | |
| 1-1127 | OMe | (CH₂)₂OEt | 1 | F | |
| 1-1128 | OMe | (CH₂)₂OCH₂-c-Pr | 1 | F | |
| 1-1129 | | | | | |
| 1-1130 | OMe | CH₂-c-Pr | 2 | F | |
| 1-1131 | OMe | (CH₂)₂OMe | 2 | F | |
| 1-1132 | OMe | (CH₂)₃OMe | 2 | F | |
| 1-1133 | OMe | (CH₂)₂OEt | 2 | F | |
| 1-1134 | OMe | (CH₂)₂OCH₂-c-Pr | 2 | F | |
| 1-1135 | | | | | |
| 1-1136 | OEt | CH₂-c-Pr | 0 | F | |
| 1-1137 | OEt | (CH₂)₂OMe | 0 | F | |
| 1-1138 | OEt | (CH₂)₃OMe | 0 | F | |
| 1-1139 | OEt | (CH₂)₂OEt | 0 | F | |
| 1-1140 | OEt | (CH₂)₂OCH₂-c-Pr | 0 | F | |
| 1-1141 | | | | | |
| 1-1142 | OEt | CH₂-c-Pr | 1 | F | |
| 1-1143 | OEt | (CH₂)₂OMe | 1 | F | |
| 1-1144 | OEt | (CH₂)₃OMe | 1 | F | |
| 1-1145 | OEt | (CH₂)₂OEt | 1 | F | |
| 1-1146 | OEt | (CH₂)₂OCH₂-c-Pr | 1 | F | |
| 1-1147 | | | | | |
| 1-1148 | OEt | CH₂-c-Pr | 2 | F | |
| 1-1149 | OEt | (CH₂)₂OMe | 2 | F | |
| 1-1150 | OEt | (CH₂)₃OMe | 2 | F | |
| 1-1151 | OEt | (CH₂)₂OEt | 2 | F | |
| 1-1152 | OEt | (CH₂)₂OCH₂-c-Pr | 2 | F | |
| 1-1153 | | | | | |
| 1-1154 | NO₂ | CH₂-c-Pr | 0 | F | |
| 1-1155 | NO₂ | (CH₂)₂OMe | 0 | F | |
| 1-1156 | NO₂ | (CH₂)₃OMe | 0 | F | |
| 1-1157 | NO₂ | (CH₂)₂OEt | 0 | F | |
| 1-1158 | NO₂ | (CH₂)₂OCH₂-c-Pr | 0 | F | |
| 1-1159 | | | | | |
| 1-1160 | NO₂ | CH₂-c-Pr | 1 | F | |
| 1-1161 | NO₂ | (CH₂)₂OMe | 1 | F | |
| 1-1162 | NO₂ | (CH₂)₃OMe | 1 | F | |
| 1-1163 | NO₂ | (CH₂)₂OEt | 1 | F | |
| 1-1164 | NO₂ | (CH₂)₂OCH₂-c-Pr | 1 | F | |
| 1-1165 | | | | | |
| 1-1166 | NO₂ | CH₂-c-Pr | 2 | F | |
| 1-1167 | NO₂ | (CH₂)₂OMe | 2 | F | |
| 1-1168 | NO₂ | (CH₂)₃OMe | 2 | F | |
| 1-1169 | NO₂ | (CH₂)₂OEt | 2 | F | |
| 1-1170 | NO₂ | (CH₂)₂OCH₂-c-Pr | 2 | F | |
| 1-1171 | | | | | |
| 1-1172 | SO₂Me | CH₂-c-Pr | 0 | F | |
| 1-1173 | SO₂Me | (CH₂)₂OMe | 0 | F | |
| 1-1174 | SO₂Me | (CH₂)₃OMe | 0 | F | |
| 1-1175 | SO₂Me | (CH₂)₂OEt | 0 | F | |
| 1-1176 | SO₂Me | (CH₂)₂OCH₂-c-Pr | 0 | F | |
| 1-1177 | | | | | |
| 1-1178 | SO₂Me | CH₂-c-Pr | 1 | F | |
| 1-1179 | SO₂Me | (CH₂)₂OMe | 1 | F | |
| 1-1180 | SO₂Me | (CH₂)₃OMe | 1 | F | |
| 1-1181 | SO₂Me | (CH₂)₂OEt | 1 | F | |
| 1-1182 | SO₂Me | (CH₂)₂OCH₂-c-Pr | 1 | F | |
| 1-1183 | | | | | |
| 1-1184 | SO₂Me | CH₂-c-Pr | 2 | F | |
| 1-1185 | SO₂Me | (CH₂)₂OMe | 2 | F | |
| 1-1186 | SO₂Me | (CH₂)₃OMe | 2 | F | |
| 1-1187 | SO₂Me | (CH₂)₂OEt | 2 | F | |
| 1-1188 | SO₂Me | (CH₂)₂OCH₂-c-Pr | 2 | F | |
| 1-1189 | | | | | |
| 1-1190 | CH₂OMe | CH₂-c-Pr | 0 | F | |
| 1-1191 | CH₂OMe | (CH₂)₂OMe | 0 | F | |
| 1-1192 | CH₂OMe | (CH₂)₃OMe | 0 | F | |
| 1-1193 | CH₂OMe | (CH₂)₂OEt | 0 | F | |
| 1-1194 | CH₂OMe | (CH₂)₂OCH₂-c-Pr | 0 | F | |
| 1-1195 | | | | | |
| 1-1196 | CH₂OMe | CH₂-c-Pr | 1 | F | |
| 1-1197 | CH₂OMe | (CH₂)₂OMe | 1 | F | |
| 1-1198 | CH₂OMe | (CH₂)₃OMe | 1 | F | |
| 1-1199 | CH₂OMe | (CH₂)₂OEt | 1 | F | |
| 1-1200 | CH₂OMe | (CH₂)₂OCH₂-c-Pr | 1 | F | |
| 1-1201 | | | | | |
| 1-1202 | CH₂OMe | CH₂-c-Pr | 2 | F | |
| 1-1203 | CH₂OMe | (CH₂)₂OMe | 2 | F | |
| 1-1204 | CH₂OMe | (CH₂)₃OMe | 2 | F | |
| 1-1205 | CH₂OMe | (CH₂)₂OEt | 2 | F | |
| 1-1206 | CH₂OMe | (CH₂)₂OCH₂-c-Pr | 2 | F | |
| 1-1207 | | | | | |
| 1-1208 | CH₂SO₂Me | CH₂-c-Pr | 0 | F | |
| 1-1209 | CH₂SO₂Me | (CH₂)₂OMe | 0 | F | |
| 1-1210 | CH₂SO₂Me | (CH₂)₃OMe | 0 | F | |
| 1-1211 | CH₂SO₂Me | (CH₂)₂OEt | 0 | F | |
| 1-1212 | CH₂SO₂Me | (CH₂)₂OCH₂-c-Pr | 0 | F | |
| 1-1213 | | | | | |
| 1-1214 | CH₂SO₂Me | CH₂-c-Pr | 1 | F | |
| 1-1215 | CH₂SO₂Me | (CH₂)₂OMe | 1 | F | |
| 1-1216 | CH₂SO₂Me | (CH₂)₃OMe | 1 | F | |
| 1-1217 | CH₂SO₂Me | (CH₂)₂OEt | 1 | F | |
| 1-1218 | CH₂SO₂Me | (CH₂)₂OCH₂-c-Pr | 1 | F | |
| 1-1219 | | | | | |
| 1-1220 | CH₂SO₂Me | CH₂-c-Pr | 2 | F | |
| 1-1221 | CH₂SO₂Me | (CH₂)₂OMe | 2 | F | |
| 1-1222 | CH₂SO₂Me | (CH₂)₃OMe | 2 | F | |
| 1-1223 | CH₂SO₂Me | (CH₂)₂OEt | 2 | F | |
| 1-1224 | CH₂SO₂Me | (CH₂)₂OCH₂-c-Pr | 2 | F | |
| 1-1225 | | | | | |
| 1-1226 | CH₂O(CH₂)₂OMe | CH₂-c-Pr | 0 | F | |
| 1-1227 | CH₂O(CH₂)₂OMe | (CH₂)₂OMe | 0 | F | |
| 1-1228 | CH₂O(CH₂)₂OMe | (CH₂)₃OMe | 0 | F | |
| 1-1229 | CH₂O(CH₂)₂OMe | (CH₂)₂OEt | 0 | F | |
| 1-1230 | CH₂O(CH₂)₂OMe | (CH₂)₂OCH₂-c-Pr | 0 | F | |
| 1-1231 | | | | | |
| 1-1232 | CH₂O(CH₂)₂OMe | CH₂-c-Pr | 1 | F | |
| 1-1233 | CH₂O(CH₂)₂OMe | (CH₂)₂OMe | 1 | F | |
| 1-1234 | CH₂O(CH₂)₂OMe | (CH₂)₃OMe | 1 | F | |
| 1-1235 | CH₂O(CH₂)₂OMe | (CH₂)₂OEt | 1 | F | |
| 1-1236 | CH₂O(CH₂)₂OMe | (CH₂)₂OCH₂-c-Pr | 1 | F | |
| 1-1237 | | | | | |
| 1-1238 | CH₂O(CH₂)₂OMe | CH₂-c-Pr | 2 | F | |
| 1-1239 | CH₂O(CH₂)₂OMe | (CH₂)₂OMe | 2 | F | |
| 1-1240 | CH₂O(CH₂)₂OMe | (CH₂)₃OMe | 2 | F | |
| 1-1241 | CH₂O(CH₂)₂OMe | (CH₂)₂OEt | 2 | F | |
| 1-1242 | CH₂O(CH₂)₂OMe | (CH₂)₂OCH₂-c-Pr | 2 | F | |
| 1-1243 | | | | | |
| 1-1244 | CH₂O(CH₂)₂OEt | CH₂-c-Pr | 0 | F | |
| 1-1245 | CH₂O(CH₂)₂OEt | (CH₂)₂OMe | 0 | F | |
| 1-1246 | CH₂O(CH₂)₂OEt | (CH₂)₃OMe | 0 | F | |
| 1-1247 | CH₂O(CH₂)₂OEt | (CH₂)₂OEt | 0 | F | |
| 1-1248 | CH₂O(CH₂)₂OEt | (CH₂)₂OCH₂-c-Pr | 0 | F | |
| 1-1249 | | | | | |
| 1-1250 | CH₂O(CH₂)₂OEt | CH₂-c-Pr | 1 | F | |
| 1-1251 | CH₂O(CH₂)₂OEt | (CH₂)₂OMe | 1 | F | |
| 1-1252 | CH₂O(CH₂)₂OEt | (CH₂)₃OMe | 1 | F | |
| 1-1253 | CH₂O(CH₂)₂OEt | (CH₂)₂OEt | 1 | F | |
| 1-1254 | CH₂O(CH₂)₂OEt | (CH₂)₂OCH₂-c-Pr | 1 | F | |
| 1-1255 | | | | | |
| 1-1256 | CH₂O(CH₂)₂OEt | CH₂-c-Pr | 2 | F | |
| 1-1257 | CH₂O(CH₂)₂OEt | (CH₂)₂OMe | 2 | F | |
| 1-1258 | CH₂O(CH₂)₂OEt | (CH₂)₃OMe | 2 | F | |
| 1-1259 | CH₂O(CH₂)₂OEt | (CH₂)₂OEt | 2 | F | |
| 1-1260 | CH₂O(CH₂)₂OEt | (CH₂)₂OCH₂-c-Pr | 2 | F | |
| 1-1261 | | | | | |
| 1-1262 | CH₂O(CH₂)₃OMe | CH₂-c-Pr | 0 | F | |
| 1-1263 | CH₂O(CH₂)₃OMe | (CH₂)₂OMe | 0 | F | |
| 1-1264 | CH₂O(CH₂)₃OMe | (CH₂)₃OMe | 0 | F | |
| 1-1265 | CH₂O(CH₂)₃OMe | (CH₂)₂OEt | 0 | F | |
| 1-1266 | CH₂O(CH₂)₃OMe | (CH₂)₂OCH₂-c-Pr | 0 | F | |
| 1-1267 | | | | | |
| 1-1268 | CH₂O(CH₂)₃OMe | CH₂-c-Pr | 1 | F | |
| 1-1269 | CH₂O(CH₂)₃OMe | (CH₂)₂OMe | 1 | F | |
| 1-1270 | CH₂O(CH₂)₃OMe | (CH₂)₃OMe | 1 | F | |
| 1-1271 | CH₂O(CH₂)₃OMe | (CH₂)₂OEt | 1 | F | |
| 1-1272 | CH₂O(CH₂)₃OMe | (CH₂)₂OCH₂-c-Pr | 1 | F | |
| 1-1273 | | | | | |
| 1-1274 | CH₂O(CH₂)₃OMe | CH₂-c-Pr | 2 | F | |
| 1-1275 | CH₂O(CH₂)₃OMe | (CH₂)₂OMe | 2 | F | |
| 1-1276 | CH₂O(CH₂)₃OMe | (CH₂)₃OMe | 2 | F | |
| 1-1277 | CH₂O(CH₂)₃OMe | (CH₂)₂OEt | 2 | F | |
| 1-1278 | CH₂O(CH₂)₃OMe | (CH₂)₂OCH₂-c-Pr | 2 | F | |
| 1-1279 | | | | | |
| 1-1280 | CH₂OCH₂OMe | CH₂-c-Pr | 0 | F | |
| 1-1281 | CH₂OCH₂OMe | (CH₂)₂OMe | 0 | F | |
| 1-1282 | CH₂OCH₂OMe | (CH₂)₃OMe | 0 | F | |
| 1-1283 | CH₂OCH₂OMe | (CH₂)₂OEt | 0 | F | |
| 1-1284 | CH₂OCH₂OMe | (CH₂)₂OCH₂-c-Pr | 0 | F | |
| 1-1285 | | | | | |
| 1-1286 | CH₂OCH₂OMe | CH₂-c-Pr | 1 | F | |
| 1-1287 | CH₂OCH₂OMe | (CH₂)₂OMe | 1 | F | |
| 1-1288 | CH₂OCH₂OMe | (CH₂)₃OMe | 1 | F | |
| 1-1289 | CH₂OCH₂OMe | (CH₂)₂OEt | 1 | F | |
| 1-1290 | CH₂OCH₂OMe | (CH₂)₂OCH₂-c-Pr | 1 | F | |
| 1-1291 | | | | | |
| 1-1292 | CH₂OCH₂OMe | CH₂-c-Pr | 2 | F | |
| 1-1293 | CH₂OCH₂OMe | (CH₂)₂OMe | 2 | F | |
| 1-1294 | CH₂OCH₂OMe | (CH₂)₃OMe | 2 | F | |
| 1-1295 | CH₂OCH₂OMe | (CH₂)₂OEt | 2 | F | |
| 1-1296 | CH₂OCH₂OMe | (CH₂)₂OCH₂-c-Pr | 2 | F | |
| 1-1297 | | | | | |
| 1-1298 | CH₂OCH₂OEt | CH₂-c-Pr | 0 | F | |
| 1-1299 | CH₂OCH₂OEt | (CH₂)₂OMe | 0 | F | |
| 1-1300 | CH₂OCH₂OEt | (CH₂)₃OMe | 0 | F | |
| 1-1301 | CH₂OCH₂OEt | (CH₂)₂OEt | 0 | F | |
| 1-1302 | CH₂OCH₂OEt | (CH₂)₂OCH₂-c-Pr | 0 | F | |
| 1-1303 | | | | | |
| 1-1304 | CH₂OCH₂OEt | CH₂-c-Pr | 1 | F | |
| 1-1305 | CH₂OCH₂OEt | (CH₂)₂OMe | 1 | F | |
| 1-1306 | CH₂OCH₂OEt | (CH₂)₃OMe | 1 | F | |
| 1-1307 | CH₂OCH₂OEt | (CH₂)₂OEt | 1 | F | |
| 1-1308 | CH₂OCH₂OEt | (CH₂)₂OCH₂-c-Pr | 1 | F | |
| 1-1309 | | | | | |
| 1-1310 | CH₂OCH₂OEt | CH₂-c-Pr | 2 | F | |
| 1-1311 | CH₂OCH₂OEt | (CH₂)₂OMe | 2 | F | |
| 1-1312 | CH₂OCH₂OEt | (CH₂)₃OMe | 2 | F | |
| 1-1313 | CH₂OCH₂OEt | (CH₂)₂OEt | 2 | F | |
| 1-1314 | CH₂OCH₂OEt | (CH₂)₂OCH₂-c-Pr | 2 | F | |
| 1-1315 | | | | | |
| 1-1316 | CH₂O(CH₂)₂SO₂Me | CH₂-c-Pr | 0 | F | |
| 1-1317 | CH₂O(CH₂)₂SO₂Me | (CH₂)₂OMe | 0 | F | |
| 1-1318 | CH₂O(CH₂)₂SO₂Me | (CH₂)₃OMe | 0 | F | |
| 1-1319 | CH₂O(CH₂)₂SO₂Me | (CH₂)₂OEt | 0 | F | |
| 1-1320 | CH₂O(CH₂)₂SO₂Me | (CH₂)₂OCH₂-c-Pr | 0 | F | |
| 1-1321 | | | | | |
| 1-1322 | CH₂O(CH₂)₂SO₂Me | CH₂-c-Pr | 1 | F | |
| 1-1323 | CH₂O(CH₂)₂SO₂Me | (CH₂)₂OMe | 1 | F | |
| 1-1324 | CH₂O(CH₂)₂SO₂Me | (CH₂)₃OMe | 1 | F | |
| 1-1325 | CH₂O(CH₂)₂SO₂Me | (CH₂)₂OEt | 1 | F | |
| 1-1326 | CH₂O(CH₂)₂SO₂Me | (CH₂)₂OCH₂-c-Pr | 1 | F | |
| 1-1327 | | | | | |
| 1-1328 | CH₂O(CH₂)₂SO₂Me | CH₂-c-Pr | 2 | F | |
| 1-1329 | CH₂O(CH₂)₂SO₂Me | (CH₂)₂OMe | 2 | F | |
| 1-1330 | CH₂O(CH₂)₂SO₂Me | (CH₂)₃OMe | 2 | F | |
| 1-1331 | CH₂O(CH₂)₂SO₂Me | (CH₂)₂OEt | 2 | F | |
| 1-1332 | CH₂O(CH₂)₂SO₂Me | (CH₂)₂OCH₂-c-Pr | 2 | F | |
| 1-1333 | | | | | |
| 1-1334 | CH₂SO₂(CH₂)₂OMe | CH₂-c-Pr | 0 | F | |
| 1-1335 | CH₂SO₂(CH₂)₂OMe | (CH₂)₂OMe | 0 | F | |
| 1-1336 | CH₂SO₂(CH₂)₂OMe | (CH₂)₃OMe | 0 | F | |
| 1-1337 | CH₂SO₂(CH₂)₂OMe | (CH₂)₂OEt | 0 | F | |
| 1-1338 | CH₂SO₂(CH₂)₂OMe | (CH₂)₂OCH₂-c-Pr | 0 | F | |
| 1-1339 | | | | | |
| 1-1340 | CH₂SO₂(CH₂)₂OMe | CH₂-c-Pr | 1 | F | |
| 1-1341 | CH₂SO₂(CH₂)₂OMe | (CH₂)₂OMe | 1 | F | |
| 1-1342 | CH₂SO₂(CH₂)₂OMe | (CH₂)₃OMe | 1 | F | |
| 1-1343 | CH₂SO₂(CH₂)₂OMe | (CH₂)₂OEt | 1 | F | |
| 1-1344 | CH₂SO₂(CH₂)₂OMe | (CH₂)₂OCH₂-c-Pr | 1 | F | |
| 1-1345 | | | | | |
| 1-1346 | CH₂SO₂(CH₂)₂OMe | CH₂-c-Pr | 2 | F | |
| 1-1347 | CH₂SO₂(CH₂)₂OMe | (CH₂)₂OMe | 2 | F | |
| 1-1348 | CH₂SO₂(CH₂)₂OMe | (CH₂)₃OMe | 2 | F | |
| 1-1349 | CH₂SO₂(CH₂)₂OMe | (CH₂)₂OEt | 2 | F | |
| 1-1350 | CH₂SO₂(CH₂)₂OMe | (CH₂)₂OCH₂-c-Pr | 2 | F | |
| 1-1351 | | | | | |
| 1-1352 | CH₂SO₂(CH₂)₂SO₂Me | CH₂-c-Pr | 0 | F | |
| 1-1353 | CH₂SO₂(CH₂)₂SO₂Me | (CH₂)₂OMe | 0 | F | |
| 1-1354 | CH₂SO₂(CH₂)₂SO₂Me | (CH₂)₃OMe | 0 | F | |
| 1-1355 | CH₂SO₂(CH₂)₂SO₂Me | (CH₂)₂OEt | 0 | F | |
| 1-1356 | CH₂SO₂(CH₂)₂SO₂Me | (CH₂)₂OCH₂-c-Pr | 0 | F | |
| 1-1357 | | | | | |
| 1-1358 | CH₂SO₂(CH₂)₂SO₂Me | CH₂-c-Pr | 1 | F | |
| 1-1359 | CH₂SO₂(CH₂)₂SO₂Me | (CH₂)₂OMe | 1 | F | |
| 1-1360 | CH₂SO₂(CH₂)₂SO₂Me | (CH₂)₃OMe | 1 | F | |
| 1-1361 | CH₂SO₂(CH₂)₂SO₂Me | (CH₂)₂OEt | 1 | F | |
| 1-1362 | CH₂SO₂(CH₂)₂SO₂Me | (CH₂)₂OCH₂-c-Pr | 1 | F | |
| 1-1363 | | | | | |
| 1-1364 | CH₂SO₂(CH₂)₂SO₂Me | CH₂-c-Pr | 2 | F | |
| 1-1365 | CH₂SO₂(CH₂)₂SO₂Me | (CH₂)₂OMe | 2 | F | |
| 1-1366 | CH₂SO₂(CH₂)₂SO₂Me | (CH₂)₃OMe | 2 | F | |
| 1-1367 | CH₂SO₂(CH₂)₂SO₂Me | (CH₂)₂OEt | 2 | F | |
| 1-1368 | CH₂SO₂(CH₂)₂SO₂Me | (CH₂)₂OCH₂-c-Pr | 2 | F | |
| 1-1369 | | | | | |
| 1-1370 | Cl | CH₂-c-Pr | 0 | Cl | 7.48 (d, 1H), 7.25 (d, 1H), 3.60 (s, 3H), 2.84 (d, 2H), 1.02 - 0.95 (m, 2H), 0.77 (m, 2H), 0.52 - 0.45 (m, 4H), 0.15 (m, 2H) |
| 1-1371 | Cl | (CH₂)₂OMe | 0 | Cl | |
| 1-1372 | Cl | (CH₂)₃OMe | 0 | Cl | |
| 1-1373 | Cl | (CH₂)₂OEt | 0 | Cl | |
| 1-1374 | Cl | (CH₂)₂OCH₂-c-Pr | 0 | Cl | |
| 1-1375 | | | | | |
| 1-1376 | Cl | CH₂-c-Pr | 1 | Cl | |
| 1-1377 | Cl | (CH₂)₂OMe | 1 | Cl | |
| 1-1378 | Cl | (CH₂)₃OMe | 1 | Cl | |
| 1-1379 | Cl | (CH₂)₂OEt | 1 | Cl | |
| 1-1380 | Cl | (CH₂)₂OCH₂-c-Pr | 1 | Cl | |
| 1-1381 | | | | | |
| 1-1382 | Cl | CH₂-c-Pr | 2 | Cl | 7.62 (d, 1H), 7.44 (d, 1H), 3.61 (s, 3H), 3.38 (d, 2H), 1.15 (m, 1H), 0.97 (m, 1H), 0.79 (m, 2H), 0.68 - 0.52 (m, 4H), 0.28 (m, 2H) |
| 1-1383 | Cl | (CH₂)₂OMe | 2 | Cl | |
| 1-1384 | Cl | (CH₂)₃OMe | 2 | Cl | |
| 1-1385 | Cl | (CH₂)₂OEt | 2 | Cl | |
| 1-1386 | Cl | (CH₂)₂OCH₂-c-Pr | 2 | Cl | |
| 1-1387 | | | | | |
| 1-1388 | Br | CH₂-c-Pr | 0 | Cl | |
| 1-1389 | Br | (CH₂)₂OMe | 0 | Cl | |
| 1-1390 | Br | (CH₂)₃OMe | 0 | Cl | |
| 1-1391 | Br | (CH₂)₂OEt | 0 | Cl | |
| 1-1392 | Br | (CH₂)₂OCH₂-c-Pr | 0 | Cl | |
| 1-1393 | | | | | |
| 1-1394 | Br | CH₂-c-Pr | 1 | Cl | |
| 1-1395 | Br | (CH₂)₂OMe | 1 | Cl | |
| 1-1396 | Br | (CH₂)₃OMe | 1 | Cl | |
| 1-1397 | Br | (CH₂)₂OEt | 1 | Cl | |
| 1-1398 | Br | (CH₂)₂OCH₂-c-Pr | 1 | Cl | |
| 1-1399 | | | | | |
| 1-1400 | Br | CH₂-c-Pr | 2 | Cl | |
| 1-1401 | Br | (CH₂)₂OMe | 2 | Cl | |
| 1-1402 | Br | (CH₂)₃OMe | 2 | Cl | |
| 1-1403 | Br | (CH₂)₂OEt | 2 | Cl | |
| 1-1404 | Br | (CH₂)₂OCH₂-c-Pr | 2 | Cl | |
| 1-1405 | | | | | |
| 1-1406 | Me | CH₂-c-Pr | 0 | Cl | 7.40 (d, 1H), 7.21 (d, 1H), 3.58 (s, 3H), 2.75 (d, 2H), 2.62 (s, 3H), 0.99 - 0.90 (m, 2H), 0.77 (m, 2H), 0.51 (m, 4H), 0.13 (m, 2H) |
| 1-1407 | Me | (CH₂)₂OMe | 0 | Cl | 7.41 (d, 1H), 7.22 (d, 1H), 3.59 (s, 3H), 3.47 (t, 2H), 3.31 (s, 3H), 3.02 (t, 2H), 2.60 (s, 3H), 0.94 (m, 1H), 0.77 (m, 2H), 0.52 (m, 2H) |
| 1-1408 | Me | (CH₂)₃OMe | 0 | Cl | |
| 1-1409 | Me | (CH₂)₂OEt | 0 | Cl | |
| 1-1410 | Me | (CH₂)₂OCH₂-c-Pr | 0 | Cl | |
| 1-1411 | | | | | |
| 1-1412 | Me | CH₂-c-Pr | 1 | Cl | |
| 1-1413 | Me | (CH₂)₂OMe | 1 | Cl | 7.33 (s, 2H), 3.92 (m, 1H), 3.76 (m, 1H), 3.60 - 3.54 (m, 1H), 3.59 (s, 3H), 3.39 (s, 3H), 3.32 (m, 1H), 2.64 (s, 3H), 0.94 (m, 1H), 0.79 (m, 2H), 0.57 (m, 2H) |
| 1-1414 | Me | (CH₂)₃OMe | 1 | Cl | |
| 1-1415 | Me | (CH₂)₂OEt | 1 | Cl | |
| 1-1416 | Me | (CH₂)₂OCH₂-c-Pr | 1 | Cl | |
| 1-1417 | | | | | |
| 1-1418 | Me | CH₂-c-Pr | 2 | Cl | 7.51 (d, 1H), 7.41 (d, 1H), 3.60 (s, 3H), 3.37 (d, 2H), 2.77 (s, 3H), 1.08 (m, 1H), 0.88 (m, 1H), 0.78 (m, 2H), 0.62 - 0.51 (m, 4H), 0.26 (m, 2H) |
| 1-1419 | Me | (CH₂)₂OMe | 2 | Cl | 7.51 (d, 1H), 7.39 (d, 1H), 3.83 (t, 2H), 3.71 (t, 2H), 3.59 (s, 3H), 3.22 (s, 3H), 2.72 (s, 3H), 0.88 (m, 1H), 0.78 (m, 2H), 0.54 (m, 2H) |
| 1-1420 | Me | (CH₂)₃OMe | 2 | Cl | |
| 1-1421 | Me | (CH₂)₂OEt | 2 | Cl | |
| 1-1422 | Me | (CH₂)₂OCH₂-c-Pr | 2 | Cl | |
| 1-1423 | | | | | |
| 1-1424 | Et | CH₂-c-Pr | 0 | Cl | |
| 1-1425 | Et | (CH₂)₂OMe | 0 | Cl | |
| 1-1426 | Et | (CH₂)₃OMe | 0 | Cl | |
| 1-1427 | Et | (CH₂)₂OEt | 0 | Cl | |
| 1-1428 | Et | (CH₂)₂OCH₂-c-Pr | 0 | Cl | |
| 1-1429 | | | | | |
| 1-1430 | Et | CH₂-c-Pr | 1 | Cl | |
| 1-1431 | Et | (CH₂)₂OMe | 1 | Cl | |
| 1-1432 | Et | (CH₂)₃OMe | 1 | Cl | |
| 1-1433 | Et | (CH₂)₂OEt | 1 | Cl | |
| 1-1434 | Et | (CH₂)₂OCH₂-c-Pr | 1 | Cl | |
| 1-1435 | | | | | |
| 1-1436 | Et | CH₂-c-Pr | 2 | Cl | |
| 1-1437 | Et | (CH₂)₂OMe | 2 | Cl | |
| 1-1438 | Et | (CH₂)₃OMe | 2 | Cl | |
| 1-1439 | Et | (CH₂)₂OEt | 2 | Cl | |
| 1-1440 | Et | (CH₂)₂OCH₂-c-Pr | 2 | Cl | |
| 1-1441 | | | | | |
| 1-1442 | CF₃ | CH₂-c-Pr | 0 | Cl | |
| 1-1443 | CF₃ | (CH₂)₂OMe | 0 | Cl | |
| 1-1444 | CF₃ | (CH₂)₃OMe | 0 | Cl | |
| 1-1445 | CF₃ | (CH₂)₂OEt | 0 | Cl | |
| 1-1446 | CF₃ | (CH₂)₂OCH₂-c-Pr | 0 | Cl | |
| 1-1447 | | | | | |
| 1-1448 | CF₃ | CH₂-c-Pr | 1 | Cl | |
| 1-1449 | CF₃ | (CH₂)₂OMe | 1 | Cl | |
| 1-1450 | CF₃ | (CH₂)₃OMe | 1 | Cl | |
| 1-1451 | CF₃ | (CH₂)₂OEt | 1 | Cl | |
| 1-1452 | CF₃ | (CH₂)₂OCH₂-c-Pr | 1 | Cl | |
| 1-1453 | | | | | |
| 1-1454 | CF₃ | CH₂-c-Pr | 2 | Cl | |
| 1-1455 | CF₃ | (CH₂)₂OMe | 2 | Cl | |
| 1-1456 | CF₃ | (CH₂)₃OMe | 2 | Cl | |
| 1-1457 | CF₃ | (CH₂)₂OEt | 2 | Cl | |
| 1-1458 | CF₃ | (CH₂)₂OCH₂-c-Pr | 2 | Cl | |
| 1-1459 | | | | | |
| 1-1460 | OMe | CH₂-c-Pr | 0 | Cl | |
| 1-1461 | OMe | (CH₂)₂OMe | 0 | Cl | |
| 1-1462 | OMe | (CH₂)₃OMe | 0 | Cl | |
| 1-1463 | OMe | (CH₂)₂OEt | 0 | Cl | |
| 1-1464 | OMe | (CH₂)₂OCH₂-c-Pr | 0 | Cl | |
| 1-1465 | | | | | |
| 1-1466 | OMe | CH₂-c-Pr | 1 | Cl | |
| 1-1467 | OMe | (CH₂)₂OMe | 1 | Cl | |
| 1-1468 | OMe | (CH₂)₃OMe | 1 | Cl | |
| 1-1469 | OMe | (CH₂)₂OEt | 1 | Cl | |
| 1-1470 | OMe | (CH₂)₂OCH₂-c-Pr | 1 | Cl | |
| 1-1471 | | | | | |
| 1-1472 | OMe | CH₂-c-Pr | 2 | Cl | |
| 1-1473 | OMe | (CH₂)₂OMe | 2 | Cl | |
| 1-1474 | OMe | (CH₂)₃OMe | 2 | Cl | |
| 1-1475 | OMe | (CH₂)₂OEt | 2 | Cl | |
| 1-1476 | OMe | (CH₂)₂OCH₂-c-Pr | 2 | Cl | |
| 1-1477 | | | | | |
| 1-1478 | OEt | CH₂-c-Pr | 0 | Cl | |
| 1-1479 | OEt | (CH₂)₂OMe | 0 | Cl | |
| 1-1480 | OEt | (CH₂)₃OMe | 0 | Cl | |
| 1-1481 | OEt | (CH₂)₂OEt | 0 | Cl | |
| 1-1482 | OEt | (CH₂)₂OCH₂-c-Pr | 0 | Cl | |
| 1-1483 | | | | | |
| 1-1484 | OEt | CH₂-c-Pr | 1 | Cl | |
| 1-1485 | OEt | (CH₂)₂OMe | 1 | Cl | |
| 1-1486 | OEt | (CH₂)₃OMe | 1 | Cl | |
| 1-1487 | OEt | (CH₂)₂OEt | 1 | Cl | |
| 1-1488 | OEt | (CH₂)₂OCH₂-c-Pr | 1 | Cl | |
| 1-1489 | | | | | |
| 1-1490 | OEt | CH₂-c-Pr | 2 | Cl | |
| 1-1491 | OEt | (CH₂)₂OMe | 2 | Cl | |
| 1-1492 | OEt | (CH₂)₃OMe | 2 | Cl | |
| 1-1493 | OEt | (CH₂)₂OEt | 2 | Cl | |
| 1-1494 | OEt | (CH₂)₂OCH₂-c-Pr | 2 | Cl | |
| 1-1495 | | | | | |
| 1-1496 | NO₂ | CH₂-c-Pr | 0 | Cl | |
| 1-1497 | NO₂ | (CH₂)₂OMe | 0 | Cl | |
| 1-1498 | NO₂ | (CH₂)₃OMe | 0 | Cl | |
| 1-1499 | NO₂ | (CH₂)₂OEt | 0 | Cl | |
| 1-1500 | NO₂ | (CH₂)₂OCH₂-c-Pr | 0 | Cl | |
| 1-1501 | | | | | |
| 1-1502 | NO₂ | CH₂-c-Pr | 1 | Cl | |
| 1-1503 | NO₂ | (CH₂)₂OMe | 1 | Cl | |
| 1-1504 | NO₂ | (CH₂)₃OMe | 1 | Cl | |
| 1-1505 | NO₂ | (CH₂)₂OEt | 1 | Cl | |
| 1-1506 | NO₂ | (CH₂)₂OCH₂-c-Pr | 1 | Cl | |
| 1-1507 | | | | | |
| 1-1508 | NO₂ | CH₂-c-Pr | 2 | Cl | |
| 1-1509 | NO₂ | (CH₂)₂OMe | 2 | Cl | |
| 1-1510 | NO₂ | (CH₂)₃OMe | 2 | Cl | |
| 1-1511 | NO₂ | (CH₂)₂OEt | 2 | Cl | |
| 1-1512 | NO₂ | (CH₂)₂OCH₂-c-Pr | 2 | Cl | |
| 1-1513 | | | | | |
| 1-1514 | SO₂Me | CH₂-c-Pr | 0 | Cl | |
| 1-1515 | SO₂Me | (CH₂)₂OMe | 0 | Cl | |
| 1-1516 | SO₂Me | (CH₂)₃OMe | 0 | Cl | |
| 1-1517 | SO₂Me | (CH₂)₂OEt | 0 | Cl | |
| 1-1518 | SO₂Me | (CH₂)₂OCH₂-c-Pr | 0 | Cl | |
| 1-1519 | | | | | |
| 1-1520 | SO₂Me | CH₂-c-Pr | 1 | Cl | |
| 1-1521 | SO₂Me | (CH₂)₂OMe | 1 | Cl | |
| 1-1522 | SO₂Me | (CH₂)₃OMe | 1 | Cl | |
| 1-1523 | SO₂Me | (CH₂)₂OEt | 1 | Cl | |
| 1-1524 | SO₂Me | (CH₂)₂OCH₂-c-Pr | 1 | Cl | |
| 1-1525 | | | | | |
| 1-1526 | SO₂Me | CH₂-c-Pr | 2 | Cl | |
| 1-1527 | SO₂Me | (CH₂)₂OMe | 2 | Cl | |
| 1-1528 | SO₂Me | (CH₂)₃OMe | 2 | Cl | |
| 1-1529 | SO₂Me | (CH₂)₂OEt | 2 | Cl | |
| 1-1530 | SO₂Me | (CH₂)₂OCH₂-c-Pr | 2 | Cl | |
| 1-1531 | | | | | |
| 1-1532 | CH₂OMe | CH₂-c-Pr | 0 | Cl | |
| 1-1533 | CH₂OMe | (CH₂)₂OMe | 0 | Cl | |
| 1-1534 | CH₂OMe | (CH₂)₃OMe | 0 | Cl | |
| 1-1535 | CH₂OMe | (CH₂)₂OEt | 0 | Cl | |
| 1-1536 | CH₂OMe | (CH₂)₂OCH₂-c-Pr | 0 | Cl | |
| 1-1537 | | | | | |
| 1-1538 | CH₂OMe | CH₂-c-Pr | 1 | Cl | |
| 1-1539 | CH₂OMe | (CH₂)₂OMe | 1 | Cl | |
| 1-1540 | CH₂OMe | (CH₂)₃OMe | 1 | Cl | |
| 1-1541 | CH₂OMe | (CH₂)₂OEt | 1 | Cl | |
| 1-1542 | CH₂OMe | (CH₂)₂OCH₂-c-Pr | 1 | Cl | |
| 1-1543 | | | | | |
| 1-1544 | CH₂OMe | CH₂-c-Pr | 2 | Cl | |
| 1-1545 | CH₂OMe | (CH₂)₂OMe | 2 | Cl | |
| 1-1546 | CH₂OMe | (CH₂)₃OMe | 2 | Cl | |
| 1-1547 | CH₂OMe | (CH₂)₂OEt | 2 | Cl | |
| 1-1548 | CH₂OMe | (CH₂)₂OCH₂-c-Pr | 2 | Cl | |
| 1-1549 | | | | | |
| 1-1550 | CH₂SO₂Me | CH₂-c-Pr | 0 | Cl | |
| 1-1551 | CH₂SO₂Me | (CH₂)₂OMe | 0 | Cl | |
| 1-1552 | CH₂SO₂Me | (CH₂)₃OMe | 0 | Cl | |
| 1-1553 | CH₂SO₂Me | (CH₂)₂OEt | 0 | Cl | |
| 1-1554 | CH₂SO₂Me | (CH₂)₂OCH₂-c-Pr | 0 | Cl | |
| 1-1555 | | | | | |
| 1-1556 | CH₂SO₂Me | CH₂-c-Pr | 1 | Cl | |
| 1-1557 | CH₂SO₂Me | (CH₂)₂OMe | 1 | Cl | |
| 1-1558 | CH₂SO₂Me | (CH₂)₃OMe | 1 | Cl | |
| 1-1559 | CH₂SO₂Me | (CH₂)₂OEt | 1 | Cl | |
| 1-1560 | CH₂SO₂Me | (CH₂)₂OCH₂-c-Pr | 1 | Cl | |
| 1-1561 | | | | | |
| 1-1562 | CH₂SO₂Me | CH₂-c-Pr | 2 | Cl | |
| 1-1563 | CH₂SO₂Me | (CH₂)₂OMe | 2 | Cl | |
| 1-1564 | CH₂SO₂Me | (CH₂)₃OMe | 2 | Cl | |
| 1-1565 | CH₂SO₂Me | (CH₂)₂OEt | 2 | Cl | |
| 1-1566 | CH₂SO₂Me | (CH₂)₂OCH₂-c-Pr | 2 | Cl | |
| 1-1567 | | | | | |
| 1-1568 | CH₂O(CH₂)₂OMe | CH₂-c-Pr | 0 | Cl | |
| 1-1569 | CH₂Oc(CH₂)₂OcMe | (CH₂)₂OMe | 0 | Cl | |
| 1-1570 | CH₂O(CH₂)₂OMe | (CH₂)₃OMe | 0 | Cl | |
| 1-1571 | CH₂O(CH₂)₂OMe | (CH₂)₂OEt | 0 | Cl | |
| 1-1572 | CH₂O(CH₂)₂OMe | (CH₂)₂OCH₂-c-Pr | 0 | Cl | |
| 1-1573 | | | | | |
| 1-1574 | CH₂O(CH₂)₂OMe | CH₂-c-Pr | 1 | Cl | |
| 1-1575 | CH₂O(CH₂)₂OMe | (CH₂)₂OMe | 1 | Cl | |
| 1-1576 | CH₂O(CH₂)₂OMe | (CH₂)₃OMe | 1 | Cl | |
| 1-1577 | CH₂O(CH₂)₂OMe | (CH₂)₂OEt | 1 | Cl | |
| 1-1578 | CH₂O(CH₂)₂OMe | (CH₂)₂OCH₂-c-Pr | 1 | Cl | |
| 1-1579 | | | | | |
| 1-1580 | CH₂O(CH₂)₂OMe | CH₂-c-Pr | 2 | Cl | |
| 1-1581 | CH₂O(CH₂)₂OMe | (CH₂)₂OMe | 2 | Cl | |
| 1-1582 | CH₂O(CH₂)₂OMe | (CH₂)₃OMe | 2 | Cl | |
| 1-1583 | CH₂O(CH₂)₂OMe | (CH₂)₂OEt | 2 | Cl | |
| 1-1584 | CH₂O(CH₂)₂OMe | (CH₂)₂OCH₂-c-Pr | 2 | Cl | |
| 1-1585 | | | | | |
| 1-1586 | CH₂O(CH₂)₂OEt | CH₂-c-Pr | 0 | Cl | |
| 1-1587 | CH₂O(CH₂)₂OEt | (CH₂)₂OMe | 0 | Cl | |
| 1-1588 | CH₂O(CH₂)₂OEt | (CH₂)₃OMe | 0 | Cl | |
| 1-1589 | CH₂O(CH₂)₂OEt | (CH₂)₂OEt | 0 | Cl | |
| 1-1590 | CH₂O(CH₂)₂OEt | (CH₂)₂OCH₂-c-Pr | 0 | Cl | |
| 1-1591 | | | | | |
| 1-1592 | CH₂O(CH₂)₂OEt | CH₂-c-Pr | 1 | Cl | |
| 1-1593 | CH₂O(CH₂)₂OEt | (CH₂)₂OMe | 1 | Cl | |
| 1-1594 | CH₂O(CH₂)₂OEt | (CH₂)₃OMe | 1 | Cl | |
| 1-1595 | CH₂O(CH₂)₂OEt | (CH₂)₂OEt | 1 | Cl | |
| 1-1596 | CH₂O(CH₂)₂OEt | (CH₂)₂OCH₂-c-Pr | 1 | Cl | |
| 1-1597 | | | | | |
| 1-1598 | CH₂O(CH₂)₂OEt | CH₂-c-Pr | 2 | Cl | |
| 1-1599 | CH₂O(CH₂)₂OEt | (CH₂)₂OMe | 2 | Cl | |
| 1-1600 | CH₂O(CH₂)₂OEt | (CH₂)₃OMe | 2 | Cl | |
| 1-1601 | CH₂O(CH₂)₂OEt | (CH₂)₂OEt | 2 | Cl | |
| 1-1602 | CH₂O(CH₂)₂OEt | (CH₂)₂OCH₂-c-Pr | 2 | Cl | |
| 1-1603 | | | | | |
| 1-1604 | CH₂O(CH₂)₃OMe | CH₂-c-Pr | 0 | Cl | |
| 1-1605 | CH₂O(CH₂)₃OMe | (CH₂)₂OMe | 0 | Cl | |
| 1-1606 | CH₂O(CH₂)₃OMe | (CH₂)₃OMe | 0 | Cl | |
| 1-1607 | CH₂O(CH₂)₃OMe | (CH₂)₂OEt | 0 | Cl | |
| 1-1608 | CH₂O(CH₂)₃OMe | (CH₂)₂OCH₂-c-Pr | 0 | Cl | |
| 1-1609 | | | | | |
| 1-1610 | CH₂O(CH₂)₃OMe | CH₂-c-Pr | 1 | Cl | |
| 1-1611 | CH₂O(CH₂)₃OMe | (CH₂)₂OMe | 1 | Cl | |
| 1-1612 | CH₂O(CH₂)₃OMe | (CH₂)₃OMe | 1 | Cl | |
| 1-1613 | CH₂O(CH₂)₃OMe | (CH₂)₂OEt | 1 | Cl | |
| 1-1614 | CH₂O(CH₂)₃OMe | (CH₂)₂OCH₂-c-Pr | 1 | Cl | |
| 1-1615 | | | | | |
| 1-1616 | CH₂O(CH₂)₃OMe | CH₂-c-Pr | 2 | Cl | |
| 1-1617 | CH₂O(CH₂)₃OMe | (CH₂)₂OMe | 2 | Cl | |
| 1-1618 | CH₂O(CH₂)₃OMe | (CH₂)₃OMe | 2 | Cl | |
| 1-1619 | CH₂O(CH₂)₃OMe | (CH₂)₂OEt | 2 | Cl | |
| 1-1620 | CH₂O(CH₂)₃OMe | (CH₂)₂OCH₂-c-Pr | 2 | Cl | |
| 1-1621 | | | | | |
| 1-1622 | CH₂OCH₂OMe | CH₂-c-Pr | 0 | Cl | |
| 1-1623 | CH₂OCH₂OMe | (CH₂)₂OMe | 0 | Cl | |
| 1-1624 | CH₂OCH₂OMe | (CH₂)₃OMe | 0 | Cl | |
| 1-1625 | CH₂OCH₂OMe | (CH₂)₂OEt | 0 | Cl | |
| 1-1626 | CH₂OCH₂OMe | (CH₂)₂OCH₂-c-Pr | 0 | Cl | |
| 1-1627 | | | | | |
| 1-1628 | CH₂OCH₂OMe | CH₂-c-Pr | 1 | Cl | |
| 1-1629 | CH₂OCH₂OMe | (CH₂)₂OMe | 1 | Cl | |
| 1-1630 | CH₂OCH₂OMe | (CH₂)₃OMe | 1 | Cl | |
| 1-1631 | CH₂OCH₂OMe | (CH₂)₂OEt | 1 | Cl | |
| 1-1632 | CH₂OCH₂OMe | (CH₂)₂OCH₂-c-Pr | 1 | Cl | |
| 1-1633 | | | | | |
| 1-1634 | CH₂OCH₂OMe | CH₂-c-Pr | 2 | Cl | |
| 1-1635 | CH₂OCH₂OMe | (CH₂)₂OMe | 2 | Cl | |
| 1-1636 | CH₂OCH₂OMe | (CH₂)₃OMe | 2 | Cl | |
| 1-1637 | CH₂OCH₂OMe | (CH₂)₂OEt | 2 | Cl | |
| 1-1638 | CH₂OCH₂OMe | (CH₂)₂OCH₂-c-Pr | 2 | Cl | |
| 1-1639 | | | | | |
| 1-1640 | CH₂OCH₂OEt | CH₂-c-Pr | 0 | Cl | |
| 1-1641 | CH₂OCH₂OEt | (CH₂)₂OMe | 0 | Cl | |
| 1-1642 | CH₂OCH₂OEt | (CH₂)₃OMe | 0 | Cl | |
| 1-1643 | CH₂OCH₂OEt | (CH₂)₂OEt | 0 | Cl | |
| 1-1644 | CH₂OCH₂OEt | (CH₂)₂OCH₂-c-Pr | 0 | Cl | |
| 1-1645 | | | | | |
| 1-1646 | CH₂OCH₂OEt | CH₂-c-Pr | 1 | Cl | |
| 1-1647 | CH₂OCH₂OEt | (CH₂)₂OMe | 1 | Cl | |
| 1-1648 | CH₂OCH₂OEt | (CH₂)₃OMe | 1 | Cl | |
| 1-1649 | CH₂OCH₂OEt | (CH₂)₂OEt | 1 | Cl | |
| 1-1650 | CH₂OCH₂OEt | (CH₂)₂OCH₂-c-Pr | 1 | Cl | |
| 1-1651 | | | | | |
| 1-1652 | CH₂OCH₂OEt | CH₂-c-Pr | 2 | Cl | |
| 1-1653 | CH₂OCH₂OEt | (CH₂)₂OMe | 2 | Cl | |
| 1-1654 | CH₂OCH₂OEt | (CH₂)₃OMe | 2 | Cl | |
| 1-1655 | CH₂OCH₂OEt | (CH₂)₂OEt | 2 | Cl | |
| 1-1656 | CH₂OCH₂OEt | (CH₂)₂OCH₂-c-Pr | 2 | Cl | |
| 1-1657 | | | | | |
| 1-1658 | CH₂O(CH₂)₂SO₂Me | CH₂-c-Pr | 0 | Cl | |
| 1-1659 | CH₂O(CH₂)₂SO₂Me | (CH₂)₂OMe | 0 | Cl | |
| 1-1660 | CH₂O(CH₂)₂SO₂Me | (CH₂)₃OMe | 0 | Cl | |
| 1-1661 | CH₂O(CH₂)₂SO₂Me | (CH₂)₂OEt | 0 | Cl | |
| 1-1662 | CH₂O(CH₂)₂SO₂Me | (CH₂)₂OCH₂-c-Pr | 0 | Cl | |
| 1-1663 | | | | | |
| 1-1664 | CH₂O(CH₂)₂SO₂Me | CH₂-c-Pr | 1 | Cl | |
| 1-1665 | CH₂O(CH₂)₂SO₂Me | (CH₂)₂OMe | 1 | Cl | |
| 1-1666 | CH₂O(CH₂)₂SO₂Me | (CH₂)₃OMe | 1 | Cl | |
| 1-1667 | CH₂O(CH₂)₂SO₂Me | (CH₂)₂OEt | 1 | Cl | |
| 1-1668 | CH₂O(CH₂)₂SO₂Me | (CH₂)₂OCH₂-c-Pr | 1 | Cl | |
| 1-1669 | | | | | |
| 1-1670 | CH₂O(CH₂)₂SO₂Me | CH₂-c-Pr | 2 | Cl | |
| 1-1671 | CH₂O(CH₂)₂SO₂Me | (CH₂)₂OMe | 2 | Cl | |
| 1-1672 | CH₂O(CH₂)₂SO₂Me | (CH₂)₃OMe | 2 | Cl | |
| 1-1673 | CH₂O(CH₂)₂SO₂Me | (CH₂)₂OEt | 2 | Cl | |
| 1-1674 | CH₂O(CH₂)₂SO₂Me | (CH₂)₂OCH₂-c-Pr | 2 | Cl | |
| 1-1675 | | | | | |
| 1-1676 | CH₂SO₂(CH₂)₂OMe | CH₂-c-Pr | 0 | Cl | |
| 1-1677 | CH₂SO₂(CH₂)₂OMe | (CH₂)₂OMe | 0 | Cl | |
| 1-1678 | CH₂SO₂(CH₂)₂OMe | (CH₂)₃OMe | 0 | Cl | |
| 1-1679 | CH₂SO₂(CH₂)₂OMe | (CH₂)₂OEt | 0 | Cl | |
| 1-1680 | CH₂SO₂(CH₂)₂OMe | (CH₂)₂OCH₂-c-Pr | 0 | Cl | |
| 1-1681 | | | | | |
| 1-1682 | CH₂SO₂(CH₂)₂OMe | CH₂-c-Pr | 1 | Cl | |
| 1-1683 | CH₂SO₂(CH₂)₂OMe | (CH₂)₂OMe | 1 | Cl | |
| 1-1684 | CH₂SO₂(CH₂)₂OMe | (CH₂)₃OMe | 1 | Cl | |
| 1-1685 | CH₂SO₂(CH₂)₂OMe | (CH₂)₂OEt | 1 | Cl | |
| 1-1686 | CH₂SO₂(CH₂)₂OMe | (CH₂)₂OCH₂-c-Pr | 1 | Cl | |
| 1-1687 | | | | | |
| 1-1688 | CH₂SO₂(CH₂)₂OMe | CH₂-c-Pr | 2 | Cl | |
| 1-1689 | CH₂SO₂(CH₂)₂OMe | (CH₂)₂OMe | 2 | Cl | |
| 1-1690 | CH₂SO₂(CH₂)₂OMe | (CH₂)₃OMe | 2 | Cl | |
| 1-1691 | CH₂SO₂(CH₂)₂OMe | (CH₂)₂OEt | 2 | Cl | |
| 1-1692 | CH₂SO₂(CH₂)₂OMe | (CH₂)₂OCH₂-c-Pr | 2 | Cl | |
| 1-1693 | | | | | |
| 1-1694 | CH₂SO₂(CH₂)₂SO₂Me | CH₂-c-Pr | 0 | Cl | |
| 1-1695 | CH₂SO₂(CH₂)₂SO₂Me | (CH₂)₂OMe | 0 | Cl | |
| 1-1696 | CH₂SO₂(CH₂)₂SO₂Me | (CH₂)₃OMe | 0 | Cl | |
| 1-1697 | CH₂SO₂(CH₂)₂SO₂Me | (CH₂)₂OEt | 0 | Cl | |
| 1-1698 | CH₂SO₂(CH₂)₂SO₂Me | (CH₂)₂OCH₂-c-Pr | 0 | Cl | |
| 1-1699 | | | | | |
| 1-1700 | CH₂SO₂(CH₂)₂SO₂Me | CH₂-c-Pr | 1 | Cl | |
| 1-1701 | CH₂SO₂(CH₂)₂SO₂Me | (CH₂)₂OMe | 1 | Cl | |
| 1-1702 | CH₂SO₂(CH₂)₂SO₂Me | (CH₂)₃OMe | 1 | Cl | |
| 1-1703 | CH₂SO₂(CH₂)₂SO₂Me | (CH₂)₂OEt | 1 | Cl | |
| 1-1704 | CH₂SO₂(CH₂)₂SO₂Me | (CH₂)₂OCH₂-c-Pr | 1 | Cl | |
| 1-1705 | | | | | |
| 1-1706 | CH₂SO₂(CH₂)₂SO₂Me | CH₂-c-Pr | 2 | Cl | |
| 1-1707 | CH₂SO₂(CH₂)₂SO₂Me | (CH₂)₂OMe | 2 | Cl | |
| 1-1708 | CH₂SO₂(CH₂)₂SO₂Me | (CH₂)₃OMe | 2 | Cl | |
| 1-1709 | CH₂SO₂(CH₂)₂SO₂Me | (CH₂)₂OEt | 2 | Cl | |
| 1-1710 | CH₂SO₂(CH₂)₂SO₂Me | (CH₂)₂OCH₂-c-Pr | 2 | Cl | |
| 1-1711 | | | | | |
| 1-1712 | Cl | CH₂-c-Pr | 0 | Br | |
| 1-1713 | Cl | (CH₂)₂OMe | 0 | Br | |
| 1-1714 | Cl | (CH₂)₃OMe | 0 | Br | |
| 1-1715 | Cl | (CH₂)₂OEt | 0 | Br | |
| 1-1716 | Cl | (CH₂)₂OCH₂-c-Pr | 0 | Br | |
| 1-1717 | | | | | |
| 1-1718 | Cl | CH₂-c-Pr | 1 | Br | |
| 1-1719 | Cl | (CH₂)₂OMe | 1 | Br | |
| 1-1720 | Cl | (CH₂)₃OMe | 1 | Br | |
| 1-1721 | Cl | (CH₂)₂OEt | 1 | Br | |
| 1-1722 | Cl | (CH₂)₂OCH₂-c-Pr | 1 | Br | |
| 1-1723 | | | | | |
| 1-1724 | Cl | CH₂-c-Pr | 2 | Br | |
| 1-1725 | Cl | (CH₂)₂OMe | 2 | Br | |
| 1-1726 | Cl | (CH₂)₃OMe | 2 | Br | |
| 1-1727 | Cl | (CH₂)₂OEt | 2 | Br | |
| 1-1728 | Cl | (CH₂)₂OCH₂-c-Pr | 2 | Br | |
| 1-1729 | | | | | |
| 1-1730 | Br | CH₂-c-Pr | 0 | Br | |
| 1-1731 | Br | (CH₂)₂OMe | 0 | Br | |
| 1-1732 | Br | (CH₂)₃OMe | 0 | Br | |
| 1-1733 | Br | (CH₂)₂OEt | 0 | Br | |
| 1-1734 | Br | (CH₂)₂OCH₂-c-Pr | 0 | Br | |
| 1-1735 | | | | | |
| 1-1736 | Br | CH₂-c-Pr | 1 | Br | |
| 1-1737 | Br | (CH₂)₂OMe | 1 | Br | |
| 1-1738 | Br | (CH₂)₃OMe | 1 | Br | |
| 1-1739 | Br | (CH₂)₂OEt | 1 | Br | |
| 1-1740 | Br | (CH₂)₂OCH₂-c-Pr | 1 | Br | |
| 1-1741 | | | | | |
| 1-1742 | Br | CH₂-c-Pr | 2 | Br | |
| 1-1743 | Br | (CH₂)₂OMe | 2 | Br | |
| 1-1744 | Br | (CH₂)₃OMe | 2 | Br | |
| 1-1745 | Br | (CH₂)₂OEt | 2 | Br | |
| 1-1746 | Br | (CH₂)₂OCH₂-c-Pr | 2 | Br | |
| 1-1747 | | | | | |
| 1-1748 | Me | CH₂-c-Pr | 0 | Br | |
| 1-1749 | Me | (CH₂)₂OMe | 0 | Br | |
| 1-1750 | Me | (CH₂)₃OMe | 0 | Br | |
| 1-1751 | Me | (CH₂)₂OEt | 0 | Br | |
| 1-1752 | Me | (CH₂)₂OCH₂-c-Pr | 0 | Br | |
| 1-1753 | | | | | |
| 1-1754 | Me | CH₂-c-Pr | 1 | Br | |
| 1-1755 | Me | (CH₂)₂OMe | 1 | Br | |
| 1-1756 | Me | (CH₂)₃OMe | 1 | Br | |
| 1-1757 | Me | (CH₂)₂OEt | 1 | Br | |
| 1-1758 | Me | (CH₂)₂OCH₂-c-Pr | 1 | Br | |
| 1-1759 | | | | | |
| 1-1760 | Me | CH₂-c-Pr | 2 | Br | |
| 1-1761 | Me | (CH₂)₂OMe | 2 | Br | |
| 1-1762 | Me | (CH₂)₃OMe | 2 | Br | |
| 1-1763 | Me | (CH₂)₂OEt | 2 | Br | |
| 1-1764 | Me | (CH₂)₂OCH₂-c-Pr | 2 | Br | |
| 1-1765 | | | | | |
| 1-1766 | Et | CH₂-c-Pr | 0 | Br | |
| 1-1767 | Et | (CH₂)₂OMe | 0 | Br | |
| 1-1768 | Et | (CH₂)₃OMe | 0 | Br | |
| 1-1769 | Et | (CH₂)₂OEt | 0 | Br | |
| 1-1770 | Et | (CH₂)₂OCH₂-c-Pr | 0 | Br | |
| 1-1771 | | | | | |
| 1-1772 | Et | CH₂-c-Pr | 1 | Br | |
| 1-1773 | Et | (CH₂)₂OMe | 1 | Br | |
| 1-1774 | Et | (CH₂)₃OMe | 1 | Br | |
| 1-1775 | Et | (CH₂)₂OEt | 1 | Br | |
| 1-1776 | Et | (CH₂)₂OCH₂-c-Pr | 1 | Br | |
| 1-1777 | | | | | |
| 1-1778 | Et | CH₂-c-Pr | 2 | Br | |
| 1-1779 | Et | (CH₂)₂OMe | 2 | Br | |
| 1-1780 | Et | (CH₂)₃OMe | 2 | Br | |
| 1-1781 | Et | (CH₂)₂OEt | 2 | Br | |
| 1-1782 | Et | (CH₂)₂OCH₂-c-Pr | 2 | Br | |
| 1-1783 | | | | | |
| 1-1784 | CF₃ | CH₂-c-Pr | 0 | Br | |
| 1-1785 | CF₃ | (CH₂)₂OMe | 0 | Br | |
| 1-1786 | CF₃ | (CH₂)₃OMe | 0 | Br | |
| 1-1787 | CF₃ | (CH₂)₂OEt | 0 | Br | |
| 1-1788 | CF₃ | (CH₂)₂OCH₂-c-Pr | 0 | Br | |
| 1-1789 | | | | | |
| 1-1790 | CF₃ | CH₂-c-Pr | 1 | Br | |
| 1-1791 | CF₃ | (CH₂)₂OMe | 1 | Br | |
| 1-1792 | CF₃ | (CH₂)₃OMe | 1 | Br | |
| 1-1793 | CF₃ | (CH₂)₂OEt | 1 | Br | |
| 1-1794 | CF₃ | (CH₂)₂OCH₂-c-Pr | 1 | Br | |
| 1-1795 | | | | | |
| 1-1796 | CF₃ | CH₂-c-Pr | 2 | Br | |
| 1-1797 | CF₃ | (CH₂)₂OMe | 2 | Br | |
| 1-1798 | CF₃ | (CH₂)₃OMe | 2 | Br | |
| 1-1799 | CF₃ | (CH₂)₂OEt | 2 | Br | |
| 1-1800 | CF₃ | (CH₂)₂OCH₂-c-Pr | 2 | Br | |
| 1-1801 | | | | | |
| 1-1802 | OMe | CH₂-c-Pr | 0 | Br | |
| 1-1803 | OMe | (CH₂)₂OMe | 0 | Br | |
| 1-1804 | OMe | (CH₂)₃OMe | 0 | Br | |
| 1-1805 | OMe | (CH₂)₂OEt | 0 | Br | |
| 1-1806 | OMe | (CH₂)₂OCH₂-c-Pr | 0 | Br | |
| 1-1807 | | | | | |
| 1-1808 | OMe | CH₂-c-Pr | 1 | Br | |
| 1-1809 | OMe | (CH₂)₂OMe | 1 | Br | |
| 1-1810 | OMe | (CH₂)₃OMe | 1 | Br | |
| 1-1811 | OMe | (CH₂)₂OEt | 1 | Br | |
| 1-1812 | OMe | (CH₂)₂OCH₂-c-Pr | 1 | Br | |
| 1-1813 | | | | | |
| 1-1814 | OMe | CH₂-c-Pr | 2 | Br | |
| 1-1815 | OMe | (CH₂)₂OMe | 2 | Br | |
| 1-1816 | OMe | (CH₂)₃OMe | 2 | Br | |
| 1-1817 | OMe | (CH₂)₂OEt | 2 | Br | |
| 1-1818 | OMe | (CH₂)₂OCH₂-c-Pr | 2 | Br | |
| 1-1819 | | | | | |
| 1-1820 | OEt | CH₂-c-Pr | 0 | Br | |
| 1-1821 | OEt | (CH₂)₂OMe | 0 | Br | |
| 1-1822 | OEt | (CH₂)₃OMe | 0 | Br | |
| 1-1823 | OEt | (CH₂)₂OEt | 0 | Br | |
| 1-1824 | OEt | (CH₂)₂OCH₂-c-Pr | 0 | Br | |
| 1-1825 | | | | | |
| 1-1826 | OEt | CH₂-c-Pr | 1 | Br | |
| 1-1827 | OEt | (CH₂)₂OMe | 1 | Br | |
| 1-1828 | OEt | (CH₂)₃OMe | 1 | Br | |
| 1-1829 | OEt | (CH₂)₂OEt | 1 | Br | |
| 1-1830 | OEt | (CH₂)₂OCH₂-c-Pr | 1 | Br | |
| 1-1831 | | | | | |
| 1-1832 | OEt | CH₂-c-Pr | 2 | Br | |
| 1-1833 | OEt | (CH₂)₂OMe | 2 | Br | |
| 1-1834 | OEt | (CH₂)₃OMe | 2 | Br | |
| 1-1835 | OEt | (CH₂)₂OEt | 2 | Br | |
| 1-1836 | OEt | (CH₂)₂OCH₂-c-Pr | 2 | Br | |
| 1-1837 | | | | | |
| 1-1838 | NO₂ | CH₂-c-Pr | 0 | Br | |
| 1-1839 | NO₂ | (CH₂)₂OMe | 0 | Br | |
| 1-1840 | NO₂ | (CH₂)₃OMe | 0 | Br | |
| 1-1841 | NO₂ | (CH₂)₂OEt | 0 | Br | |
| 1-1842 | NO₂ | (CH₂)₂OCH₂-c-Pr | 0 | Br | |
| 1-1843 | | | | | |
| 1-1844 | NO₂ | CH₂-c-Pr | 1 | Br | |
| 1-1845 | NO₂ | (CH₂)₂OMe | 1 | Br | |
| 1-1846 | NO₂ | (CH₂)₃OMe | 1 | Br | |
| 1-1847 | NO₂ | (CH₂)₂OEt | 1 | Br | |
| 1-1848 | NO₂ | (CH₂)₂OCH₂-c-Pr | 1 | Br | |
| 1-1849 | | | | | |
| 1-1850 | NO₂ | CH₂-c-Pr | 2 | Br | |
| 1-1851 | NO₂ | (CH₂)₂OMe | 2 | Br | |
| 1-1852 | NO₂ | (CH₂)₃OMe | 2 | Br | |
| 1-1853 | NO₂ | (CH₂)₂OEt | 2 | Br | |
| 1-1854 | NO₂ | (CH₂)₂OCH₂-c-Pr | 2 | Br | |
| 1-1855 | | | | | |
| 1-1856 | SO₂Me | CH₂-c-Pr | 0 | Br | |
| 1-1857 | SO₂Me | (CH₂)₂OMe | 0 | Br | |
| 1-1858 | SO₂Me | (CH₂)₃OMe | 0 | Br | |
| 1-1859 | SO₂Me | (CH₂)₂OEt | 0 | Br | |
| 1-1860 | SO₂Me | (CH₂)₂OCH₂-c-Pr | 0 | Br | |
| 1-1861 | | | | | |
| 1-1862 | SO₂Me | CH₂-c-Pr | 1 | Br | |
| 1-1863 | SO₂Me | (CH₂)₂OMe | 1 | Br | |
| 1-1864 | SO₂Me | (CH₂)₃OMe | 1 | Br | |
| 1-1865 | SO₂Me | (CH₂)₂OEt | 1 | Br | |
| 1-1866 | SO₂Me | (CH₂)₂OCH₂-c-Pr | 1 | Br | |
| 1-1867 | | | | | |
| 1-1868 | SO₂Me | CH₂-c-Pr | 2 | Br | |
| 1-1869 | SO₂Me | (CH₂)₂OMe | 2 | Br | |
| 1-1870 | SO₂Me | (CH₂)₃OMe | 2 | Br | |
| 1-1871 | SO₂Me | (CH₂)₂OEt | 2 | Br | |
| 1-1872 | SO₂Me | (CH₂)₂OCH₂-c-Pr | 2 | Br | |
| 1-1873 | | | | | |
| 1-1874 | CH₂OMe | CH₂-c-Pr | 0 | Br | |
| 1-1875 | CH₂OMe | (CH₂)₂OMe | 0 | Br | |
| 1-1876 | CH₂OMe | (CH₂)₃OMe | 0 | Br | |
| 1-1877 | CH₂OMe | (CH₂)₂OEt | 0 | Br | |
| 1-1878 | CH₂OMe | (CH₂)₂OCH₂-c-Pr | 0 | Br | |
| 1-1879 | | | | | |
| 1-1880 | CH₂OMe | CH₂-c-Pr | 1 | Br | |
| 1-1881 | CH₂OMe | (CH₂)₂OMe | 1 | Br | |
| 1-1882 | CH₂OMe | (CH₂)₃OMe | 1 | Br | |
| 1-1883 | CH₂OMe | (CH₂)₂OEt | 1 | Br | |
| 1-1884 | CH₂OMe | (CH₂)₂OCH₂-c-Pr | 1 | Br | |
| 1-1885 | | | | | |
| 1-1886 | CH₂OMe | CH₂-c-Pr | 2 | Br | |
| 1-1887 | CH₂OMe | (CH₂)₂OMe | 2 | Br | |
| 1-1888 | CH₂OMe | (CH₂)₃OMe | 2 | Br | |
| 1-1889 | CH₂OMe | (CH₂)₂OEt | 2 | Br | |
| 1-1890 | CH₂OMe | (CH₂)₂OCH₂-c-Pr | 2 | Br | |
| 1-1891 | | | | | |
| 1-1892 | CH₂SO₂Me | CH₂-c-Pr | 0 | Br | |
| 1-1893 | CH₂SO₂Me | (CH₂)₂OMe | 0 | Br | |
| 1-1894 | CH₂SO₂Me | (CH₂)₃OMe | 0 | Br | |
| 1-1895 | CH₂SO₂Me | (CH₂)₂OEt | 0 | Br | |
| 1-1896 | CH₂SO₂Me | (CH₂)₂OCH₂-c-Pr | 0 | Br | |
| 1-1897 | | | | | |
| 1-1898 | CH₂SO₂Me | CH₂-c-Pr | 1 | Br | |
| 1-1899 | CH₂SO₂Me | (CH₂)₂OMe | 1 | Br | |
| 1-1900 | CH₂SO₂Me | (CH₂)₃OMe | 1 | Br | |
| 1-1901 | CH₂SO₂Me | (CH₂)₂OEt | 1 | Br | |
| 1-1902 | CH₂SO₂Me | (CH₂)₂OCH₂-c-Pr | 1 | Br | |
| 1-1903 | | | | | |
| 1-1904 | CH₂SO₂Me | CH₂-c-Pr | 2 | Br | |
| 1-1905 | CH₂SO₂Me | (CH₂)₂OMe | 2 | Br | |
| 1-1906 | CH₂SO₂Me | (CH₂)₃OMe | 2 | Br | |
| 1-1907 | CH₂SO₂Me | (CH₂)₂OEt | 2 | Br | |
| 1-1908 | CH₂SO₂Me | (CH₂)₂OCH₂-c-Pr | 2 | Br | |
| 1-1909 | | | | | |
| 1-1910 | CH₂O(CH₂)₂OMe | CH₂-c-Pr | 0 | Br | |
| 1-1911 | CH₂O(CH₂)₂OMe | (CH₂)₂OMe | 0 | Br | |
| 1-1912 | CH₂O(CH₂)₂OMe | (CH₂)₃OMe | 0 | Br | |
| 1-1913 | CH₂O(CH₂)₂OMe | (CH₂)₂OEt | 0 | Br | |
| 1-1914 | CH₂O(CH₂)₂OMe | (CH₂)₂OCH₂-c-Pr | 0 | Br | |
| 1-1915 | | | | | |
| 1-1916 | CH₂O(CH₂)₂OMe | CH₂-c-Pr | 1 | Br | |
| 1-1917 | CH₂O(CH₂)₂OMe | (CH₂)₂OMe | 1 | Br | |
| 1-1918 | CH₂O(CH₂)₂OMe | (CH₂)₃OMe | 1 | Br | |
| 1-1919 | CH₂O(CH₂)₂OMe | (CH₂)₂OEt | 1 | Br | |
| 1-1920 | CH₂O(CH₂)₂OMe | (CH₂)₂OCH₂-c-Pr | 1 | Br | |
| 1-1921 | | | | | |
| 1-1922 | CH₂O(CH₂)₂OMe | CH₂-c-Pr | 2 | Br | |
| 1-1923 | CH₂O(CH₂)₂OMe | (CH₂)₂OMe | 2 | Br | |
| 1-1924 | CH₂O(CH₂)₂OMe | (CH₂)₃OMe | 2 | Br | |
| 1-1925 | CH₂O(CH₂)₂OMe | (CH₂)₂OEt | 2 | Br | |
| 1-1926 | CH₂O(CH₂)₂OMe | (CH₂)₂OCH₂-c-Pr | 2 | Br | |
| 1-1927 | | | | | |
| 1-1928 | CH₂O(CH₂)₂OEt | CH₂-c-Pr | 0 | Br | |
| 1-1929 | CH₂O(CH₂)₂OEt | (CH₂)₂OMe | 0 | Br | |
| 1-1930 | CH₂O(CH₂)₂OEt | (CH₂)₃OMe | 0 | Br | |
| 1-1931 | CH₂O(CH₂)₂OEt | (CH₂)₂OEt | 0 | Br | |
| 1-1932 | CH₂O(CH₂)₂OEt | (CH₂)₂OCH₂-c-Pr | 0 | Br | |
| 1-1933 | | | | | |
| 1-1934 | CH₂O(CH₂)₂OEt | CH₂-c-Pr | 1 | Br | |
| 1-1935 | CH₂O(CH₂)₂OEt | (CH₂)₂OMe | 1 | Br | |
| 1-1936 | CH₂O(CH₂)₂OEt | (CH₂)₃OMe | 1 | Br | |
| 1-1937 | CH₂O(CH₂)₂OEt | (CH₂)₂OEt | 1 | Br | |
| 1-1938 | CH₂O(CH₂)₂OEt | (CH₂)₂OCH₂-c-Pr | 1 | Br | |
| 1-1939 | | | | | |
| 1-1940 | CH₂O(CH₂)₂OEt | CH₂-c-Pr | 2 | Br | |
| 1-1941 | CH₂O(CH₂)₂OEt | (CH₂)₂OMe | 2 | Br | |
| 1-1942 | CH₂O(CH₂)₂OEt | (CH₂)₃OMe | 2 | Br | |
| 1-1943 | CH₂O(CH₂)₂OEt | (CH₂)₂OEt | 2 | Br | |
| 1-1944 | CH₂O(CH₂)₂OEt | (CH₂)₂OCH₂-c-Pr | 2 | Br | |
| 1-1945 | | | | | |
| 1-1946 | CH₂O(CH₂)₃OMe | CH₂-c-Pr | 0 | Br | |
| 1-1947 | CH₂O(CH₂)₃OMe | (CH₂)₂OMe | 0 | Br | |
| 1-1948 | CH₂O(CH₂)₃OMe | (CH₂)₃OMe | 0 | Br | |
| 1-1949 | CH₂O(CH₂)₃OMe | (CH₂)₂OEt | 0 | Br | |
| 1-1950 | CH₂O(CH₂)₃OMe | (CH₂)₂OCH₂-c-Pr | 0 | Br | |
| 1-1951 | | | | | |
| 1-1952 | CH₂O(CH₂)₃OMe | CH₂-c-Pr | 1 | Br | |
| 1-1953 | CH₂O(CH₂)₂OMe | (CH₂)₂OMe | 1 | Br | |
| 1-1954 | CH₂O(CH₂)₃OMe | (CH₂)₃OMe | 1 | Br | |
| 1-1955 | CH₂O(CH₂)₃OMe | (CH₂)₂OEt | 1 | Br | |
| 1-1956 | CH₂O(CH₂)₃OMe | (CH₂)₂OCH₂-c-Pr | 1 | Br | |
| 1-1957 | | | | | |
| 1-1958 | CH₂O(CH₂)₃OMe | CH₂-c-Pr | 2 | Br | |
| 1-1959 | CH₂O(CH₂)₃OMe | (CH₂)₂OMe | 2 | Br | |
| 1-1960 | CH₂O(CH₂)₃OMe | (CH₂)₃OMe | 2 | Br | |
| 1-1961 | CH₂O(CH₂)₃OMe | (CH₂)₂OEt | 2 | Br | |
| 1-1962 | CH₂O(CH₂)₃OMe | (CH₂)₂OCH₂-c-Pr | 2 | Br | |
| 1-1963 | | | | | |
| 1-1964 | CH₂OCH₂OMe | CH₂-c-Pr | 0 | Br | |
| 1-1965 | CH₂OCH₂OMe | (CH₂)₂OMe | 0 | Br | |
| 1-1966 | CH₂OCH₂OMe | (CH₂)₃OMe | 0 | Br | |
| 1-1967 | CH₂OCH₂OMe | (CH₂)₂OEt | 0 | Br | |
| 1-1968 | CH₂OCH₂OMe | (CH₂)₂OCH₂-c-Pr | 0 | Br | |
| 1-1969 | | | | | |
| 1-1970 | CH₂OCH₂OMe | CH₂-c-Pr | 1 | Br | |
| 1-1971 | CH₂OCH₂OMe | (CH₂)₂OMe | 1 | Br | |
| 1-1972 | CH₂OCH₂OMe | (CH₂)₃OMe | 1 | Br | |
| 1-1973 | CH₂OCH₂OMe | (CH₂)₂OEt | 1 | Br | |
| 1-1974 | CH₂OCH₂OMe | (CH₂)₂OCH₂-c-Pr | 1 | Br | |
| 1-1975 | | | | | |
| 1-1976 | CH₂OCH₂OMe | CH₂-c-Pr | 2 | Br | |
| 1-1977 | CH₂OCH₂OMe | (CH₂)₂OMe | 2 | Br | |
| 1-1978 | CH₂OCH₂OMe | (CH₂)₃OMe | 2 | Br | |
| 1-1979 | CH₂OCH₂OMe | (CH₂)₂OEt | 2 | Br | |
| 1-1980 | CH₂OCH₂OMe | (CH₂)₂OCH₂-c-Pr | 2 | Br | |
| 1-1981 | | | | | |
| 1-1982 | CH₂OCH₂OEt | CH₂-c-Pr | 0 | Br | |
| 1-1983 | CH₂OCH₂OEt | (CH₂)₂OMe | 0 | Br | |
| 1-1984 | CH₂OCH₂OEt | (CH₂)₃OMe | 0 | Br | |
| 1-1985 | CH₂OCH₂OEt | (CH₂)₂OEt | 0 | Br | |
| 1-1986 | CH₂OCH₂OEt | (CH₂)₂OCH₂-c-Pr | 0 | Br | |
| 1-1987 | | | | | |
| 1-1988 | CH₂OCH₂OEt | CH₂-c-Pr | 1 | Br | |
| 1-1989 | CH₂OCH₂OEt | (CH₂)₂OMe | 1 | Br | |
| 1-1990 | CH₂OCH₂OEt | (CH₂)₃OMe | 1 | Br | |
| 1-1991 | CH₂OCH₂OEt | (CH₂)₂OEt | 1 | Br | |
| 1-1992 | CH₂OCH₂OEt | (CH₂)₂OCH₂-c-Pr | 1 | Br | |
| 1-1993 | | | | | |
| 1-1994 | CH₂OCH₂OEt | CH₂-c-Pr | 2 | Br | |
| 1-1995 | CH₂OCH₂OEt | (CH₂)₂OMe | 2 | Br | |
| 1-1996 | CH₂OCH₂OEt | (CH₂)₃OMe | 2 | Br | |
| 1-1997 | CH₂OCH₂OEt | (CH₂)₂OEt | 2 | Br | |
| 1-1998 | CH₂OCH₂OEt | (CH₂)₂OCH₂-c-Pr | 2 | Br | |
| 1-1999 | | | | | |
| 1-2000 | CH₂O(CH₂)₂SO₂Me | CH₂-c-Pr | 0 | Br | |
| 1-2001 | CH₂O(CH₂)₂SO₂Me | (CH₂)₂OMe | 0 | Br | |
| 1-2002 | CH₂O(CH₂)₂SO₂Me | (CH₂)₃OMe | 0 | Br | |
| 1-2003 | CH₂O(CH₂)₂SO₂Me | (CH₂)₂OEt | 0 | Br | |
| 1-2004 | CH₂0(CH₂)₂SO₂Me | (CH₂)₂OCH₂-c-Pr | 0 | Br | |
| 1-2005 | | | | | |
| 1-2006 | CH₂O(CH₂)₂SO₂Me | CH₂-c-Pr | 1 | Br | |
| 1-2007 | CH₂O(CH₂)₂SO₂Me | (CH₂)₂OMe | 1 | Br | |
| 1-2008 | CH₂O(CH₂)₂SO₂Me | (CH₂)₃OMe | 1 | Br | |
| 1-2009 | CH₂O(CH₂)₂SO₂Me | (CH₂)₂OEt | 1 | Br | |
| 1-2010 | CH₂O(CH₂)₂SO₂Me | (CH₂)₂OCH₂-c-Pr | 1 | Br | |
| 1-2011 | | | | | |
| 1-2012 | CH₂O(CH₂)₂SO₂Me | CH₂-c-Pr | 2 | Br | |
| 1-2013 | CH₂O(CH₂)₂SO₂Me | (CH₂)₂OMe | 2 | Br | |
| 1-2014 | CH₂O(CH₂)₂SO₂Me | (CH₂)₃OMe | 2 | Br | |
| 1-2015 | CH₂O(CH₂)₂SO₂Me | (CH₂)₂OEt | 2 | Br | |
| 1-2016 | CH₂O(CH₂)₂SO₂Me | (CH₂)₂OCH₂-c-Pr | 2 | Br | |
| 1-2017 | | | | | |
| 1-2018 | CH₂SO₂(CH₂)₂OMe | CH₂-c-Pr | 0 | Br | |
| 1-2019 | CH₂SO₂(CH₂)₂OMe | (CH₂)₂OMe | 0 | Br | |
| 1-2020 | CH₂SO₂(CH₂)₂OMe | (CH₂)₃OMe | 0 | Br | |
| 1-2021 | CH₂SO₂(CH₂)₂OMe | (CH₂)₂OEt | 0 | Br | |
| 1-2022 | CH₂SO₂(CH₂)₂OMe | (CH₂)₂OCH₂-c-Pr | 0 | Br | |
| 1-2023 | | | | | |
| 1-2024 | CH₂SO₂(CH₂)₂OMe | CH₂-c-Pr | 1 | Br | |
| 1-2025 | CH₂SO₂(CH₂)₂OMe | (CH₂)₂OMe | 1 | Br | |
| 1-2026 | CH₂SO₂(CH₂)₂OMe | (CH₂)₃OMe | 1 | Br | |
| 1-2027 | CH₂SO₂(CH₂)₂OMe | (CH₂)₂OEt | 1 | Br | |
| 1-2028 | CH₂SO₂(CH₂)₂OMe | (CH₂)₂OCH₂-c-Pr | 1 | Br | |
| 1-2029 | | | | | |
| 1-2030 | CH₂SO₂(CH₂)₂OMe | CH₂-c-Pr | 2 | Br | |
| 1-2031 | CH₂SO₂(CH₂)₂OMe | (CH₂)₂OMe | 2 | Br | |
| 1-2032 | CH₂SO₂(CH₂)₂OMe | (CH₂)₃OMe | 2 | Br | |
| 1-2033 | CH₂SO₂(CH₂)₂OMe | (CH₂)₂OEt | 2 | Br | |
| 1-2034 | CH₂SO₂(CH₂)₂OMe | (CH₂)₂OCH₂-c-Pr | 2 | Br | |
| 1-2035 | | | | | |
| 1-2036 | CH₂SO₂(CH₂)₂SO₂Me | CH₂-c-Pr | 0 | Br | |
| 1-2037 | CH₂SO₂(CH₂)₂SO₂Me | (CH₂)₂OMe | 0 | Br | |
| 1-2038 | CH₂SO₂(CH₂)₂SO₂Me | (CH₂)₃OMe | 0 | Br | |
| 1-2039 | CH₂SO₂(CH₂)₂SO₂Me | (CH₂)₂OEt | 0 | Br | |
| 1-2040 | CH₂SO₂(CH₂)₂SO₂Me | (CH₂)₂OCH₂-c-Pr | 0 | Br | |
| 1-2041 | | | | | |
| 1-2042 | CH₂SO₂(CH₂)₂SO₂Me | CH₂-c-Pr | 1 | Br | |
| 1-2043 | CH₂SO₂(CH₂)₂SO₂Me | (CH₂)₂OMe | 1 | Br | |
| 1-2044 | CH₂SO₂(CH₂)₂SO₂Me | (CH₂)₃OMe | 1 | Br | |
| 1-2045 | CH₂SO₂(CH₂)₂SO₂Me | (CH₂)₂OEt | 1 | Br | |
| 1-2046 | CH₂SO₂(CH₂)₂SO₂Me | (CH₂)₂OCH₂-c-Pr | 1 | Br | |
| 1-2047 | | | | | |
| 1-2048 | CH₂SO₂(CH₂)₂SO₂Me | CH₂-c-Pr | 2 | Br | |
| 1-2049 | CH₂SO₂(CH₂)₂SO₂Me | (CH₂)₂OMe | 2 | Br | |
| 1-2050 | CH₂SO₂(CH₂)₂SO₂Me | (CH₂)₃OMe | 2 | Br | |
| 1-2051 | CH₂SO₂(CH₂)₂SO₂Me | (CH₂)₂OEt | 2 | Br | |
| 1-2052 | CH₂SO₂(CH₂)₂SO₂Me | (CH₂)₂OCH₂-c-Pr | 2 | Br | |
| 1-2053 | | | | | |
| 1-2054 | Cl | CH₂-c-Pr | 0 | CF₃ | |
| 1-2055 | Cl | (CH₂)₂OMe | 0 | CF₃ | |
| 1-2056 | Cl | (CH₂)₃OMe | 0 | CF₃ | |
| 1-2057 | Cl | (CH₂)₂OEt | 0 | CF₃ | |
| 1-2058 | Cl | (CH₂)₂OCH₂-c-Pr | 0 | CF₃ | |
| 1-2059 | | | | | |
| 1-2060 | Cl | CH₂-c-Pr | 1 | CF₃ | |
| 1-2061 | Cl | (CH₂)₂OMe | 1 | CF₃ | |
| 1-2062 | Cl | (CH₂)₃OMe | 1 | CF₃ | |
| 1-2063 | Cl | (CH₂)₂OEt | 1 | CF₃ | |
| 1-2064 | Cl | (CH₂)₂OCH₂-c-Pr | 1 | CF₃ | |
| 1-2065 | | | | | |
| 1-2066 | Cl | CH₂-c-Pr | 2 | CF₃ | |
| 1-2067 | Cl | (CH₂)₂OMe | 2 | CF₃ | |
| 1-2068 | Cl | (CH₂)₃OMe | 2 | CF₃ | |
| 1-2069 | Cl | (CH₂)₂OEt | 2 | CF₃ | |
| 1-2070 | Cl | (CH₂)₂OCH₂-c-Pr | 2 | CF₃ | |
| 1-2071 | | | | | |
| 1-2072 | Br | CH₂-c-Pr | 0 | CF₃ | |
| 1-2073 | Br | (CH₂)₂OMe | 0 | CF₃ | |
| 1-2074 | Br | (CH₂)₃OMe | 0 | CF₃ | |
| 1-2075 | Br | (CH₂)₂OEt | 0 | CF₃ | |
| 1-2076 | Br | (CH₂)₂OCH₂-c-Pr | 0 | CF₃ | |
| 1-2077 | | | | | |
| 1-2078 | Br | CH₂-c-Pr | 1 | CF₃ | |
| 1-2079 | Br | (CH₂)₂OMe | 1 | CF₃ | |
| 1-2080 | Br | (CH₂)₃OMe | 1 | CF₃ | |
| 1-2081 | Br | (CH₂)₂OEt | 1 | CF₃ | |
| 1-2082 | Br | (CH₂)₂OCH₂-c-Pr | 1 | CF₃ | |
| 1-2083 | | | | | |
| 1-2084 | Br | CH₂-c-Pr | 2 | CF₃ | |
| 1-2085 | Br | (CH₂)₂OMe | 2 | CF₃ | |
| 1-2086 | Br | (CH₂)₃OMe | 2 | CF₃ | |
| 1-2087 | Br | (CH₂)₂OEt | 2 | CF₃ | |
| 1-2088 | Br | (CH₂)₂OCH₂-c-Pr | 2 | CF₃ | |
| 1-2089 | | | | | |
| 1-2090 | Me | CH₂-c-Pr | 0 | CF₃ | |
| 1-2091 | Me | (CH₂)₂OMe | 0 | CF₃ | 7.68 (d, 1H), 7.39 (d, 1H), 3.61 (s, 3H), 3.52 (t, 2H), 3.32 (s, 3H), 2.91 (t, 2H), 2.64 (s, 3H), 0.90 - 0.83 (m, 1H), 0.76 (m, 2H), 0.47 (m, 2H) |
| 1-2092 | Me | (CH₂)₃OMe | 0 | CF₃ | |
| 1-2093 | Me | (CH₂)₂OEt | 0 | CF₃ | |
| 1-2094 | Me | (CH₂)₂OCH₂-c-Pr | 0 | CF₃ | |
| 1-2095 | | | | | |
| 1-2096 | Me | CH₂-c-Pr | 1 | CF₃ | |
| 1-2097 | Me | (CH₂)₂OMe | 1 | CF₃ | 7.72 (d, 1H), 7.51 (d, 1H), 3.95 (dt, 1H), 3.83 (dt, 1H), 3.62 - 3.58 (m, 1H), 3.61 (s, 3H), 3.41 (s, 3H), 3.13 (m, 1H), 2.82 (s, 3H), 0.91 - 0.72 (m, 3H), 0.58 (m, 1H), 0.46 (m, 1H) |
| 1-2098 | Me | (CH₂)₃OMe | 1' | CF₃ | |
| 1-2099 | Me | (CH₂)₂OEt | 1 | CF₃ | |
| 1-2100 | Me | (CH₂)₂OCH₂-c-Pr | 1 | CF₃ | |
| 1-2101 | | | | | |
| 1-2102 | Me | CH₂-c-Pr | 2 | CF₃ | |
| 1-2103 | Me | (CH₂)₂OMe | 2 | CF₃ | |
| 1-2104 | Me | (CH₂)₃OMe | 2 | CF₃ | |
| 1-2105 | Me | (CH₂)₂OEt | 2 | CF₃ | |
| 1-2106 | Me | (CH₂)₂OCH₂-c-Pr | 2 | CF₃ | |
| 1-2107 | | | | | |
| 1-2108 | Et | CH₂-c-Pr | 0 | CF₃ | |
| 1-2109 | Et | (CH₂)₂OMe | 0 | CF₃ | |
| 1-2110 | Et | (CH₂)₃OMe | 0 | CF₃ | |
| 1-2111 | Et | (CH₂)₂OEt | 0 | CF₃ | |
| 1-2112 | Et | (CH₂)₂OCH₂-c-Pr | 0 | CF₃ | |
| 1-2113 | | | | | |
| 1-2114 | Et | CH₂-c-Pr | 1 | CF₃ | |
| 1-2115 | Et | (CH₂)₂OMe | 1 | CF₃ | |
| 1-2116 | Et | (CH₂)₃OMe | 1 | CF₃ | |
| 1-2117 | Et | (CH₂)₂OEt | 1 | CF₃ | |
| 1-2118 | Et | (CH₂)₂OCH₂-c-Pr | 1 | CF₃ | |
| 1-2119 | | | | | |
| 1-2120 | Et | CH₂-c-Pr | 2 | CF₃ | |
| 1-2121 | Et | (CH₂)₂OMe | 2 | CF₃ | |
| 1-2122 | Et | (CH₂)₃OMe | 2 | CF₃ | |
| 1-2123 | Et | (CH₂)₂OEt | 2 | CF₃ | |
| 1-2124 | Et | (CH₂)₂OCH₂-c-Pr | 2 | CF₃ | |
| 1-2125 | | | | | |
| 1-2126 | CF₃ | CH₂-c-Pr | 0 | CF₃ | |
| 1-2127 | CF₃ | (CH₂)₂OMe | 0 | CF₃ | |
| 1-2128 | CF₃ | (CH₂)₃OMe | 0 | CF₃ | |
| 1-2129 | CF₃ | (CH₂)₂OEt | 0 | CF₃ | |
| 1-2130 | CF₃ | (CH₂)₂OCH₂-c-Pr | 0 | CF₃ | |
| 1-2131 | | | | | |
| 1-2132 | CF₃ | CH₂-c-Pr | 1 | CF₃ | |
| 1-2133 | CF₃ | (CH₂)₂OMe | 1 | CF₃ | |
| 1-2134 | CF₃ | (CH₂)₃OMe | 1 | CF₃ | |
| 1-2135 | CF₃ | (CH₂)₂OEt | 1 | CF₃ | |
| 1-2136 | CF₃ | (CH₂)₂OCH₂-c-Pr | 1 | CF₃ | |
| 1-2137 | | | | | |
| 1-2138 | CF₃ | CH₂-c-Pr | 2 | CF₃ | |
| 1-2139 | CF₃ | (CH₂)₂OMe | 2 | CF₃ | |
| 1-2140 | CF₃ | (CH₂)₃OMe | 2 | CF₃ | |
| 1-2141 | CF₃ | (CH₂)₂OEt | 2 | CF₃ | |
| 1-2142 | CF₃ | (CH₂)₂OCH₂-c-Pr | 2 | CF₃ | |
| 1-2143 | | | | | |
| 1-2144 | OMe | CH₂-c-Pr | 0 | CF₃ | |
| 1-2145 | OMe | (CH₂)₂OMe | 0 | CF₃ | |
| 1-2146 | OMe | (CH₂)₃OMe | 0 | CF₃ | |
| 1-2147 | OMe | (CH₂)₂OEt | 0 | CF₃ | |
| 1-2148 | OMe | (CH₂)₂OCH₂-c-Pr | 0 | CF₃ | |
| 1-2149 | | | | | |
| 1-2150 | OMe | CH₂-c-Pr | 1 | CF₃ | |
| 1-2151 | OMe | (CH₂)₂OMe | 1 | CF₃ | |
| 1-2152 | OMe | (CH₂)₃OMe | 1 | CF₃ | |
| 1-2153 | OMe | (CH₂)₂OEt | 1 | CF₃ | |
| 1-2154 | OMe | (CH₂)₂OCH₂-c-Pr | 1 | CF₃ | |
| 1-2155 | | | | | |
| 1-2156 | OMe | CH₂-c-Pr | 2 | CF₃ | |
| 1-2157 | OMe | (CH₂)₂OMe | 2 | CF₃ | |
| 1-2158 | OMe | (CH₂)₃OMe | 2 | CF₃ | |
| 1-2159 | OMe | (CH₂)₂OEt | 2 | CF₃ | |
| 1-2160 | OMe | (CH₂)₂OCH₂-c-Pr | 2 | CF₃ | |
| 1-2161 | | | | | |
| 1-2162 | OEt | CH₂-c-Pr | 0 | CF₃ | |
| 1-2163 | OEt | (CH₂)₂OMe | 0 | CF₃ | |
| 1-2164 | OEt | (CH₂)₃OMe | 0 | CF₃ | |
| 1-2165 | OEt | (CH₂)₂OEt | 0 | CF₃ | |
| 1-2166 | OEt | (CH₂)₂OCH₂-c-Pr | 0 | CF₃ | |
| 1-2167 | | | | | |
| 1-2168 | OEt | CH₂-c-Pr | 1 | CF₃ | |
| 1-2169 | OEt | (CH₂)₂OMe | 1 | CF₃ | |
| 1-2170 | OEt | (CH₂)₃OMe | 1 | CF₃ | |
| 1-2171 | OEt | (CH₂)₂OEt | 1 | CF₃ | |
| 1-2172 | OEt | (CH₂)₂OCH₂-c-Pr | 1 | CF₃ | |
| 1-2173 | | | | | |
| 1-2174 | OEt | CH₂-c-Pr | 2 | CF₃ | |
| 1-2175 | OEt | (CH₂)₂OMe | 2 | CF₃ | |
| 1-2176 | OEt | (CH₂)₃OMe | 2 | CF₃ | |
| 1-2177 | OEt | (CH₂)₂OEt | 2 | CF₃ | |
| 1-2178 | OEt | (CH₂)₂OCH₂-c-Pr | 2 | CF₃ | |
| 1-2179 | | | | | |
| 1-2180 | NO₂ | CH₂-c-Pr | 0 | CF₃ | |
| 1-2181 | NO₂ | (CH₂)₂OMe | 0 | CF₃ | |
| 1-2182 | NO₂ | (CH₂)₃OMe | 0 | CF₃ | |
| 1-2183 | NO₂ | (CH₂)₂OEt | 0 | CF₃ | |
| 1-2184 | NO₂ | (CH₂)₂OCH₂-c-Pr | 0 | CF₃ | |
| 1-2185 | | | | | |
| 1-2186 | NO₂ | CH₂-c-Pr | 1 | CF₃ | |
| 1-2187 | NO₂ | (CH₂)₂OMe | 1 | CF₃ | |
| 1-2188 | NO₂ | (CH₂)₃OMe | 1 | CF₃ | |
| 1-2189 | NO₂ | (CH₂)₂OEt | 1 | CF₃ | |
| 1-2190 | NO₂ | (CH₂)₂OCH₂-c-Pr | 1 | CF₃ | |
| 1-2191 | | | | | |
| 1-2192 | NO₂ | CH₂-c-Pr | 2 | CF₃ | |
| 1-2193 | NO₂ | (CH₂)₂OMe | 2 | CF₃ | |
| 1-2194 | NO₂ | (CH₂)₃OMe | 2 | CF₃ | |
| 1-2195 | NO₂ | (CH₂)₂OEt | 2 | CF₃ | |
| 1-2196 | NO₂ | (CH₂)₂OCH₂-c-Pr | 2 | CF₃ | |
| 1-2197 | | | | | |
| 1-2198 | SO₂Me | CH₂-c-Pr | 0 | CF₃ | |
| 1-2199 | SO₂Me | (CH₂)₂OMe | 0 | CF₃ | |
| 1-2200 | SO₂Me | (CH₂)₃OMe | 0 | CF₃ | |
| 1-2201 | SO₂Me | (CH₂)₂OEt | 0 | CF₃ | |
| 1-2202 | SO₂Me | (CH₂)₂OCH₂-c-Pr | 0 | CF₃ | |
| 1-2203 | | | | | |
| 1-2204 | SO₂Me | CH₂-c-Pr | 1 | CF₃ | |
| 1-2205 | SO₂Me | (CH₂)₂OMe | 1 | CF₃ | |
| 1-2206 | SO₂Me | (CH₂)₃OMe | 1 | CF₃ | |
| 1-2207 | SO₂Me | (CH₂)₂OEt | 1 | CF₃ | |
| 1-2208 | SO₂Me | (CH₂)₂OCH₂-c-Pr | 1 | CF₃ | |
| 1-2209 | | | | | |
| 1-2210 | SO₂Me | CH₂-c-Pr | 2 | CF₃ | |
| 1-2211 | SO₂Me | (CH₂)₂OMe | 2 | CF₃ | |
| 1-2212 | SO₂Me | (CH₂)₃OMe | 2 | CF₃ | |
| 1-2213 | SO₂Me | (CH₂)₂OEt | 2 | CF₃ | |
| 1-2214 | SO₂Me | (CH₂)₂OCH₂-c-Pr | 2 | CF₃ | |
| 1-2215 | | | | | |
| 1-2216 | CH₂OMe | CH₂-c-Pr | 0 | CF₃ | |
| 1-2217 | CH₂OMe | (CH₂)₂OMe | 0 | CF₃ | |
| 1-2218 | CH₂OMe | (CH₂)₃OMe | 0 | CF₃ | |
| 1-2219 | CH₂OMe | (CH₂)₂OEt | 0 | CF₃ | |
| 1-2220 | CH₂OMe | (CH₂)₂OCH₂-c-Pr | 0 | CF₃ | |
| 1-2221 | | | | | |
| 1-2222 | CH₂OMe | CH₂-c-Pr | 1 | CF₃ | |
| 1-2223 | CH₂OMe | (CH₂)₂OMe | 1 | CF₃ | |
| 1-2224 | CH₂OMe | (CH₂)₃OMe | 1 | CF₃ | |
| 1-2225 | CH₂OMe | (CH₂)₂OEt | 1 | CF₃ | |
| 1-2226 | CH₂OMe | (CH₂)₂OCH₂-c-Pr | 1 | CF₃ | |
| 1-2227 | | | | | |
| 1-2228 | CH₂OMe | CH₂-c-Pr | 2 | CF₃ | |
| 1-2229 | CH₂OMe | (CH₂)₂OMe | 2 | CF₃ | |
| 1-2230 | CH₂OMe | (CH₂)₃OMe | 2 | CF₃ | |
| 1-2231 | CH₂OMe | (CH₂)₂OEt | 2 | CF₃ | |
| 1-2232 | CH₂OMe | (CH₂)₂OCH₂-c-Pr | 2 | CF₃ | |
| 1-2233 | | | | | |
| 1-2234 | CH₂SO₂Me | CH₂-c-Pr | 0 | CF₃ | |
| 1-2235 | CH₂SO₂Me | (CH₂)₂OMe | 0 | CF₃ | |
| 1-2236 | CH₂SO₂Me | (CH₂)₃OMe | 0 | CF₃ | |
| 1-2237 | CH₂SO₂Me | (CH₂)₂OEt | 0 | CF₃ | |
| 1-2238 | CH₂SO₂Me | (CH₂)₂OCH₂-c-Pr | 0 | CF₃ | |
| 1-2239 | | | | | |
| 1-2240 | CH₂SO₂Me | CH₂-c-Pr | 1 | CF₃ | |
| 1-2241 | CH₂SO₂Me | (CH₂)₂OMe | 1 | CF₃ | |
| 1-2242 | CH₂SO₂Me | (CH₂)₃OMe | 1 | CF₃ | |
| 1-2243 | CH₂SO₂Me | (CH₂)₂OEt | 1 | CF₃ | |
| 1-2244 | CH₂SO₂Me | (CH₂)₂OCH₂-c-Pr | 1 | CF₃ | |
| 1-2245 | | | | | |
| 1-2246 | CH₂SO₂Me | CH₂-c-Pr | 2 | CF₃ | |
| 1-2247 | CH₂SO₂Me | (CH₂)₂OMe | 2 | CF₃ | |
| 1-2248 | CH₂SO₂Me | (CH₂)₃OMe | 2 | CF₃ | |
| 1-2249 | CH₂SO₂Me | (CH₂)₂OEt | 2 | CF₃ | |
| 1-2250 | CH₂SO₂Me | (CH₂)₂OCH₂-c-Pr | 2 | CF₃ | |
| 1-2251 | | | | | |
| 1-2252 | CH₂O(CH₂)₂OMe | CH₂-c-Pr | 0 | CF₃ | |
| 1-2253 | CH₂O(CH₂)₂OMe | (CH₂)₂OMe | 0 | CF₃ | |
| 1-2254 | CH₂O(CH₂)₂OMe | (CH₂)₃OMe | 0 | CF₃ | |
| 1-2255 | CH₂O(CH₂)₂OMe | (CH₂)₂OEt | 0 | CF₃ | |
| 1-2256 | CH₂O(CH₂)₂OMe | (CH₂)₂OCH₂-c-Pr | 0 | CF₃ | |
| 1-2257 | | | | | |
| 1-2258 | CH₂O(CH₂)₂OMe | CH₂-c-Pr | 1 | CF₃ | |
| 1-2259 | CH₂O(CH₂)₂OMe | (CH₂)₂OMe | 1 | CF₃ | |
| 1-2260 | CH₂O(CH₂)₂OMe | (CH₂)₃OMe | 1 | CF₃ | |
| 1-2261 | CH₂O(CH₂)₂OMe | (CH₂)₂OEt | 1 | CF₃ | |
| 1-2262 | CH₂O(CH₂)₂OMe | (CH₂)₂OCH₂-c-Pr | 1 | CF₃ | |
| 1-2263 | | | | | |
| 1-2264 | CH₂O(CH₂)₂OMe | CH₂-c-Pr | 2 | CF₃ | |
| 1-2265 | CH₂O(CH₂)₂OMe | (CH₂)₂OMe | 2 | CF₃ | |
| 1-2266 | CH₂O(CH₂)₂OMe | (CH₂)₃OMe | 2 | CF₃ | |
| 1-2267 | CH₂O(CH₂)₂OMe | (CH₂)₂OEt | 2 | CF₃ | |
| 1-2268 | CH₂O(CH₂)₂OMe | (CH₂)₂OCH₂-c-Pr | 2 | CF₃ | |
| 1-2269 | | | | | |
| 1-2270 | CH₂O(CH₂)₂OEt | CH₂-c-Pr | 0 | CF₃ | |
| 1-2271 | CH₂O(CH₂)₂OEt | (CH₂)₂OMe | 0 | CF₃ | |
| 1-2272 | CH₂O(CH₂)₂OEt | (CH₂)₃OMe | 0 | CF₃ | |
| 1-2273 | CH₂O(CH₂)₂OEt | (CH₂)₂OEt | 0 | CF₃ | |
| 1-2274 | CH₂O(CH₂)₂OEt | (CH₂)₂OCH₂-c-Pr | 0 | CF₃ | |
| 1-2275 | | | | | |
| 1-2276 | CH₂O(CH₂)₂OEt | CH₂-c-Pr | 1 | CF₃ | |
| 1-2277 | CH₂O(CH₂)₂OEt | (CH₂)₂OMe | 1 | CF₃ | |
| 1-2278 | CH₂O(CH₂)₂OEt | (CH₂)₃OMe | 1 | CF₃ | |
| 1-2279 | CH₂O(CH₂)₂OEt | (CH₂)₂OEt | 1 | CF₃ | |
| 1-2280 | CH₂O(CH₂)₂OEt | (CH₂)₂OCH₂-c-Pr | 1 | CF₃ | |
| 1-2281 | | | | | |
| 1-2282 | CH₂O(CH₂)₂OEt | CH₂-c-Pr | 2 | CF₃ | |
| 1-2283 | CH₂O(CH₂)₂OEt | (CH₂)₂OMe | 2 | CF₃ | |
| 1-2284 | CH₂O(CH₂)₂OEt | (CH₂)₃OMe | 2 | CF₃ | |
| 1-2285 | CH₂O(CH₂)₂OEt | (CH₂)₂OEt | 2 | CF₃ | |
| 1-2286 | CH₂O(CH₂)₂OEt | (CH₂)₂OCH₂-c-Pr | 2 | CF₃ | |
| 1-2287 | | | | | |
| 1-2288 | CH₂O(CH₂)₃OMe | CH₂-c-Pr | 0 | CF₃ | |
| 1-2289 | CH₂O(CH₂)₃OMe | (CH₂)₂OMe | 0 | CF₃ | |
| 1-2290 | CH₂O(CH₂)₃OMe | (CH₂)₃OMe | 0 | CF₃ | |
| 1-2291 | CH₂O(CH₂)₃OMe | (CH₂)₂OEt | 0 | CF₃ | |
| 1-2292 | CH₂O(CH₂)₃OMe | (CH₂)₂OCH₂-c-Pr | 0 | CF₃ | |
| 1-2293 | | | | | |
| 1-2294 | CH₂O(CH₂)₃OMe | CH₂-c-Pr | 1 | CF₃ | |
| 1-2295 | CH₂O(CH₂)₃OMe | (CH₂)₂OMe | 1 | CF₃ | |
| 1-2296 | CH₂O(CH₂)₃OMe | (CH₂)₃OMe | 1 | CF₃ | |
| 1-2297 | CH₂O(CH₂)₃OMe | (CH₂)₂OEt | 1 | CF₃ | |
| 1-2298 | CH₂O(CH₂)₃OMe | (CH₂)₂OCH₂-c-Pr | 1 | CF₃ | |
| 1-2299 | | | | | |
| 1-2300 | CH₂O(CH₂)₃OMe | CH₂-c-Pr | 2 | CF₃ | |
| 1-2301 | CH₂O(CH₂)₃OMe | (CH₂)₂OMe | 2 | CF₃ | |
| 1-2302 | CH₂O(CH₂)₃OMe | (CH₂)₃OMe | 2 | CF₃ | |
| 1-2303 | CH₂O(CH₂)₃OMe | (CH₂)₂OEt | 2 | CF₃ | |
| 1-2304 | CH₂O(CH₂)₃OMe | (CH₂)₂OCH₂-c-Pr | 2 | CF₃ | |
| 1-2305 | | | | | |
| 1-2306 | CH₂OCH₂OMe | CH₂-c-Pr | 0 | CF₃ | |
| 1-2307 | CH₂OCH₂OMe | (CH₂)₂OMe | 0 | CF₃ | |
| 1-2308 | CH₂OCH₂OMe | (CH₂)₃OMe | 0 | CF₃ | |
| 1-2309 | CH₂OCH₂OMe | (CH₂)₂OEt | 0 | CF₃ | |
| 1-2310 | CH₂OCH₂OMe | (CH₂)₂OCH₂-c-Pr | 0 | CF₃ | |
| 1-2311 | | | | | |
| 1-2312 | CH₂OCH₂OMe | CH₂-c-Pr | 1 | CF₃ | |
| 1-2313 | CH₂OCH₂OMe | (CH₂)₂OMe | 1 | CF₃ | |
| 1-2314 | CH₂OCH₂OMe | (CH₂)₃OMe | 1 | CF₃ | |
| 1-2315 | CH₂OCH₂OMe | (CH₂)₂OEt | 1 | CF₃ | |
| 1-2316 | CH₂OCH₂OMe | (CH₂)₂OCH₂-c-Pr | 1 | CF₃ | |
| 1-2317 | | | | | |
| 1-2318 | CH₂OCH₂OMe | CH₂-c-Pr | 2 | CF₃ | |
| 1-2319 | CH₂OCH₂OMe | (CH₂)₂OMe | 2 | CF₃ | |
| 1-2320 | CH₂OCH₂OMe | (CH₂)₃OMe | 2 | CF₃ | |
| 1-2321 | CH₂OCH₂OMe | (CH₂)₂OEt | 2 | CF₃ | |
| 1-2322 | CH₂OCH₂OMe | (CH₂)₂OCH₂-c-Pr | 2 | CF₃ | |
| 1-2323 | | | | | |
| 1-2324 | CH₂OCH₂OEt | CH₂-c-Pr | 0 | CF₃ | |
| 1-2325 | CH₂OCH₂OEt | (CH₂)₂OMe | 0 | CF₃ | |
| 1-2326 | CH₂OCH₂OEt | (CH₂)₃OMe | 0 | CF₃ | |
| 1-2327 | CH₂OCH₂OEt | (CH₂)₂OEt | 0 | CF₃ | |
| 1-2328 | CH₂OCH₂OEt | (CH₂)₂OCH₂-c-Pr | 0 | CF₃ | |
| 1-2329 | | | | | |
| 1-2330 | CH₂OCH₂OEt | CH₂-c-Pr | 1 | CF₃ | |
| 1-2331 | CH₂OCH₂OEt | (CH₂)₂OMe | 1 | CF₃ | |
| 1-2332 | CH₂OCH₂OEt | (CH₂)₃OMe | 1 | CF₃ | |
| 1-2333 | CH₂OCH₂OEt | (CH₂)₂OEt | 1 | CF₃ | |
| 1-2334 | CH₂OCH₂OEt | (CH₂)₂OCH₂-c-Pr | 1 | CF₃ | |
| 1-2335 | | | | | |
| 1-2336 | CH₂OCH₂OEt | CH₂-c-Pr | 2 | CF₃ | |
| 1-2337 | CH₂OCH₂OEt | (CH₂)₂OMe | 2 | CF₃ | |
| 1-2338 | CH₂OCH₂OEt | (CH₂)₃OMe | 2 | CF₃ | |
| 1-2339 | CH₂OCH₂OEt | (CH₂)₂OEt | 2 | CF₃ | |
| 1-2340 | CH₂OCH₂OEt | (CH₂)₂OCH₂-c-Pr | 2 | CF₃ | |
| 1-2341 | | | | | |
| 1-2342 | CH₂O(CH₂)₂SO₂Me | CH₂-c-Pr | 0 | CF₃ | |
| 1-2343 | CH₂O(CH₂)₂SO₂Me | (CH₂)₂OMe | 0 | CF₃ | |
| 1-2344 | CH₂O(CH₂)₂SO₂Me | (CH₂)₃OMe | 0 | CF₃ | |
| 1-2345 | CH₂O(CH₂)₂SO₂Me | (CH₂)₂OEt | 0 | CF₃ | |
| 1-2346 | CH₂O(CH₂)₂SO₂Me | (CH₂)₂OCH₂-c-Pr | 0 | CF₃ | |
| 1-2347 | | | | | |
| 1-2348 | CH₂O(CH₂)₂SO₂Me | CH₂-c-Pr | 1 | CF₃ | |
| 1-2349 | CH₂O(CH₂)₂SO₂Me | (CH₂)₂OMe | 1 | CF₃ | |
| 1-2350 | CH₂O(CH₂)₂SO₂Me | (CH₂)₃OMe | 1 | CF₃ | |
| 1-2351 | CH₂O(CH₂)₂SO₂Me | (CH₂)₂OEt | 1 | CF₃ | |
| 1-2352 | CH₂O(CH₂)₂SO₂Me | (CH₂)₂OCH₂-c-Pr | 1 | CF₃ | |
| 1-2353 | | | | | |
| 1-2354 | CH₂O(CH₂)₂SO₂Me | CH₂-c-Pr | 2 | CF₃ | |
| 1-2355 | CH₂O(CH₂)₂SO₂Me | (CH₂)₂OMe | 2 | CF₃ | |
| 1-2356 | CH₂O(CH₂)₂SO₂Me | (CH₂)₃OMe | 2 | CF₃ | |
| 1-2357 | CH₂O(CH₂)₂SO₂Me | (CH₂)₂OEt | 2 | CF₃ | |
| 1-2358 | CH₂O(CH₂)₂SO₂Me | (CH₂)₂OCH₂-c-Pr | 2 | CF₃ | |
| 1-2359 | | | | | |
| 1-2360 | CH₂SO₂(CH₂)₂OMe | CH₂-c-Pr | 0 | CF₃ | |
| 1-2361 | CH₂SO₂(CH₂)₂OMe | (CH₂)₂OMe | 0 | CF₃ | |
| 1-2362 | CH₂SO₂(CH₂)₂OMe | (CH₂)₃OMe | 0 | CF₃ | |
| 1-2363 | CH₂SO₂(CH₂)₂OMe | (CH₂)₂OEt | 0 | CF₃ | |
| 1-2364 | CH₂SO₂(CH₂)₂OMe | (CH₂)₂OCH₂-c-Pr | 0 | CF₃ | |
| 1-2365 | | | | | |
| 1-2366 | CH₂SO₂(CH₂)₂OMe | CH₂-c-Pr | 1 | CF₃ | |
| 1-2367 | CH₂SO₂(CH₂)₂OMe | (CH₂)₂OMe | 1 | CF₃ | |
| 1-2368 | CH₂SO₂(CH₂)₂OMe | (CH₂)₃OMe | 1 | CF₃ | |
| 1-2369 | CH₂SO₂(CH₂)₂OMe | (CH₂)₂OEt | 1 | CF₃ | |
| 1-2370 | CH₂SO₂(CH₂)₂OMe | (CH₂)₂OCH₂-c-Pr | 1 | CF₃ | |
| 1-2371 | | | | | |
| 1-2372 | CH₂SO₂(CH₂)₂OMe | CH₂-c-Pr | 2 | CF₃ | |
| 1-2373 | CH₂SO₂(CH₂)₂OMe | (CH₂)₂OMe | 2 | CF₃ | |
| 1-2374 | CH₂SO₂(CH₂)₂OMe | (CH₂)₃OMe | 2 | CF₃ | |
| 1-2375 | CH₂SO₂(CH₂)₂OMe | (CH₂)₂OEt | 2 | CF₃ | |
| 1-2376 | CH₂SO₂(CH₂)₂OMe | (CH₂)₂OCH₂-c-Pr | 2 | CF₃ | |
| 1-2377 | | | | | |
| 1-2378 | CH₂SO₂(CH₂)₂SO₂Me | CH₂-c-Pr | 0 | CF₃ | |
| 1-2379 | CH₂SO₂(CH₂)₂SO₂Me | (CH₂)₂OMe | 0 | CF₃ | |
| 1-2380 | CH₂SO₂(CH₂)₂SO₂Me | (CH₂)₃OMe | 0 | CF₃ | |
| 1-2381 | CH₂SO₂(CH₂)₂SO₂Me | (CH₂)₂OEt | 0 | CF₃ | |
| 1-2382 | CH₂SO₂(CH₂)₂SO₂Me | (CH₂)₂OCH₂-c-Pr | 0 | CF₃ | |
| 1-2383 | | | | | |
| 1-2384 | CH₂SO₂(CH₂)₂SO₂Me | CH₂-c-Pr | 1 | CF₃ | |
| 1-2385 | CH₂SO₂(CH₂)₂SO₂Me | (CH₂)₂OMe | 1 | CF₃ | |
| 1-2386 | CH₂SO₂(CH₂)₂SO₂Me | (CH₂)₃OMe | 1 | CF₃ | |
| 1-2387 | CH₂SO₂(CH₂)₂SO₂Me | (CH₂)₂OEt | 1 | CF₃ | |
| 1-2388 | CH₂SO₂(CH₂)₂SO₂Me | (CH₂)₂OCH₂-c-Pr | 1 | CF₃ | |
| 1-2389 | | | | | |
| 1-2390 | CH₂SO₂(CH₂)₂SO₂Me | CH₂-c-Pr | 2 | CF₃ | |
| 1-2391 | CH₂SO₂(CH₂)₂SO₂Me | (CH₂)₂OMe | 2 | CF₃ | |
| 1-2392 | CH₂SO₂(CH₂)₂SO₂Me | (CH₂)₃OMe | 2 | CF₃ | |
| 1-2393 | CH₂SO₂(CH₂)₂SO₂Me | (CH₂)₂OEt | 2 | CF₃ | |
| 1-2394 | CH₂SO₂(CH₂)₂SO₂Me | (CH₂)₂OCH₂-c-Pr | 2 | CF₃ | |

Ebenso ganz besonders bevorzugt sind alle der oben genannten Verbindungen der Nr. 1-1 bis 1-2394, in denen R⁴ für n-Propylsulfonyl steht.

Ebenso ganz besonders bevorzugt sind alle der oben genannten Verbindungen der Nr. 1-1 bis 1-2394, in denen R⁴ für Phenylsulfonyl steht.

Ebenso ganz besonders bevorzugt sind alle der oben genannten Verbindungen der Nr. 1-1 bis 1-2394, in denen R⁴ für Methoxyethylsulfonyl steht.

Ebenso ganz besonders bevorzugt sind alle der oben genannten Verbindungen der Nr. 1-1 bis 1-2394, in denen R⁴ für Benzoylmethyl steht.

Ebenso ganz besonders bevorzugt sind alle der oben genannten Verbindungen der Nr. 1-1 bis 1-2394, in denen R⁴ für 4-Methylphenylsulfonyl steht.

Ebenso ganz besonders bevorzugt sind alle der oben genannten Verbindungen der Nr. 1-1 bis 1-2394, in denen R⁴ für Thien-2-ylsulfonyl steht.

Ebenso ganz besonders bevorzugt sind alle der oben genannten Verbindungen der Nr. 1-1 bis 1-2394, in denen R⁴ für Benzoyl steht.

Ebenso ganz besonders bevorzugt sind alle der oben genannten Verbindungen der Nr. 1-1 bis 1-2394, in denen R⁴ für 4-Methylbenzoylmethyl steht.

Ebenso ganz besonders bevorzugt sind alle der oben genannten Verbindungen der Nr. 1-1 bis 1-2394, in denen R⁴ für (Ethylthio)carbonyl steht.

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

**75 Gew.-Teile einer Verbindung der allgemeinen Formel (I),**

| | | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

**25 Gew.-Teile einer Verbindung der allgemeinen Formel (I),**

| | | |
|---|---|---|
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 800l/ha in einer Dosierung von 320 Gramm pro Hektar auf die Oberfläche der Abdeckerde appliziert. Anschließend werden die Töpfe zur weiteren Kultivierung der Pflanzen im Gewächshaus unter optimalen Bedingungen gehalten. Die optische Bewertung der Schäden an den Schadpflanzen erfolgt 3-4 Wochen nach der Behandlung. Dabei zeigen die Verbindungen der Nr. 1-3, 1-15, 1-44 und 1-1407 eine mindestens 90%-ige Wirkung gegen Echinochioa crus galli. Die Verbindungen der Nr. 1-15, 1-45, 1-1407 und 1-1419 zeigen eine mindestens 90%-ige Wirkung gegen Abutilon theophrasti. Die Verbindungen der Nr. 1-3, 1-45, 1-1406 und 1-1418 zeigen eine mindestens 90%-ige Wirkung gegen Amaranthus retroflexus. Die Verbindungen der Nr. 1-44, 1-1406, 1-1407 und 1-1419 zeigen eine mindestens 90%-ige Wirkung gegen Stellaria media.

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha in einer Dosierung von 80 Gramm pro Hektar auf die Oberfläche der grünen Pflanzenteile gesprüht. Die optische Bewertung der Schäden an den Schadpflanzen erfolgt 3-4 Wochen nach der Behandlung. Dabei zeigen die Verbindungen der Nr. 1-39, 1-40, 1-1382 und 1-2091 eine mindestens 90%-ige Wirkung gegen Echinochloa crus galli. Die Verbindungen der Nr. 1-3, 1-44 und 1-2091 zeigen eine mindestens 90%-ige Wirkung gegen Abutilon theophrasti. Die Verbindungen der Nr. 1-39, 1-40 und 1-1382 zeigen eine mindestens 90%-ige Wirkung gegen Veronica persica. Die Verbindungen der Nr. 1-3, 1-38, 1-39, 1-1406 und 1-1407 zeigen eine mindestens 90%-ige Wirkung gegen Stellaria media.

### 3. Vergleichsversuche

Um die Überlegenheit der erfindungsgemäßen Verbindungen gegenüber den aus dem Stand der Technik bekannten Verbindungen (WO 97/41106 und WO 00/03993) zu zeigen, wurde die herbizide Wirkung gegen Schadpflanzen beziehungsweise die Schädigung von Kulturpflanzen unter den oben genannten Bedingungen im Vor- und im Nachauflauf in Vergleichsversuchen gegenübergestellt. Die nachfolgenden Vergleichsversuche der Tabellen 1 bis 24 zeigen die Überlegenheit der erfindungsgemäßen Verbindungen gegenüber den aus dem Stand der Technik bekannten Verbindungen.

Die darin verwendeten Abkürzungen bedeuten:

**Schadpflanzen**

| | | | |
|---|---|---|---|
| ABUTH | Abutilon theophrasti | ALOMY | Alopecurus myosuroides |
| AMARE | Amaranthus retroflexus | AVEFA | Avena fatua |
| CHEAL | Chenopodium album | ECHCG | Echinochloa crus galli |
| GALAP | Galium aparine | LOLMU | Lolium multiflorum |
| MATIN | Matricaria inodora | PHBPU | Pharbitis purpureum |
| POLCO | Fallopia convolvulus | STEME | Stellaria media |
| VERPE | Veronica persica | VIOTR | Viola tricolor |
| XANST | Xanthium strumarium | | |

**Kulturpflanzen**

| | | | |
|---|---|---|---|
| *GLXMA* | *Glycine max (Sojabohne)* | *TRZAS* | *Triticum aestivum (Weizen)* |
| *ZEAMX* | *Zea mays (Mais)* | | |

**Tabelle 1: Wirkung im Vorauflauf**

| Verbindung Nr. | Dosierung | Herbizide Wirkung in % gegen |
|---|---|---|
| | [g a.i./ha] | VIOTR |
| 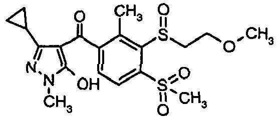 | 80 | 70 |
| erfindungsgemäßes Beispiel Nr. 1-45 | | |
| 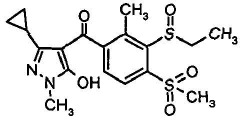 | 80 | 60 |
| aus dem Stand der Technik bekannte Verbindung | | |

**Tabelle 2: Wirkung im Vorauflauf**

| Verbindung Nr. | Dosierung | Herbizide Wirkung in % gegen | | |
|---|---|---|---|---|
| | [g a.i./ha] | PHBPU | POLCO | XANST |
| 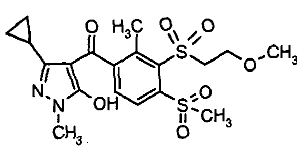 | 320 | 60 | 60 | 70 |
| erfindungsgemäßes Beispiel Nr. 1-51 | | | | |
| 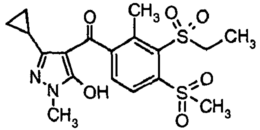 | 320 | 20 | 20 | 0 |
| aus dem Stand der Technik bekannte Verbindung | | | | |

**Tabelle 3: Wirkung im Vorauflauf**

| Verbindung Nr. | Dosierung | Herbizide Wirkung in % gegen | | |
|---|---|---|---|---|
| | [g a.i./ha] | PHBPU | POLCO | XANST |
| 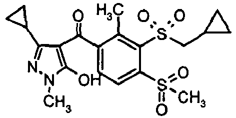 | 320 | 50 | 50 | 60 |
| erfindungsgemäßes Beispiel Nr. 1-50 | | | | |
| 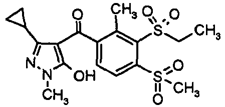 | 320 | 20 | 20 | 0 |
| aus dem Stand der Technik bekannte Verbindung | | | | |

**Tabelle 4: Wirkung im Vorauflauf**

| Verbindung Nr. | Dosierung | Herbizide Wirkung in % gegen | | |
|---|---|---|---|---|
| | [g a.i./ha] | MATIN | STEME | VERPE |
| 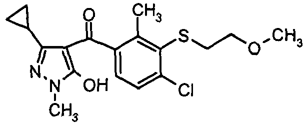 | 80 | 60 | 90 | 90 |
| erfindungsgemäßes Beispiel Nr.1-1407 | | | | |
| 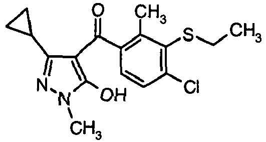 | 80 | 30 | 60 | 30 |
| aus dem Stand der Technik bekannte Verbindung | | | | |

**Tabelle 5: Wirkung im Vorauflauf**

| Verbindung Nr. | Dosierung | Herbizide Wirkung in % gegen | |
|---|---|---|---|
| | [g a.i./ha] | ABUTH | MATIN |
| 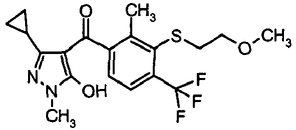 | 320 | 100 | 60 |
| erfindungsgemäßes Beispiel Nr. 1-2091 | | | |
| 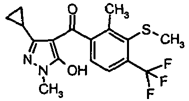 | 320 | 50 | 0 |
| aus dem Stand der Technik bekannte Verbindung | | | |

**Tabelle 6: Wirkung im Vorauflauf**

| Verbindung Nr. | Dosierung | Herbizide Wirkung in % gegen | |
|---|---|---|---|
| | [g a.i./ha] | ABUTH | MATIN |
| 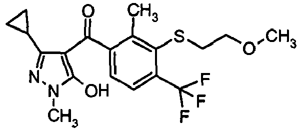 | 320 | 100 | 60 |
| erfindungsgemäßes Beispiel Nr. 1-2091 | | | |
| 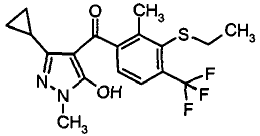 | 320 | 90 | 0 |
| aus dem Stand der Technik bekannte Verbindung | | | |

**Tabelle 7: Wirkung im Vorauflauf**

| Verbindung Nr. | Dosierung | Herbizide Wirkung in % gegen | | | | |
|---|---|---|---|---|---|---|
| | [g a.i./ha] | ABUTH | AMARE | MATIN | POLCO | VIOTR |
| 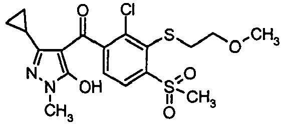 | 320 | 100 | 100 | 60 | 50 | 100 |
| erfindungsgemäßes Beispiel Nr. 1-3 | | | | | | |
| 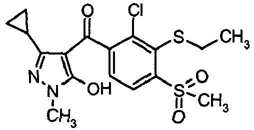 | 320 | 70 | 90 | 0 | 0 | 50 |
| aus dem Stand der Technik bekannte Verbindung | | | | | | |

**Tabelle 8: Wirkung im Vorauflauf**

| Verbindung Nr. | Dosierung | Herbizide Wirkung in % gegen | | | | |
|---|---|---|---|---|---|---|
| | [g a.i./ha] | ABUTH | AMARE | MATIN | POLCO | VIOTR |
| 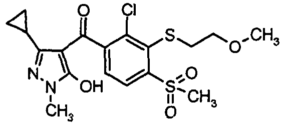 | 320 | 100 | 100 | 60 | 50 | 100 |
| erfindungsgemäßes Beispiel Nr. 1-3 | | | | | | |
| 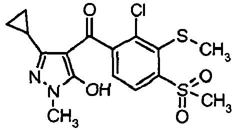 | 320 | 60 | 90 | 0 | 40 | 50 |
| aus dem Stand der Technik bekannte Verbindung | | | | | | |

**Tabelle 9: Wirkung im Nachauflauf**

| Verbindung Nr. | Dosierung | Herbizide Wirkung in % gegen | | |
|---|---|---|---|---|
| | [g a.i./ha] | ECHCG | VIOTR | XANST |
| 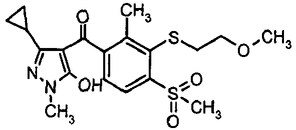 | 80 | 90 | 90 | 90 |
| erfindungsgemäßes Beispiel Nr. 1-39 | | | | |
| 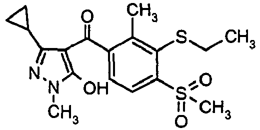 | 80 | 80 | 70 | 80 |
| aus dem Stand der Technik bekannte Verbindung | | | | |

**Tabelle 10: Wirkung im Nachauflauf**

| Verbindung Nr. | Dosierung | Herbizide Wirkung in % gegen | | |
|---|---|---|---|---|
| | [g a.i./ha] | ECHCG | VIOTR | XANST |
| 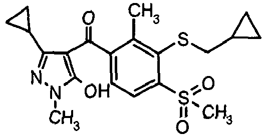 | 80 | 100 | 90 | 90 |
| erfindungsgemäßes Beispiel Nr. 1-38 | | | | |
| 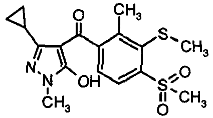 | 80 | 80 | 70 | 60 |
| aus dem Stand der Technik bekannte Verbindung | | | | |

**Tabelle 11: Wirkung im Nachauflauf**

| Verbindung Nr. | Dosierung | Herbizide Wirkung in % gegen | | |
|---|---|---|---|---|
| | [g a.i./ha] | PHBPU | POLCO | VIOTR |
| 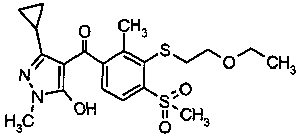 | 80 | 60 | 70 | 80 |
| erfindungsgemäßes Beispiel Nr. 1-41 | | | | |
| 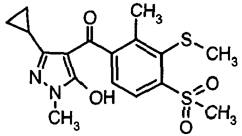 | 80 | 40 | 50 | 70 |
| aus dem Stand der Technik bekannte Verbindung | | | | |

**Tabelle 12: Wirkung im Nachauflauf**

| Verbindung Nr. | Dosierung | Herbizide Wirkung in % gegen | | *Schädigung von* |
|---|---|---|---|---|
| | [g a.i./ha] | VERPE | VIOTR | *TRZAS* |
| 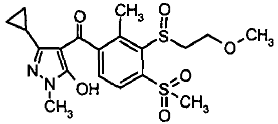 | 80 | 100 | 70 | 20 |
| erfindungsgemäßes Beispiel Nr. 1-45 | | | | |
| 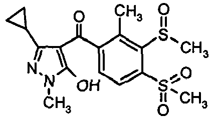 | 80 | 80 | 60 | 50 |
| aus dem Stand der Technik bekannte Verbindung | | | | |

**Tabelle 13: Wirkung im Nachauflauf**

| Verbindung Nr. | Dosierung | Herbizide Wirkung in % gegen | | | *Schädigung von* | |
|---|---|---|---|---|---|---|
| | [g a.i./ha] | VERPE | VIOTR | GALAP | *TRZAS* | *ZEAMX* |
| 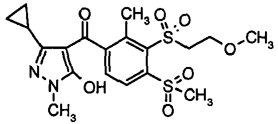 | 20 | 90 | 70 | 50 | 0 | 0 |
| erfindungsgemäßes Beispiel Nr. 1-51 | | | | | | |
| 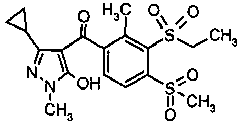 | 20 | 20 | 20 | 20 | *80* | 10 |
| aus dem Stand der Technik bekannte Verbindung | | | | | | |

**Tabelle 14: Wirkung im Vorauflauf**

| Verbindung Nr. | Dosierung | Herbizide Wirkung in % | | *Schädigung von* | |
|---|---|---|---|---|---|
| | [g a.i./ha] | GALAP | CHEAL | *TRZAS* | *GLXMA* |
| 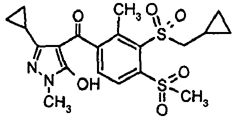 | 320 | 100 | 100 | *0* | *0* |
| erfindungsgemäßes Beispiel Nr. 1-50 | | | | | |
| 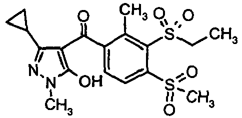 | 320 | 90 | 90 | 50 | 50 |
| aus dem Stand der Technik bekannte Verbindung | | | | | |

**Tabelle 15: Wirkung im Nachauflauf**

| Verbindung Nr. | Dosierung | Herbizide Wirkung in % gegen | | | |
|---|---|---|---|---|---|
| | [g a.i./ha] | ECHCG | PHBPU | MATIN | STEME |
| 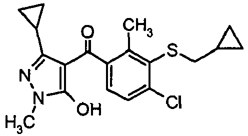 | 80 | 90 | 70 | 70 | 100 |
| erfindungsgemäßes Beispiel Nr. 1-1406 | | | | | |
| 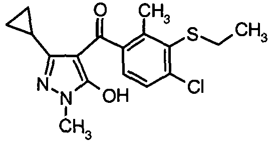 | 80 | 80 | 40 | 40 | 90 |
| aus dem Stand der Technik bekannte Verbindung | | | | | |

**Tabelle 16: Wirkung im Nachauflauf**

| Verbindung Nr. | Dosierung | Herbizide Wirkung in % gegen | | | |
|---|---|---|---|---|---|
| | [g a.i./ha] | ECHCG | PHBPU | MATIN | STEME |
| 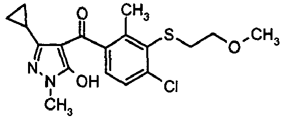 | 80 | 90 | 80 | 80 | 100 |
| erfindungsgemäßes Beispiel Nr.1-1407 | | | | | |
| 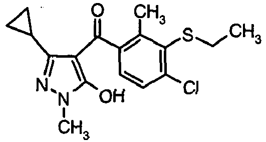 | 80 | 80 | 40 | 40 | 90 |
| aus dem Stand der Technik bekannte Verbindung | | | | | |

**Tabelle 17: Wirkung im Vorauflauf**

| Verbindung Nr. | Dosierung | Herbizide Wirkung in % gegen | | |
|---|---|---|---|---|
| | [g a.i./ha] | AMARE | STEME | VERPE |
| 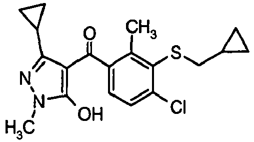 | 80 | 80 | 90 | 70 |
| erfindungsgemäßes Beispiel Nr. 1-1406 | | | | |
| 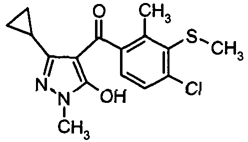 | 80 | 70 | 80 | 50 |
| aus dem Stand der Technik bekannte Verbindung | | | | |

**Tabelle 18: Wirkung im Nachauflauf**

| Verbindung Nr. | Dosierung | Herbizide Wirkung in % gegen | |
|---|---|---|---|
| | [g a.i./ha] | ECHCG | ABUTH |
| 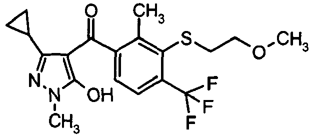 | 20 | 80 | 80 |
| erfindungsgemäßes Beispiel Nr. 1-2091 | | | |
| 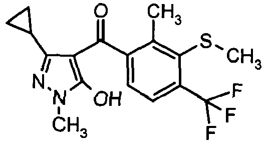 | 20 | 60 | 70 |
| aus dem Stand der Technik bekannte Verbindung | | | |

**Tabelle19: Wirkung im Nachauflauf**

| Verbindung Nr. | Dosierung | Herbizide Wirkung in % gegen | | |
|---|---|---|---|---|
| | [g a.i./ha] | ABUTH | MATIN | VIOTR |
| 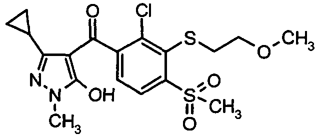 | 80 | 90 | 70 | 70 |
| erfindungsgemäßes Beispiel Nr. 1-3 | | | | |
| 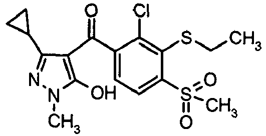 | 80 | 80 | 0 | 60 |
| aus dem Stand der Technik bekannte Verbindung | | | | |

**Tabelle 20: Wirkung im Nachauflauf**

| Verbindung Nr. | Dosierung | Herbizide Wirkung in % gegen | | |
|---|---|---|---|---|
| | [g a.i./ha] | ABUTH | MATIN | VIOTR |
| 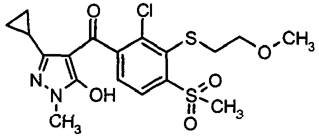 | 80 | 90 | 70 | 70 |
| erfindungsgemäßes Beispiel Nr. 1-3 | | | | |
| 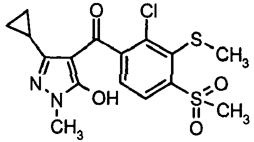 | 80 | 80 | 30 | 60 |
| aus dem Stand der Technik bekannte Verbindung | | | | |

**Tabelle 21: Wirkung im Nachauflauf**

| Verbindung Nr. | Dosierung | Herbizide Wirkung in % gegen | | |
|---|---|---|---|---|
| | [g a.i./ha] | PHBPU | STEME | VERPE |
| 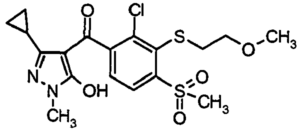 | 80 | 60 | 90 | 100 |
| erfindungsgemäßes Beispiel Nr. 1-3 | | | | |
| 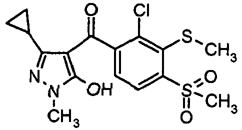 | 80 | 50 | 80 | 80 |
| aus dem Stand der Technik bekannte Verbindung | | | | |

**Tabelle 22: Wirkung im Nachauflauf**

| Verbindung Nr. | Dosierung | Herbizide Wirkung in % gegen | |
|---|---|---|---|
| | [g a.i./ha] | ABUTH | XANST |
| 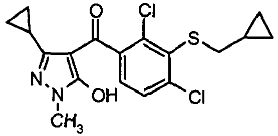 | 20 | 60 | 70 |
| erfindungsgemäßes Beispiel Nr. 1-1370 | | | |
| 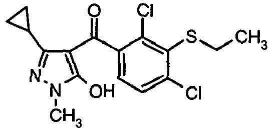 | 20 | 40 | 50 |
| aus dem Stand der Technik bekannte Verbindung | | | |

**Tabelle 23: Wirkung im Nachauflauf**

| Verbindung Nr. | Dosierung | Herbizide Wirkung in % gegen | | | | |
|---|---|---|---|---|---|---|
| | [g a.i./ha] | ALOMY | AVEFA | ECHCG | LOLMU | ABUTH |
| 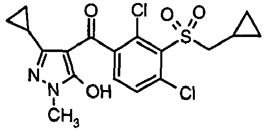 | 80 | 70 | 50 | 90 | 60 | 70 |
| erfindungsgemäßes Beispiel Nr. 1-1382 | | | | | | |
| 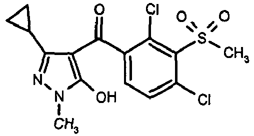 | 80 | 20 | 20 | 30 | 20 | 60 |
| aus dem Stand der Technik bekannte Verbindung | | | | | | |

**Tabelle 24: Wirkung im Nachauflauf**

| Verbindung Nr. | Dosierung | Herbizide Wirkung in % gegen | | | | |
|---|---|---|---|---|---|---|
| | [g a.i./ha] | GALAP | MATIN | STEME | VERPE | XANST |
| 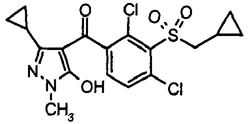 | 80 | 70 | 50 | 100 | 90 | 90 |
| erfindungsgemäßes Beispiel Nr. 1-1382 | | | | | | |
| 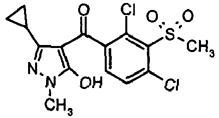 | 80 | 40 | 0 | 80 | 60 | 70 |
| aus dem Stand der Technik bekannte Verbindung | | | | | | |

## Patentansprüche

1. 3-Cyclopropyl-4-(3-thiobenzoyl)pyrazole der Formel (I) oder deren Salze 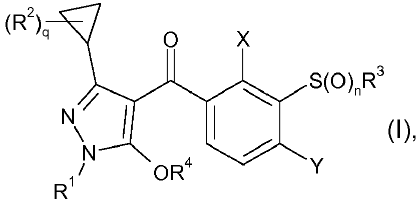 worin
R¹ bedeutet (C₁-C₄)-Alkyl,
R² bedeutet Halogen oder (C₁-C₄)-Alkyl,
R³ bedeutet (C₃-C₈)-Cycloalkyl-(C₁-C₉)-alkyl, (C₁-C₆)-Halogenalkyl, (C₃-C₈)-Halogencycloalkyl-(C₁-C₉)-alkyl, (C₂-C₆)-Nitroalkyl, (C₃-C₈)-Cycloalkoxy-(C₁-C₉)-alkyl, (C₃-C₈)-Cycloalkyl-(C₁-C₉)-alkoxy-(C₁-C₉)-alkyl, (C₁-C₆)-Alkoxy-(C₁-C₉)-alkyl, (C₂-C₆)-Alkenyloxy-(C₁-C₉)-alkyl, (C₂-C₆)-Alkinyloxy-(C₁-C₉)-alkyl, (C₁-C₆)-Halogenalkoxy-(C₁-C₉)-alkyl, (C₃-C₈)-Halogencycloalkyl(C₁-C₉)-alkoxy-(C₁-C₉)-alkyl, (C₂-C₆)-Halogenalkenyloxy-(C₁-C₉)-alkyl, (C₂-C₆)-Halogenalkinyloxy-(C₁-C₉)-alkyl, (C₂-C₆)-Nitroalkoxy-(C₁-C₉)-alkyl, Phenyloxy-(C₁-C₉)-alkyl, wobei die Phenyl-Gruppe jeweils durch m gleiche oder verschiedene Reste aus der Gruppe bestehend aus (C₁-C₃)-Alkyl, Halogen, Nitro, (C₁-C₃)-Alkoxy substituiert sein kann,
R⁴ bedeutet Wasserstoff, (C₁-C₆)-Alkylsulfonyl, (C₁-C₄)-Alkoxy-(C₁-C₆)-alkylsulfonyl, oder jeweils durch m gleiche oder verschiedene Reste aus der Gruppe bestehend aus Halogen, (C₁-C₄)-Alkyl und (C₁-C₄)-Alkoxy substituiertes Phenylsulfonyl, Thien-2-ylsulfonyl, (Ethylthio)carbonyl, Benzoyl, Benzoyl-(C₁-C₆)-alkyl oder Benzyl,
X und Y bedeuten unabhängig voneinander Wasserstoff, Mercapto, Nitro, Halogen, Cyano, Rhodano, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₃-C₆)-Halogenalkinyl, (C₃-C₆)-Cycloalkyl, OR⁵, Methylsulfonylethoxymethyl, Methylsulfonylethylsulfonylmethyl, Methoxyethylsulfonylmethyl, OCOR⁵, OSO₂R⁵, S(O)ₙR⁵, SO₂OR⁵, SO₂N(R⁵)₂, (C₁-C₃)-Alkoxy-(C₁-C₃)-alkoxy)-(C₁-C₃)-alkyl, NR⁵SO₂R⁵, NR⁵COR⁵, (C₁-C₆)-AlkylS(O)ₙR⁵, (C₁-C₆)-Alkyl-OR⁵, (C₁-C₆)-Alkyl-OCOR⁵, (C₁-C₆)-Alkyl-OSO2R⁵, (C₁-C₆)-Alkyl-SO₂OR⁵, (C₁-C₆)-Alkyl-SO₂N(R⁵)₂ oder (C₁-C₆)-Alkyl-NR⁵COR⁵;
R⁵ bedeutet Wasserstoff, (C₁-C₆)-Alkyl, (C₂-C₆)-Alkenyl, (C₂-C₆)-Alkinyl, (C₃-C₆)-Cycloalkyl, Phenyl oder Phenyl-(C₁-C₆)-alkyl, wobei die sechs letztgenannten Reste durch s Reste der Gruppe Hydroxy, Mercapto, Amino, Cyano, Nitro, Rhodano, OR⁶, SR⁶, N(R⁶)₂, NOR⁶, OCOR⁶, SCOR⁶, NR⁶COR⁶, CO₂R⁶, COSR⁶, CON(R⁶)₂, (C₁)-C₄-Alkyliminooxy, (C₁-C₄)-Alkoxyamino, (C₁-C₄)-Alkylcarbonyl, (C₁-C₄)-Alkoxy-(C₂-C₆)-alkoxycarbonyl und (C₁-C₄)-Alkylsulfonyl substituiert sind;
R⁶ bedeutet Wasserstoff, (C₁-C₆)-Alkyl, (C₂-C₆)-Alkenyl oder (C₂-C₆)-Alkinyl,
m bedeutet 0, 1, 2, 3, 4 oder 5,
n bedeutet 0, 1 oder 2,
q bedeutet 0, 1, 2, 3, 4 oder 5,
s bedeutet 0, 1, 2 oder 3,
mit der Maßgabe, dass R³ nicht (C₁-C₆)-Halogenalkyl bedeutet, wenn n für 0 steht.

2. 3-Cyclopropyl-4-(3-thiobenzoyl)pyrazole nach Anspruch 1, worin
R¹ bedeutet (C₁-C₄)-Alkyl,
R² bedeutet Halogen, Methyl oder Ethyl,
R³ bedeutet cyclo-Propylmethyl, cyclo-Propylmethoxyethyl, Methoxyethyl, Methoxypropyl, Ethoxyethyl,
R⁴ bedeutet Wasserstoff, n-Propylsulfonyl, Phenylsulfonyl, Methoxyethylsulfonyl, Benzoylmethyl, Benzoyl, 4-Methylbenzoylmethyl, (Ethylthio)carbonyl, 4-Methylphenylsulfonyl, Thien-2-ylsulfonyl,
X bedeutet Nitro, Halogen, (C₁-C₄)-Alkyl, Trifluormethyl, (C₁-C₄)-Alkoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
Y bedeutet Halogen, Trifluormethyl, (C₁-C₄)-Alkoxy, Methylsulfonyl oder Ethylsulfonyl,
n bedeutet 0, 1 oder 2,
q bedeutet 0, 1 oder 2.

3. 3-Cyclopropyl-4-(3-thiobenzoyl)pyrazole nach Anspruch 1, worin
R¹ bedeutet Methyl oder Ethyl,
R³ bedeutet cyclo-Propylmethyl, cyclo-Propylmethoxyethyl, Methoxyethyl, Methoxypropyl, Ethoxyethyl,
R⁴ bedeutet Wasserstoff, n-Propylsulfonyl, Phenylsulfonyl, Methoxyethylsulfonyl, Benzoylmethyl, Benzoyl, 4-Methylbenzoylmethyl, (Ethylthio)carbonyl, 4-Methylphenylsulfonyl, Thien-2-ylsulfonyl,
X bedeutet Nitro, Brom, Chlor, Fluor, Methyl, Ethyl, Trifluormethyl, Methoxy, Ethoxy, Methylsulfonyl, Methoxymethyl, Methoxymethoxymethyl, Ethoxyethoxymethyl, Ethoxymethoxymethyl, Methoxyethoxymethyl, Methoxypropoxymethyl, Methylsulfonylmethyl, Methylsulfonylethoxymethyl, Methoxyethylsulfonylmethyl, Methylsulfonylethylsulfonylmethyl,
Y bedeutet Brom, Chlor, Fluor, Trifluormethyl, Methoxy, Methylsulfonyl oder Ethylsulfonyl,
n bedeutet 0, 1 oder 2,
q bedeutet 0.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach Anspruch 4 oder 5 auf die Pflanzen oder auf den Ort des unerwünschten Pflanzenwachstums appliziert.

7. Verwendung von Verbindungen der Formel (I) gemäß einem der Ansprüche 1 bis 3 oder von herbiziden Mitteln nach Anspruch 4 oder 5 zur Bekämpfung unerwünschter Pflanzen.

8. Verwendung nach Anspruch 7, **dadurch gekennzeichnet, daß** die Verbindungen der Formel (I) zur Bekämpfung unerwünschter Pflanzen in Kulturen von Nutzpflanzen eingesetzt werden.

9. Verwendung nach Anspruch 8, **dadurch gekennzeichnet, daß** die Nutzpflanzen transgene Nutzpflanzen sind.

## Claims

1. 3-Cyclopropyl-4-(3-thiobenzoyl)pyrazole of the formula (I) or a salt thereof 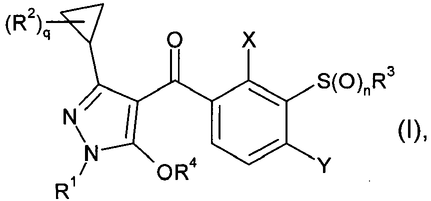 in which
R¹ is (C₁-C₄)-alkyl,
R² is halogen or (C₁-C₄)-alkyl,
R³ is (C₃-C₈)-cycloalkyl-(C₁-C₉)-alkyl, (C₁-C₆)-haloalkyl, (C₃-C₈) -halocycloalkyl-(C₁-C₉)-alkyl, (C₂-C₆)-nitroalkyl, (C₃-C₈) -cycloalkoxy-(C₁-C₉)-alkyl, (C₃-C₈)-cycloalkyl- (C₁-C₉)-alkoxy- (C₁-C₉)-alkyl, (C₁-C₆)-alkoxy-(C₁-C₉)-alkyl, (C₂-C₆)-alkenyloxy-(C₁-C₉)-alkyl, (C₂-C₆)-alkynyloxy-(C₁-C₉)-alkyl, (C₁-C₆)-haloalkoxy-(C₁-C₉)-alkyl, (C₃-C₈)-halocycloalkyl-(C₁-C₉)-alkoxy-(C₁-C₉)-alkyl, (C₂-C₆)-haloalkenyloxy-(C₁-C₉)-alkyl, (C₂-C₆)-haloalkynyloxy-(C₁-C₉)-alkyl, (C₂-C₆)-nitroalkoxy-(C₁-C₉)-alkyl, phenyloxy-(C₁-C₉)-alkyl, where the phenyl group may in each case be substituted by m identical or different radicals from the group consisting of (C₁-C₃)-alkyl, halogen, nitro, (C₁-C₃)-alkoxy,
R⁴ is hydrogen, (C₁-C₆)-alkylsulfonyl, (C₁-C₉)-alkoxy-(C₁-C₆)-alkylsulfonyl, or is phenylsulfonyl, thien-2-ylsulfonyl, (ethylthio)carbonyl, benzoyl, benzoyl-(C₁-C₆)-alkyl or benzyl, each of which is substituted by m identical or different radicals from the group consisting of halogen, (C₁-C₄)-alkyl and (C₁-C₉)-alkoxy,
X and Y independently of one another are hydrogen, mercapto, nitro, halogen, cyano, thiocyanato, (C₁-C₆)-alkyl, (C₁-C₆)-haloalkyl, (C₂-C₆)-alkenyl, (C₂-C₆)-haloalkenyl, (C₂-C₆)-alkynyl, (C₃-C₆)-haloalkynyl, (C₃-C₆)-cycloalkyl, OR⁵, methylsulfonylethoxymethyl, methylsulfonylethylsulfonylmethyl, methoxyethylsulfonylmethyl, OCOR⁵, OSO₂R⁵, S(O)ₙR⁵, SO₂OR⁵, SO₂N(R⁵)₂, (C₁-C₃)-alkoxy-(C₁-C₃)-alkoxy-(C₁-C₃)-alkyl, NR⁵SO₂R⁵, NR⁵COR⁵, (C₁-C₆)-alkyl-S(O)ₙR⁵, (C₁-C₆)-alkyl-OR⁵, (C₁-C₆)-alkyl-OCOR⁵, (C₁-C₆)-alkyl-OSO₂R⁵, (C₁-C₆)-alkyl-SO₂OR⁵, (C₁-C₆)-alkyl-SO₂N(R⁵)₂ or (C₁-C₆)-alkyl-NR⁵COR⁵;
R⁵ is hydrogen, (C₁-C₆)-alkyl, (C₂-C₆)-alkenyl, (C₂-C₆)-alkynyl, (C₃-C₆)-cycloalkyl, phenyl or phenyl-(C₁-C₆)-alkyl, where the six lastmentioned radicals are substituted by s radicals from the group consisting of hydroxyl, mercapto, amino, cyano, nitro, thiocyanato, OR⁶, SR⁶, N(R⁶)₂, NOR⁶, OCOR⁶, SCOR⁶, NR⁶COR⁶, CO₂R⁶, COSR⁶, CON(R⁶)₂, (C₁-C₄)-alkyliminooxy, (C₁-C₄)-alkoxyamino, (C₁-C₄)-alkylcarbonyl, (C₁-C₄)-alkoxy-(C₂-C₆)-alkoxycarbonyl and (C₁-C₄)-alkylsulfonyl;
R⁶ is hydrogen, (C₁-C₆)-alkyl, (C₂-C₆)-alkenyl or (C₂-C₆)-alkynyl,
m is 0, 1, 2, 3, 4 or 5,
n is 0, 1 or 2,
q is 0, 1, 2, 3, 4 or 5,
s is 0, 1, 2 or 3,
with the proviso that R³ is not (C₁-C₆)-haloalkyl if n is 0.

2. 3-Cyclopropyl-4-(3-thiobenzoyl)pyrazole according to Claim 1 in which
R¹ is (C₁-C₄)-alkyl,
R² is halogen, methyl or ethyl,
R³ is cyclopropylmethyl, cyclopropylmethoxyethyl, methoxyethyl, methoxypropyl, ethoxyethyl,
R⁴ is hydrogen, n-propylsulfonyl, phenylsulfonyl, methoxyethylsulfonyl, benzoylmethyl, benzoyl, 4-methylbenzoylmethyl, (ethylthio)carbonyl, 4-methylphenylsulfonyl, thien-2-ylsulfonyl,
X is nitro, halogen, (C₁-C₄)-alkyl, trifluoromethyl, (C₁-C₄)-alkoxy, methylsulfonyl, methoxymethyl, methoxymethoxymethyl, ethoxyethoxymethyl, ethoxymethoxymethyl, methoxyethoxymethyl, methoxypropoxymethyl, methylsulfonylmethyl, methylsulfonylethoxymethyl, methoxyethylsulfonylmethyl, methylsulfonylethylsulfonylmethyl,
Y is halogen, trifluoromethyl, (C₁-C₉)-alkoxy, methylsulfonyl or ethylsulfonyl,
n is 0, 1 or 2,
q is 0, 1 or 2.

3. 3-Cyclopropyl-4-(3-thiobenzoyl)pyrazole according to Claim 1 in which
R¹ is methyl or ethyl,
R³ is cyclopropylmethyl, cyclopropylmethoxyethyl, methoxyethyl, methoxypropyl, ethoxyethyl,
R⁴ is hydrogen, n-propylsulfonyl, phenylsulfonyl, methoxyethylsulfonyl, benzoylmethyl, benzoyl, 4-methylbenzoylmethyl, (ethylthio)carbonyl, 4-methylphenylsulfonyl, thien-2-ylsulfonyl,
X is nitro, bromine, chlorine, fluorine, methyl, ethyl, trifluoromethyl, methoxy, ethoxy, methylsulfonyl, methoxymethyl, methoxymethoxymethyl, ethoxyethoxymethyl, ethoxymethoxymethyl, methoxyethoxymethyl, methoxypropoxymethyl, methylsulfonylmethyl, methylsulfonylethoxymethyl, methoxyethylsulfonylmethyl, methylsulfonylethylsulfonylmethyl,
Y is bromine, chlorine, fluorine, trifluoromethyl, methoxy, methylsulfonyl or ethylsulfonyl,
n is 0, 1 or 2,
q is 0.

4. Herbicidal composition which comprises a herbicidally effective amount of at least one compound of the formula (I) according to any of Claims 1 to 3.

5. Herbicidal composition according to Claim 4 as a mixture with formulation auxiliaries.

6. Method for controlling unwanted plants which comprises applying an effective amount of at least one compound of the formula (I) according to any of Claims 1 to 3 or of a herbicidal composition according to Claim 4 or 5 to the plants or to the site of the unwanted plant growth.

7. Use of compounds of the formula (I) according to any of Claims 1 to 3 or of herbicidal compositions according to Claim 4 or 5 for controlling unwanted plants.

8. Use according to Claim 7 wherein the compounds of the formula (I) are used for controlling unwanted plants in crops of useful plants.

9. Use according to Claim 8 wherein the useful plants are transgenic useful plants.

## Revendications

1. 3-Cyclopropyl-4-(3-thiobenzoyl)pyrazoles de formule (I) ou leurs sels 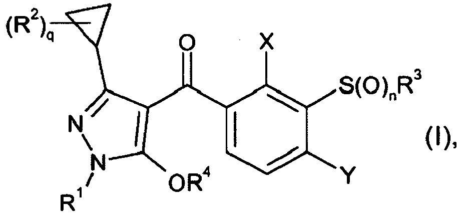 dans laquelle
R¹ signifie alkyle en (C₁-C₄),
R² signifie halogène ou alkyle en (C₁-C₄),
R³ signifie cycloalkyle en (C₃-C₈)-alkyle en (C₁-C₉), halogénoalkyle en (C₁-C₆), halogénocycloalkyle en (C₃-C₈)-alkyle en (C₁-C₉), nitroalkyle en (C₂-C₆), cycloalcoxy en (C₃-C₈)-alkyle en (C₁-C₉), cycloalkyle en (C₃-C₈)-alcoxy en (C₁-C₉)-alkyle en (C₁-C₉), alcoxy en (C₁-C₆)-alkyle en (C₁-C₉), alcényloxy en (C₂-C₆)-alkyle en (C₁-C₉), alcynyloxy en (C₂-C₆)-alkyle en (C₁-C₉), halogénoalcoxy en (C₁-C₆)-alkyle en (C₁-C₉), halogénocycloalkyle en (C₃-C₈) -alcoxy en (C₁-C₉)-alkyle en (C₁-C₉), halogénoalcényloxy en (C₂-C₆)-alkyle en (C₁-C₉), halogénoalcynyloxy en (C₂-C₆)-alkyle en (C₁-C₉), nitroalcoxy en (C₂-C₆)-alkyle en (C₁-C₉), phényloxy-alkyle en (C₁-C₉), le groupe phényle pouvant à chaque fois être substitué par m radicaux identiques ou différents du groupe constitué par alkyle en (C₁-C₃), halogène, nitro, alcoxy en (C₁-C₃),
R⁴ signifie hydrogène, alkylsulfonyle en (C₁-C₆), alcoxy en (C₁-C₄)-alkylsulfonyle en (C₁-C₆); ou phénylsulfonyle, thién-2-ylsulfonyle, (éthylthio)carbonyle, benzoyle, benzoyl-alkyle en (C₁-C₆) ou benzyle, chacun substitué par m radicaux identiques ou différents du groupe constitué par halogène, alkyle en (C₁-C₄) et alcoxy en (C₁-C₄),
X et Y signifient indépendamment l'un de l'autre hydrogène, mercapto, nitro, halogène, cyano, rhodano, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₃-C₆), cycloalkyle en (C₃-C₆), OR⁵, méthylsulfonyléthoxyméthyle, méthylsulfonyléthylsulfonylméthyle, méthoxyéthylsulfonylméthyle, OCOR⁵, OSO₂R⁵, S(O)ₙR⁵, SO₂OR⁵, SO₂N(R⁵)₂, alcoxy en (C₁-C₃)-alcoxy en (C₁-C₃)-alkyle en (C₁-C₃), NR⁵SO₂R⁵, NR⁵COR⁵, alkyle en (C₁-C₆)-S(O)ₙR⁵, alkyle en (C₁-C₆)-OR⁵, alkyle en (C₁-C₆)-OCOR⁵, alkyle en (C₁-C₆)-OSO₂R⁵, alkyle en (C₁-C₆)-SO₂OR⁵, alkyle en (C₁-C₆)-SO₂N(R⁵)₂ ou alkyle en (C₁-C₆)-NR⁵COR⁵ ;
R⁵ signifie hydrogène, alkyle en (C₁-C₆), alcényle en (C₂-C₆), alcynyle en (C₂-C₆), cycloalkyle en (C₃-C₆), phényle ou phényl-alkyle en (C₁-C₆), les six derniers radicaux cités étant substitués par s radicaux du groupe constitué par hydroxy, mercapto, amino, cyano, nitro, rhodano, OR⁶, SR⁶, N(R⁶)₂, NOR⁶, OCOR⁶, SCOR⁶, NR⁶COR⁶, CO₂R⁶, COSR⁶, CON(R⁶)₂, alkyliminooxy en (C₁-C₄), alcoxyamino en (C₁-C₄), alkylcarbonyle en (C₁-C₄), alcoxy en (C₁-C₄)-alcoxycarbonyle en (C₂-C₆) et alkylsulfonyle en (C₁-C₄) ;
R⁶ signifie hydrogène, alkyle en (C₁-C₆), alcényle en (C₂-C₆) ou alcynyle en (C₂-C₆),
m signifie 0, 1, 2, 3, 4 ou 5,
n signifie 0, 1 ou 2,
q signifie 0, 1, 2, 3, 4 ou 5,
s signifie 0, 1, 2 ou 3,
à condition que R³ ne signifie pas halogénoalkyle en (C₁-C₆) lorsque n représente 0.

2. 3-Cyclopropyl-4-(3-thiobenzoyl)pyrazoles selon la revendication 1, dans lesquels
R¹ signifie alkyle en (C₁-C₄),
R² signifie halogène, méthyle ou éthyle,
R³ signifie cyclo-propylméthyle, cyclo-propylméthoxyéthyle, méthoxyéthyle, méthoxypropyle, éthoxyéthyle,
R⁴ signifie hydrogène, n-propylsulfonyle, phénylsulfonyle, méthoxyéthylsulfonyle, benzoylméthyle, benzoyle, 4-méthylbenzoylméthyle, (éthylthio)carbonyle, 4-méthylphénylsulfonyle, thién-2-ylsulfonyle,
X signifie nitro, halogène, alkyle en (C₁-C₄), trifluorométhyle, alcoxy en (C₁-C₄), méthylsulfonyle, méthoxyméthyle, méthoxyméthoxyméthyle, éthoxyéthoxyméthyle, éthoxyméthoxyméthyle, méthoxyéthoxyméthyle, méthoxypropoxyméthyle, méthylsulfonylméthyle, méthylsulfonyléthoxyméthyle, méthoxyéthylsulfonylméthyle, méthylsulfonyléthylsulfonylméthyle,
Y signifie halogène, trifluorométhyle, alcoxy en (C₁-C₄), méthylsulfonyle ou éthylsulfonyle,
n signifie 0, 1 ou 2,
q signifie 0, 1 ou 2.

3. 3-Cyclopropyl-4-(3-thiobenzoyl)pyrazoles selon la revendication 1, dans lesquels
R¹ signifie méthyle ou éthyle,
R³ signifie cyclo-propylméthyle, cyclo-propylméthoxyéthyle, méthoxyéthyle, méthoxypropyle, éthoxyéthyle,
R⁴ signifie hydrogène, n-propylsulfonyle, phénylsulfonyle, méthoxyéthylsulfonyle, benzoylméthyle, benzoyle, 4-méthylbenzoylméthyle, (éthylthio)carbonyle, 4-méthylphénylsulfonyle, thién-2-ylsulfonyle,
X signifie nitro, brome, chlore, fluor, méthyle, éthyle, trifluorométhyle, méthoxy, éthoxy, méthylsulfonyle, méthoxyméthyle, méthoxyméthoxyméthyle, éthoxyéthoxyméthyle, éthoxyméthoxyméthyle, méthoxyéthoxyméthyle, méthoxypropoxyméthyle, méthylsulfonylméthyle, méthylsulfonyléthoxyméthyle, méthoxyéthylsulfonylméthyle, méthylsulfonyléthylsulfonylméthyle,
Y signifie brome, chlore, fluor, trifluorométhyle, méthoxy, méthylsulfonyle ou éthylsulfonyle,
n signifie 0, 1 ou 2,
q signifie 0.

4. Agent herbicide, **caractérisé par** une teneur herbicide efficace en au moins un composé de formule (I) selon l'une quelconque des revendications 1 à 3.

5. Agent herbicide selon la revendication 4, en mélange avec des adjuvants de formulation.

6. Procédé de lutte contre des plantes indésirables, **caractérisé en ce qu'**une quantité efficace d'au moins un composé de formule (I) selon l'une quelconque des revendications 1 à 3 ou d'un agent herbicide selon la revendication 4 ou 5 est appliquée sur les plantes ou à l'emplacement de la végétation indésirable.

7. Utilisation de composés de formule (I) selon l'une quelconque des revendications 1 à 3 ou d'agents herbicides selon la revendication 4 ou 5 pour lutter contre des plantes indésirables.

8. Utilisation selon la revendication 7, **caractérisée en ce que** les composés de formule (I) sont utilisés pour lutter contre des plantes indésirables dans des cultures de plantes utiles.

9. Utilisation selon la revendication 8, **caractérisée en ce que** les plantes utiles sont des plantes utiles transgéniques.
